# EUROPEAN PATENT SPECIFICATION

(11) **EP 4 146 283 B1**
(45) Date of publication and mention of the grant of the patent: **11.11.2026**
(21) Application number: 21726806.9
(22) Date of filing: 30.04.2021
(51) Int. Cl.: A61K 47/68, A61P 35/00

(54) **ANTIBODY-DRUG CONJUGATES (ADCS) COMPRISING AN ANTI-TROP-2 ANTIBODY, COMPOSITIONS COMPRISING SUCH ADCS, AS WELL AS METHODS OF MAKING AND USING THE SAME**
ANTIKÖRPER-WIRKSTOFF-KONJUGATE (ADCS) MIT EINEM ANTI-TROP-2-ANTIKÖRPER, ZUSAMMENSETZUNGEN MIT SOLCHEN ADCS SOWIE VERFAHREN ZUR HERSTELLUNG UND VERWENDUNG DAVON
CONJUGUÉS ANTICORPS-MÉDICAMENT (ADC) COMPRENANT UN ANTICORPS ANTI-TROP-2, COMPOSITIONS COMPRENANT DE TELS ADC, AINSI QUE PROCÉDÉS DE FABRICATION ET D'UTILISATION DE CEUX-CI

(30) Priority: 03.05.2020 WO PCT/CN2020/088565; 13.04.2021 WO PCT/CN2021/086849
(43) Date of publication of application: 15.03.2023
(73) Proprietor: Levena (Suzhou) Biopharma Co., Ltd., Suzhou, Jiangsu 215123 (CN); Shanghai Escugen Biotechnology Co., Ltd., Pudong, Shanghai 200120 (CN)
(72) Inventor: ZHU, Tong, Suzhou, Jiangsu 215123 (CN); KHASANOV, Alisher B., Suzhou, Jiangsu 215123 (CN); LI, Hui, Suzhou, Jiangsu 215123 (CN); GUO, Maojun, Suzhou, Jiangsu 215123 (CN); LI, Haihong, Suzhou, Jiangsu 215123 (CN); XU, Chuanying, Shanghai 200120 (CN); HE, Feng, Shanghai 200120 (CN); ZHOU, Qing, Shanghai 200120 (CN)
(74) Representative: Script Intellectual Property LLP
(86) International application number: PCT/US2021/030198
(87) International publication number: WO 2021/225892

(56) References cited:
- WO-A1-2016/046574
- WO-A1-2016/165580
- DEONARAIN M P: "Preparation of drug conjugates and biological materials and their therapeutic uses", WO2016046574 A1, 1 January 2016 (2016-01-01), pages 1 - 8, XP055820787
- LIU J: "Preparation of anti-human c-met antibodies and immunoconjugates with cytotoxic agents for treatment of c-Met-associated disease or cancer", WO2016165580 A1, 1 January 2016 (2016-01-01), pages 1 - 2, XP055820785

## Description

### CROSS-REFERENCE TO RELATED APPLICATIONS

This application claims priority to International Application No. PCT/CN2020/088565, filed on May 3, 2020, and to International Application No. PCT/CN2021/086849, filed on April 13, 2021.

### INTRODUCTION AND SUMMARY

This disclosure relates to an antibody-drug conjugate (ADC), comprising an anti-Trop-2 antibody and methods of making and using the same.

ADCs can target drugs to specific cells, such as cancer cells, thus permitting the delivery of drugs that would be highly toxic if used alone. SN-38 (7-ethyl-10-hydroxy camptothecin) is a camptothecin that is the active component of irinotecan (CPT-11), a topoisomerase I inhibitor. There have been efforts to develop ADCs comprising SN-38 and an anti-Trop-2 antibody. Trop-2 (trophoblastic cell-surface antigen; also, termed epithelial glycoprotein-1 or EGP-1) is a glycoprotein that is highly expressed by many epithelial cancers. For further background regarding SN-38 and Trop-2, see, e.g., Ocean et al., Cancer 123:3843-54 (2017) and references cited therein.

It has been challenging to provide anti-Trop-2 ADCs comprising SN-38 that are effective while maintaining favorable safety profiles. For example, Ocean et al., *supra,* report a clinical trial with the IMMU-132 (sacituzumab govitecan; also referred to as ADC-CL2A-SN38) ADC. Although the ADC was characterized as providing an "encouraging overall response," there was a high frequency of adverse events -80 of 81 and 89 of 97 patients receiving 8 mg/kg and 10 mg/kg doses, respectively (see Table 2 of Ocean et al. and accompanying text). Notably, the linker in ADC-CL2A-SN38 has been reported to allow "SN-38 to dissociate from the conjugate in serum with a half-life of approximately 1 day," which may explain or contribute to the high adverse event frequency. See Govindan et al., Mol Cancer Ther 12:968-978 (2013). The chemical structure of the CL2A-SN38 linker-drug moiety is shown below (depicted as the reactive maleimide form used for conjugating to the antibody). 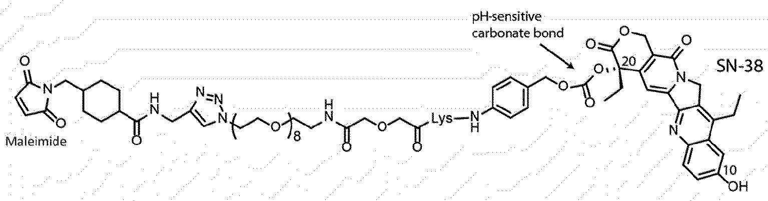 Another ADC comprising SN-38, ADC-CL2E-SN38, uses a carbamate-containing linker instead of the pH-sensitive carbonate bond and has a different point of attachment to SN38 than ADC-CL2A-SN38. A conjugate with the CL2E linker was reported to have a serum half-life of 87.5 days, but also to show "diminished efficacy *in vivo"* and was considered "inferior to the less stable CL2A-linked SN-38." Govindan et al., *supra,* p. 972 and 977.

It is contemplated herein to improve the safety and/or reduce the frequency of adverse events following treatment with an anti-Trop-2 ADC comprising SN-38 by using a linker in the ADC that mitigates undesired release of SN-38 away from cancer cells, while permitting on-target release sufficient for efficacy.

Accordingly, the present disclosure provides ADCs of formula (I) comprising an anti-Trop-2 antibody conjugated to SN-38 via a linker moiety. The ADC compounds of formula (I) can provide more stability and provide better toxicity data than certain other SN-38 ADCs. The improved activity of ADC compounds described herein is attributed to the linker moiety of formula (I), which permits selective release of SN-38 at the target Trop-2-expressing cells. In some embodiments, an ADC described herein exhibits greater stability than ADC-CL2A-SN38 (e.g., at neutral pH, such as the exemplary conditions in Example B3, or in vivo, such as the exemplary conditions in Example B4) and/or greater in vivo efficacy than ADC-CL2E-SN38. In some embodiments, an ADC described herein exhibits improved safety (e.g., reduced frequency of adverse events) relative to ADC-CL2A-SN38 and/or greater in vivo efficacy than ADC-CL2E-SN38. In some embodiments, an ADC described herein exhibits greater stability (e.g., at neutral pH, such as the exemplary conditions in Example B3, or in vivo, such as the exemplary conditions in Example B4) than ADC-CL2A-SN38 and improved safety (e.g., reduced frequency of adverse events) relative to ADC-CL2A-SN38, and may further exhibit greater in vivo efficacy than ADC-CL2E-SN38.

WO2016046574 describes biological materials and uses thereof. WO2016165580 describes an anti-c-Met antibody and anti-c-Met antibody-cytotoxic drug conjugate and pharmaceutical use thereof.

Any references herein to methods of treatment are to be interpreted as references to compounds, compositions and medicaments for use in methods of treatment of the human or animal body by therapy.

The aspects and embodiments of the present invention are set forth in the claims.

Embodiment 1 is an antibody-drug conjugate (ADC) which is of formula (I): 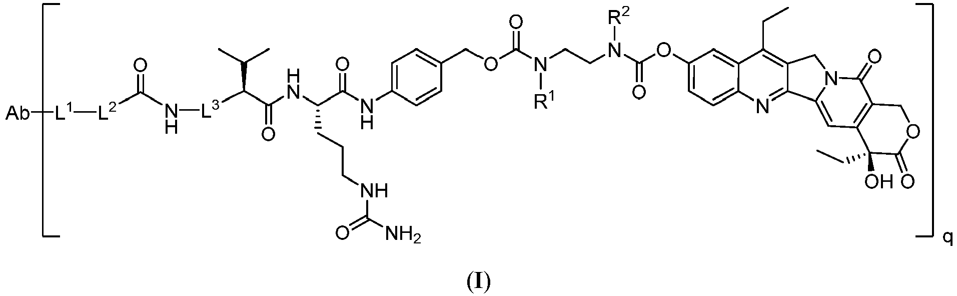 or is a pharmaceutically acceptable salt thereof, wherein:
Ab is an anti-Trop-2 antibody;
q is a value in the range of 1 to 20;
L¹ is a linker bound to the anti-Trop-2 antibody;
L² is -(CH₂)ₚ- where p is 4, 5, 6, 7, or 8;
L³ is a bond or a polyoxyethylene-based divalent linker; and
R¹ and R² are each independently C₁₋₆ alkyl.

Embodiment 2 is the ADC of embodiment 1, wherein L¹ is a linker bound to a sulfur of the anti-Trop-2 antibody.

Embodiment 3 is the ADC of embodiment 1 or 2, wherein -L¹-L²- is

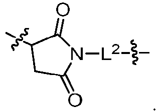

Embodiment 4 is the ADC of any one of embodiments 1-3, wherein q is 1, 2, 3, 4, 5, 6, 7, 8, 9, 10, 11, 12, 13, 14, 15, 16, 17, 18, 19, or 20.

Embodiment 5 is the ADC of any one of embodiments 1-3, wherein q is a value in the range of 1 to 10.

Embodiment 6 is the ADC of embodiment 5, wherein q is a value in the range of 6 to 8.

Embodiment 7 is the ADC of any one of embodiments 1-6, wherein p is 4, 5, or 6.

Embodiment 8 is the ADC of embodiment 7, wherein p is 5.

Embodiment 9 is the ADC of any one of embodiments 1-8, wherein L³ is a bond.

Embodiment 10 is the ADC of any one of embodiments 1-8, wherein L³ is a polyoxyethylene-based divalent linker.

Embodiment 11 is the ADC of any one of embodiments 1-10, wherein R¹ is C₁₋₄ alkyl.

Embodiment 12 is the ADC of embodiment 11, wherein R¹ is C₁₋₃ alkyl.

Embodiment 13 is the ADC of embodiment 12, wherein R¹ is methyl.

Embodiment 14 is the ADC of embodiment 12, wherein R¹ is ethyl.

Embodiment 15 is the ADC of any one of embodiments 1-14, wherein R² is C₁₋₄ alkyl.

Embodiment 16 is the ADC of embodiment 15, wherein R² is C₁₋₃ alkyl.

Embodiment 17 is the ADC of embodiment 16, wherein R² is methyl.

Embodiment 18 is the ADC of embodiment 16, wherein R² is ethyl.

Embodiment 19 is the ADC of any one of embodiments 1-18, wherein R¹ and R² are identical.

Embodiment 20 is the ADC of any one of embodiments 1-13 and 15-17, wherein the ADC is of formula (IIa): 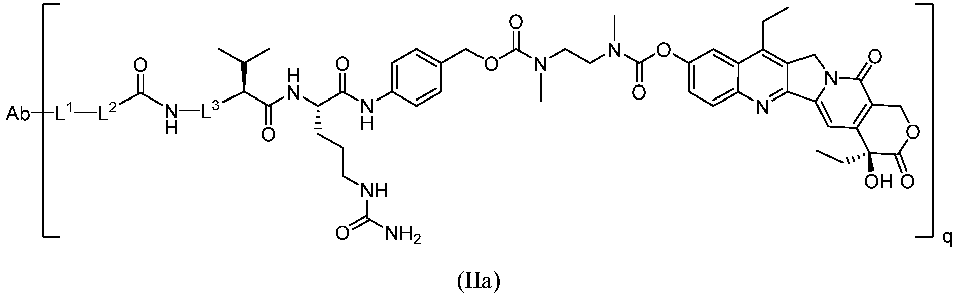 or a pharmaceutically acceptable salt thereof.

Embodiment 21 is the ADC of embodiment 20, wherein the ADC is of formula (IIa-1): 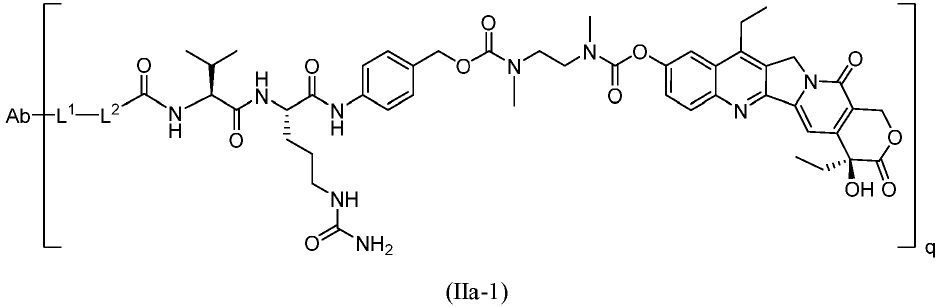 or a pharmaceutically acceptable salt thereof.

Embodiment 22 is the ADC of any one of embodiments 1-19, wherein the ADC is of formula (IIb): 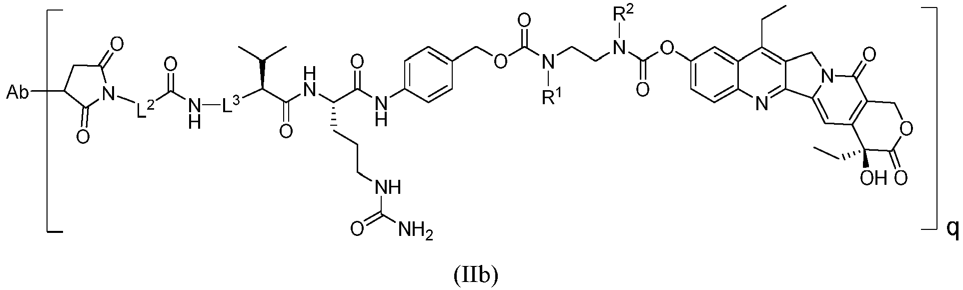 or a pharmaceutically acceptable salt thereof.

Embodiment 23 is the ADC of embodiment 22, wherein the ADC is of formula (IIb-1): 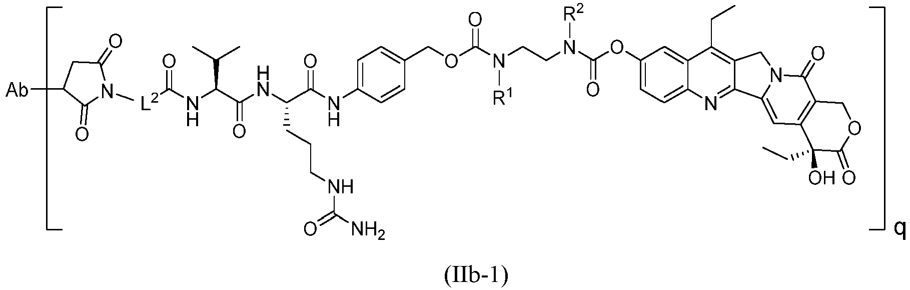 or a pharmaceutically acceptable salt thereof.

Embodiment 24 is the ADC of any one of embodiments 1-19, wherein the ADC is of formula (IIc): 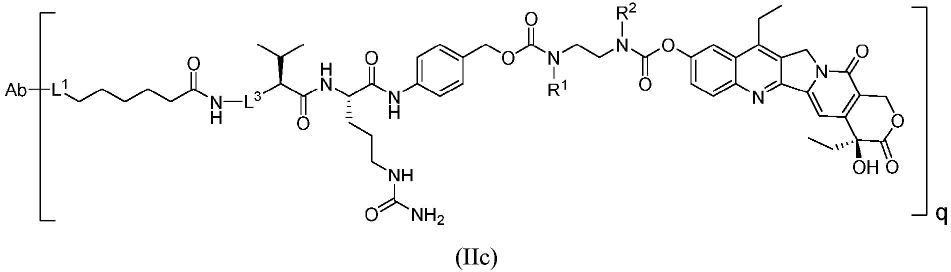 or a pharmaceutically acceptable salt thereof.

Embodiment 25 is the ADC of embodiment 24, wherein the ADC is of formula (IIc-1): 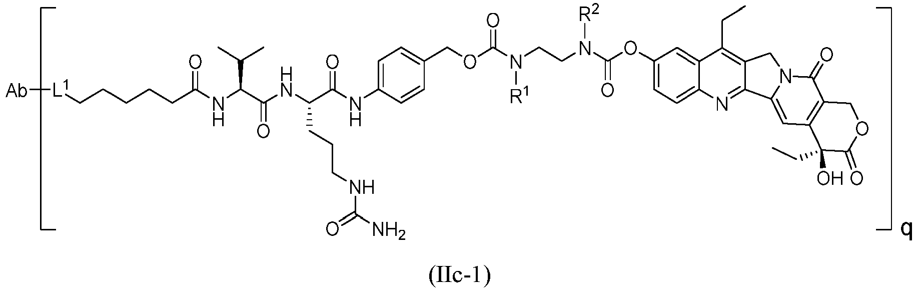 or a pharmaceutically acceptable salt thereof.

Embodiment 26 is the ADC of embodiment 20, wherein the ADC is of formula (IIIa): 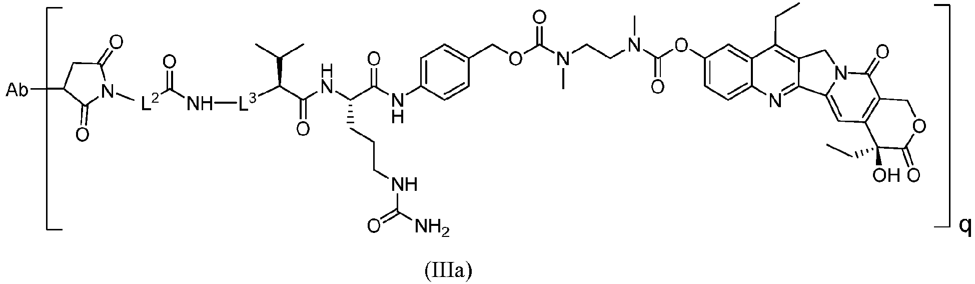 or a pharmaceutically acceptable salt thereof.

Embodiment 27 is the ADC of embodiment 26, wherein the ADC is of formula (IIIa-1): 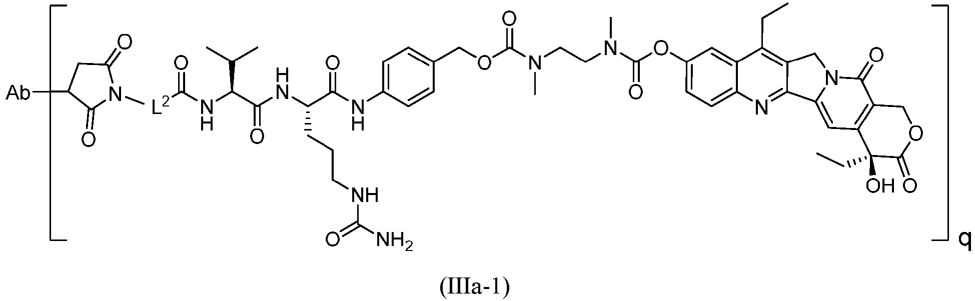 or a pharmaceutically acceptable salt thereof.

Embodiment 28 is the ADC of embodiment 22, wherein the ADC is of formula (IIIb): 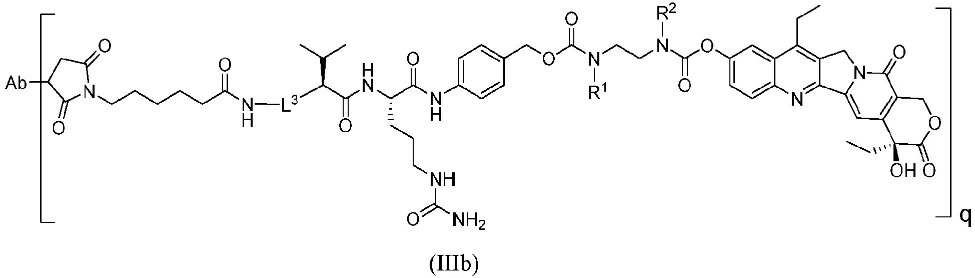 or a pharmaceutically acceptable salt thereof.

Embodiment 29 is the ADC embodiment 28, wherein the ADC is of formula (IIIb-1): 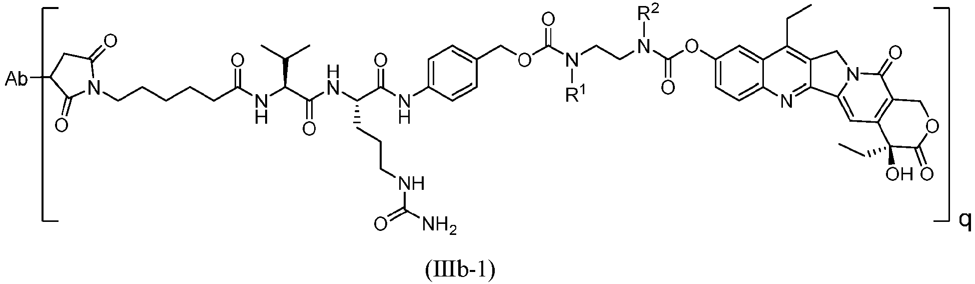 or a pharmaceutically acceptable salt thereof.

Embodiment 30 is the ADC of embodiment 22, wherein the ADC is of formula (IIIc): 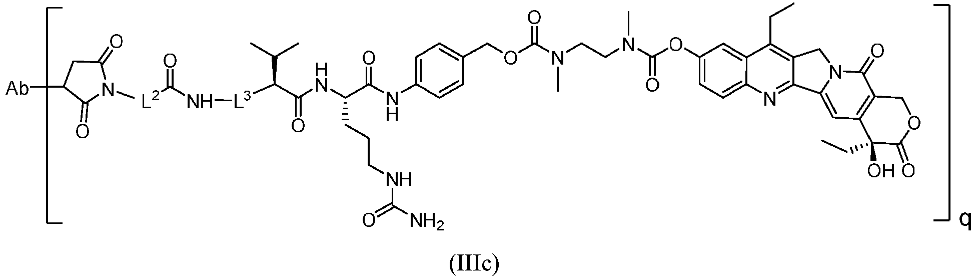 or a pharmaceutically acceptable salt thereof.

Embodiment 31 is the ADC of embodiment 30, wherein the ADC is of formula (IIIc-1): 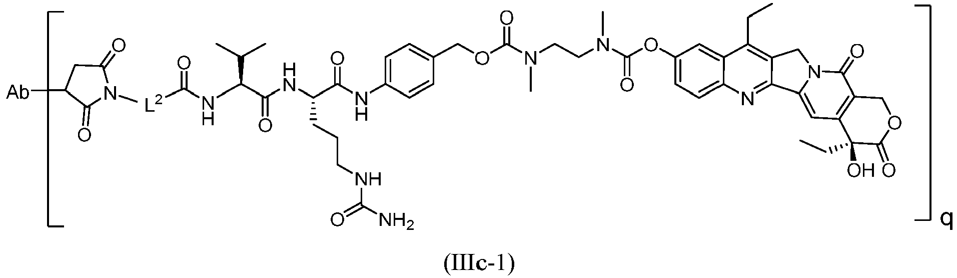 or a pharmaceutically acceptable salt thereof.

Embodiment 32 is the ADC of embodiment 1, wherein the ADC is of formula (IV): 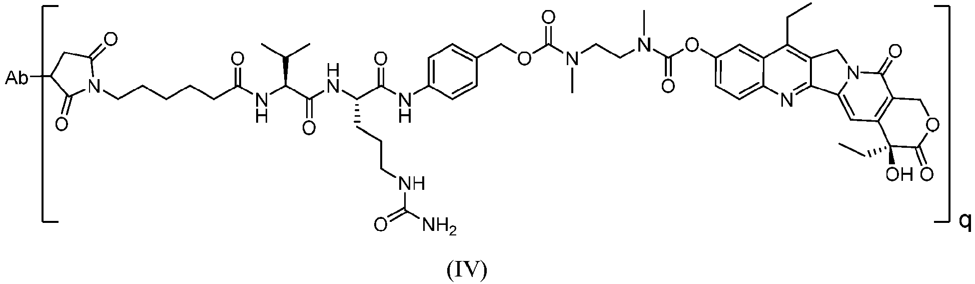 or a pharmaceutically acceptable salt thereof.

Embodiment 33 is the ADC of any one of embodiments 1-32, wherein the anti-Trop-2 antibody comprises a VL HVR1 comprising the sequence of SEQ ID NO: 1, a VL HVR2 comprising the sequence of SEQ ID NO: 2, a VL HVR3 comprising the sequence of SEQ ID NO: 3, a VH HVR1 comprising the sequence of SEQ ID NO: 4, a VH HVR2 comprising the sequence of SEQ ID NO: 5, and a VH HVR3 comprising the sequence of SEQ ID NO: 6.

Embodiment 34 is the ADC of any one of embodiments 1-33, wherein the anti-Trop-2 antibody comprises a VL having a sequence with at least 95%, 96%, 97%, 98%, or 99% identity to SEQ ID NO: 7.

Embodiment 35 is the ADC of any one of embodiments 1-34, wherein the anti-Trop-2 antibody comprises a VH having a sequence with at least 95%, 96%, 97%, 98%, or 99% identity to SEQ ID NO: 8.

Embodiment 36 is the ADC of any one of embodiments 1-35, wherein the anti-Trop-2 antibody comprises a VL having the sequence of SEQ ID NO: 7.

Embodiment 37 is the ADC of any one of embodiments 1-36, wherein the anti-Trop-2 antibody comprises a VH having the sequence of SEQ ID NO: 8.

Embodiment 38 is the ADC of any one of embodiments 1-37, wherein the anti-Trop-2 antibody comprises a kappa light chain.

Embodiment 39 is the ADC of any one of embodiments 1-38, wherein the anti-Trop-2 antibody is an IgG antibody, optionally wherein the anti-Trop-2 antibody is an IgG1 antibody.

Embodiment 40 is the ADC of any one of embodiments 1-39, wherein the anti-Trop-2 antibody binds a human Trop-2, optionally wherein the human Trop-2 has the amino acid sequence of SEQ ID NO: 9.

Embodiment 41 is the ADC of any one of embodiments 1-40, for use in therapy.

Embodiment 42 is the ADC of embodiment 41, for use in treating a Trop-2-expressing cancer.

Embodiment 43 is a method of treating a Trop-2-expressing cancer in a subject, comprising administering the ADC of any one of embodiments 1-40 to a subject in need thereof.

Embodiment 44 is use of the ADC of any one of embodiments 1-40 for the manufacture of a medicament.

Embodiment 45 is use of the ADC of any one of embodiments 1-40 for the manufacture of a medicament for treating a Trop-2-expressing cancer.

Embodiment 46 is the ADC for use, method, or use of any one of embodiments 42, 43, or 45, wherein the Trop-2-expressing cancer is an epithelial-cell-derived cancer.

Embodiment 47 is the ADC for use, method, or use of embodiment 46, wherein the Trop-2-expressing cancer is a carcinoma.

Embodiment 48 is the ADC for use, method, or use of embodiment 47, wherein the carcinoma is a basal cell carcinoma, a squamous cell carcinoma, a renal cell carcinoma, a ductal carcinoma in situ, an invasive ductal carcinoma, or an adenocarcinoma.

Embodiment 49 is the ADC for use, method, or use of any one of embodiments 46-48, wherein the Trop-2-expressing cancer comprises a solid tumor.

Embodiment 50 is the ADC for use, method, or use of any one of embodiments 46-49, wherein the Trop-2-expressing cancer is metastatic.

Embodiment 51 is the ADC for use, method, or use of any one of embodiments 46-50, wherein the Trop-2-expressing cancer is a relapsed cancer.

Embodiment 52 is the ADC for use, method, or use of any one of embodiments 42, 43, and 45-51, wherein the Trop-2-expressing cancer is a pancreatic cancer, a gastric cancer, a breast cancer, a melanoma, a kidney cancer, a colorectal cancer, an endometrial cancer, a prostate cancer, a urothelial cancer, a glioblastoma, a lung cancer, a cervical cancer, an esophageal cancer, or an ovarian cancer.

Embodiment 53 is the ADC for use, method, or use of embodiment 52, wherein the Trop-2-expressing cancer is a pancreatic cancer.

Embodiment 54 is the ADC for use, method, or use of embodiment 52, wherein the Trop-2-expressing cancer is a gastric cancer.

Embodiment 55 is the ADC for use, method, or use of embodiment 52, wherein the Trop-2-expressing cancer is a breast cancer.

Embodiment 56 is the ADC for use, method, or use of embodiment 55, wherein the Trop-2-expressing cancer is triple-negative breast cancer.

Embodiment 57 is the ADC for use, method, or use of any one of embodiments 52-56, wherein the cancer is metastatic.

Embodiment 58 is a method of preparing the ADC of embodiment 1, comprising reacting an anti-Trop-2 antibody with a molecule of formula (P-I): 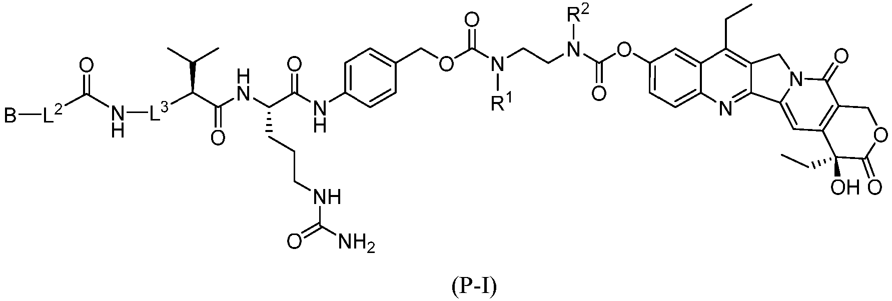 or a pharmaceutically acceptable salt thereof, wherein:
B is a reactive moiety capable of forming a bond with the anti-Trop-2 antibody;
L² is -(CH₂)ₚ- where p is 4, 5, 6, 7, or 8;
L³ is a bond or a polyoxyethylene-based divalent linker; and
R¹ and R² are each independently C₁₋₆ alkyl.

Embodiment 59 is the method of embodiment 58, wherein B is a reactive moiety capable of forming a bond with a sulfhydryl of the anti-Trop-2 antibody.

Embodiment 60 is the method of embodiment 58 or 59, wherein B is N-maleimido.

Embodiment 61 is the method of any one of embodiments 58-60, wherein the ADC is the ADC of any one of embodiments 1-40.

Embodiment 62 is the method of any one of embodiments 58-61, wherein p is 4, 5, or 6.

Embodiment 63 is the method of embodiment 62, wherein p is 5.

Embodiment 64 is the method of any one of embodiments 58-63, wherein R¹ is C₁₋₄ alkyl.

Embodiment 65 is the method of embodiment 64, wherein R¹ is C₁₋₃ alkyl.

Embodiment 66 is the method of embodiment 65, wherein R¹ is methyl.

Embodiment 67 is the method of embodiment 65, wherein R¹ is ethyl.

Embodiment 68 is the method of any one of embodiments 58-67, wherein R² is C₁₋₄ alkyl.

Embodiment 69 is the method of embodiment 68, wherein R² is C₁₋₃ alkyl.

Embodiment 70 is the method of embodiment 69, wherein R² is methyl.

Embodiment 71 is the method of embodiment 69, wherein R² is ethyl.

Embodiment 72 is the method of any one of embodiments 58-71, wherein R¹ and R² are identical.

Embodiment 73 is the method of any one of embodiments 58-72, wherein L³ is a bond.

Embodiment 74 is the method of any one of embodiments 58-72, wherein L³ is a polyoxyethylene-based divalent linker.

Embodiment 75 is the method of any one of embodiments 58-66, 68-70, 73, and 74, wherein the molecule is of formula (P-IIa): 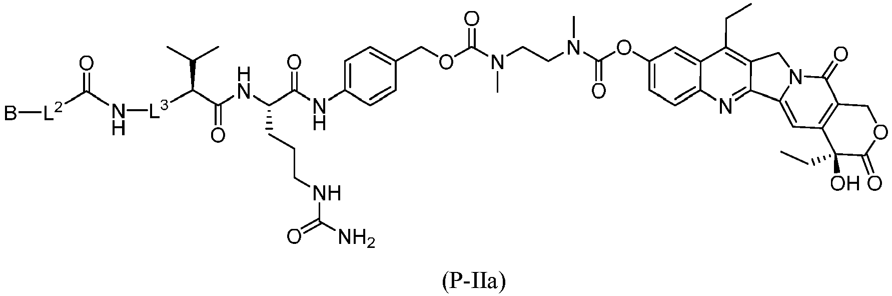 or a pharmaceutically acceptable salt thereof.

Embodiment 76 is the method of embodiment 75, wherein the molecule is of formula (P-IIa-1): 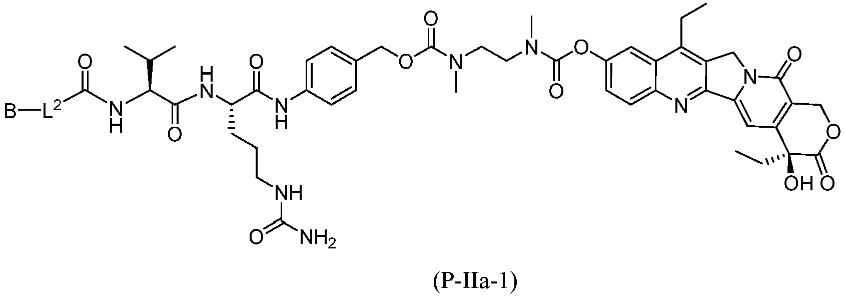 or a pharmaceutically acceptable salt thereof.

Embodiment 77 is the method of any one of embodiments 58-74, wherein the molecule is of formula (P-IIb): 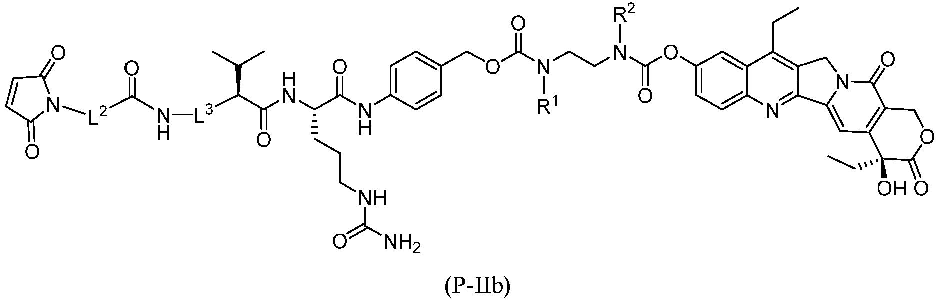 or a pharmaceutically acceptable salt thereof.

Embodiment 78 is the method of embodiment 77, wherein the molecule is of formula (P-IIb-1): 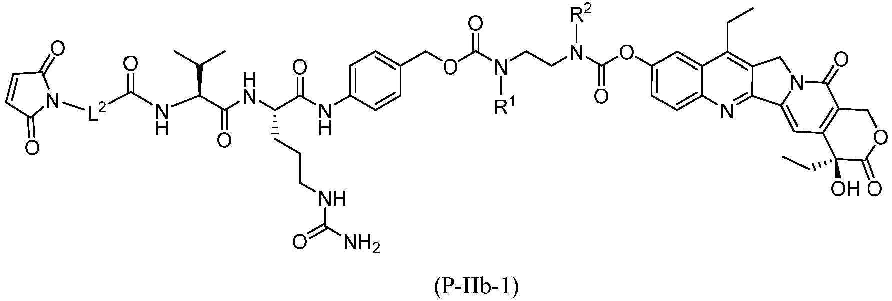 or a pharmaceutically acceptable salt thereof.

Embodiment 79 is the method of any one of embodiments 58-74, wherein the molecule is of formula (P-IIc): 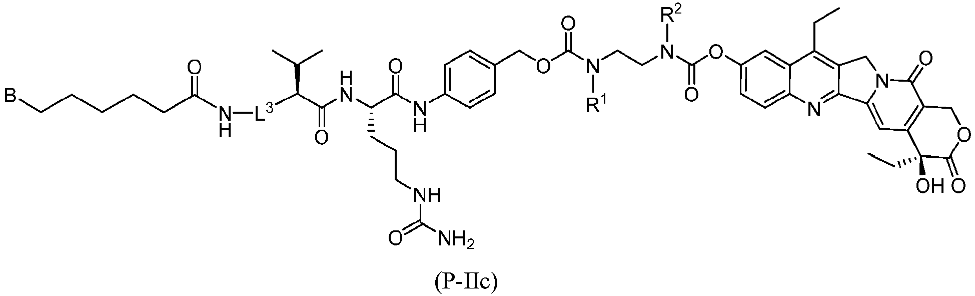 or a pharmaceutically acceptable salt thereof.

Embodiment 80 is the method of embodiment 79, wherein the molecule is of formula (P-IIc-1): 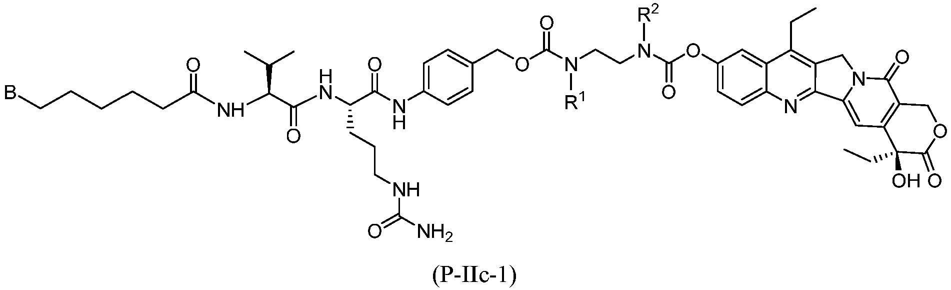 or a pharmaceutically acceptable salt thereof.

Embodiment 81 is the method of any one of embodiments 58-74, wherein the molecule is of formula (P-IIIa): 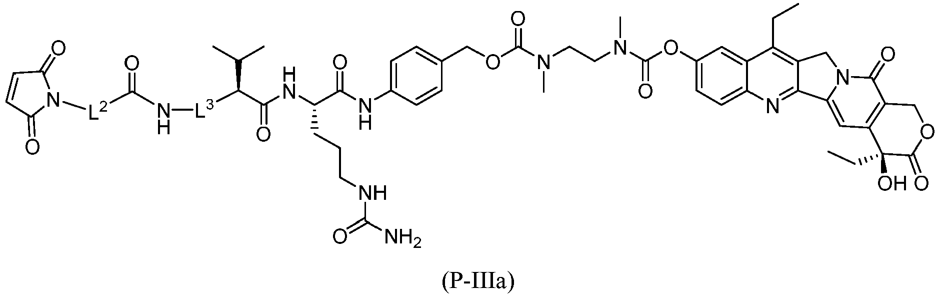 or a pharmaceutically acceptable salt thereof.

Embodiment 82 is the method of embodiment 81, wherein the molecule is of formula (P-IIIa-1): 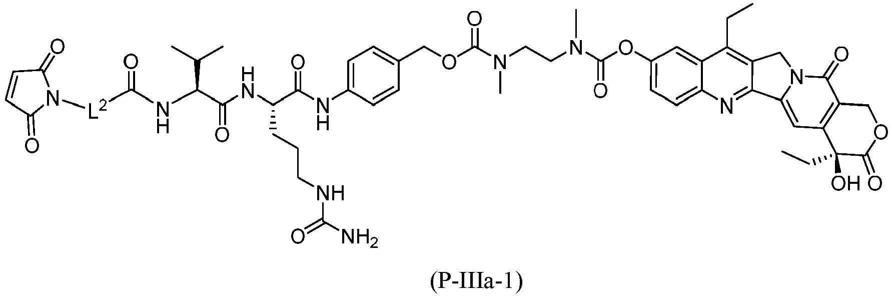 or a pharmaceutically acceptable salt thereof.

Embodiment 83 is the method of any one of embodiments 58-74, wherein the molecule is of formula (P-IIIb): 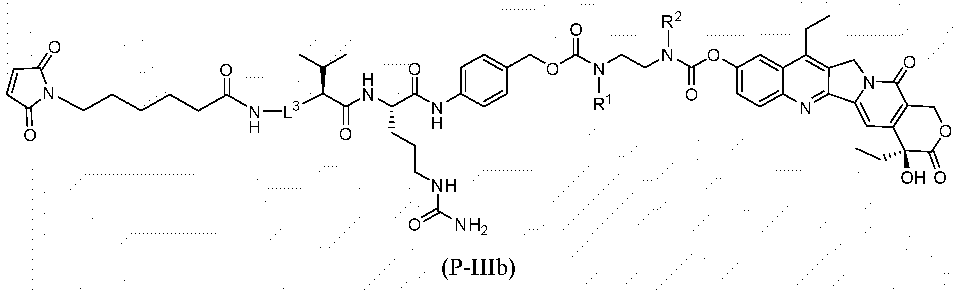 or a pharmaceutically acceptable salt thereof.

Embodiment 84 is the method of embodiment 83, wherein the molecule is of formula (P-IIIb-1): 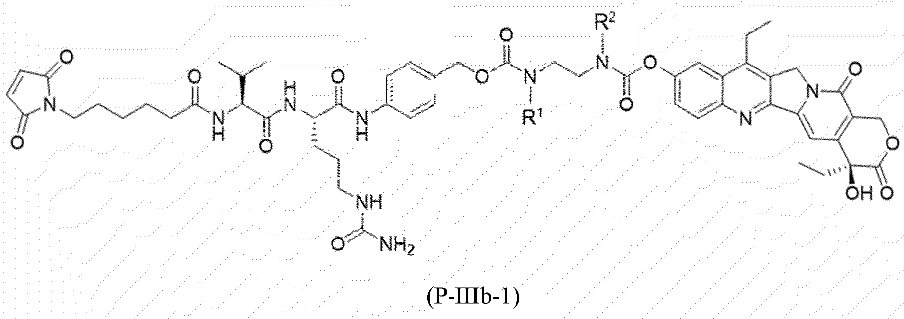 or a pharmaceutically acceptable salt thereof.

Embodiment 85 is the method of embodiment 75, wherein the molecule is of formula (P-IIIc): 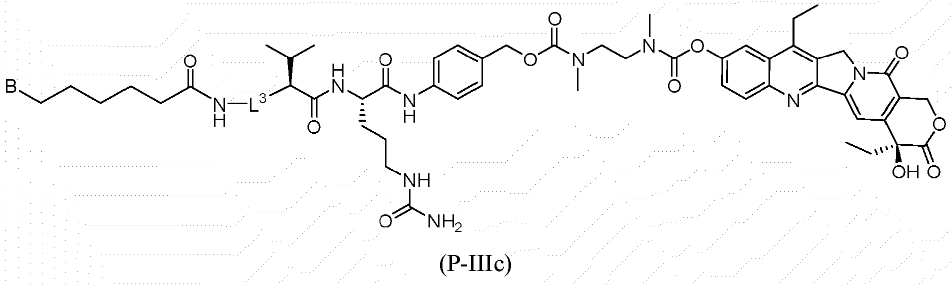 or a pharmaceutically acceptable salt thereof.

Embodiment 86 is the method of embodiment 85, wherein the molecule is of formula (P-IIIc-1): 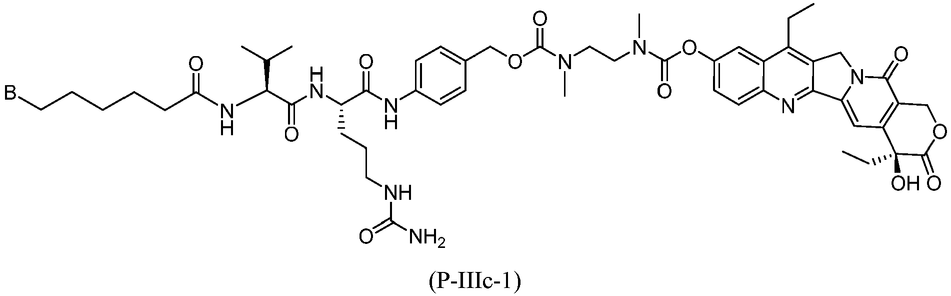 or a pharmaceutically acceptable salt thereof.

Embodiment 87 is the method of embodiment 58, wherein the molecule is of formula (P-IV): 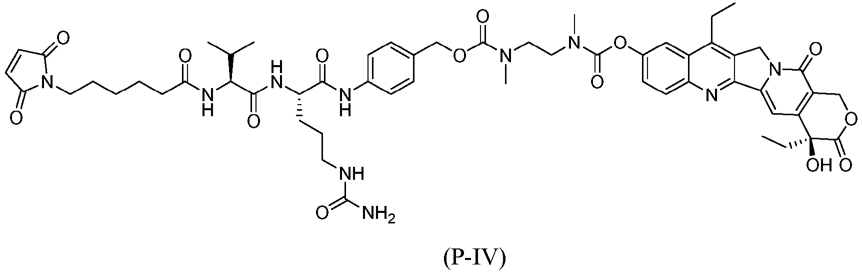 or a pharmaceutically acceptable salt thereof.

### FIGURE LEGENDS

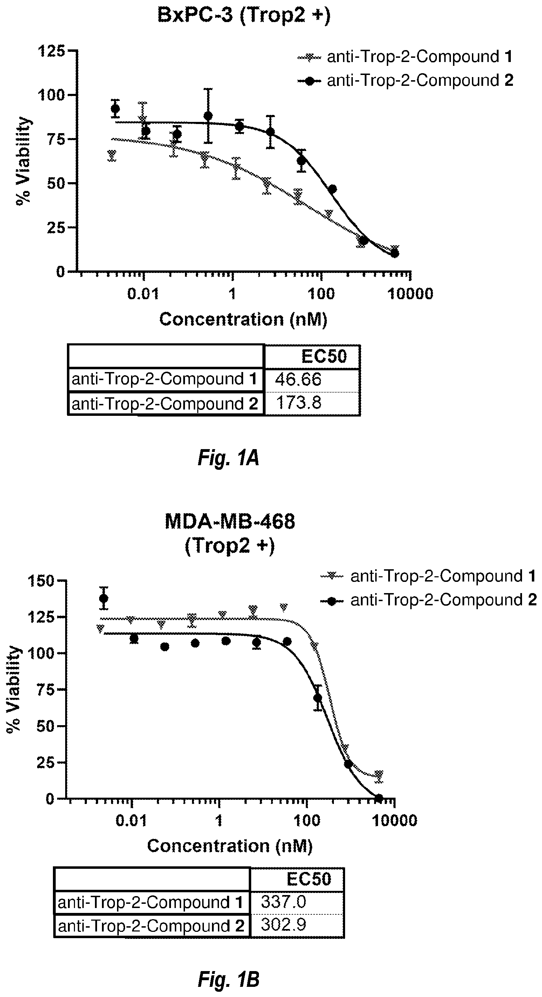

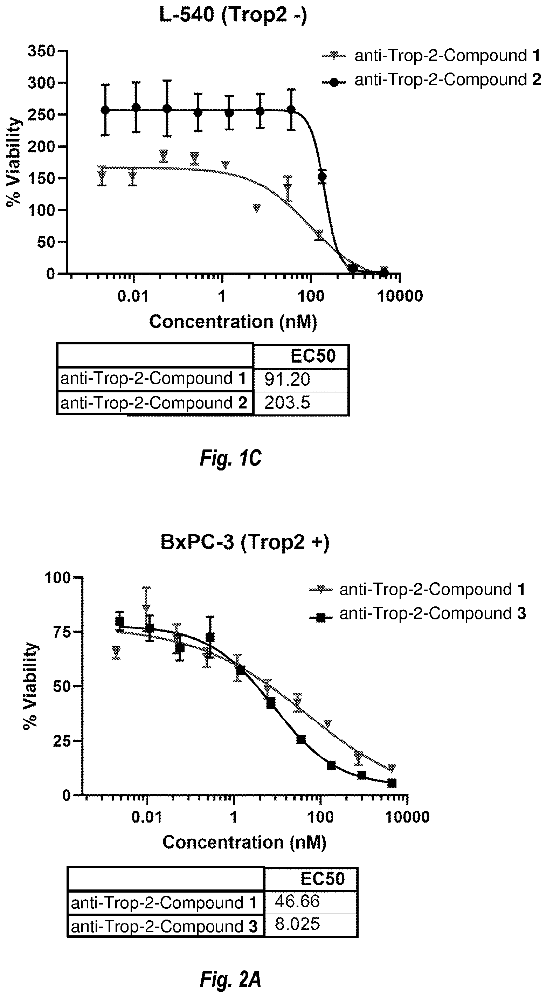

FIG. 1 shows results of an *in vitro* efficacy study of anti-Trop-2-Compound **1** (shown with triangles) and anti-Trop-2-Compound **2** (shown with circles) using: A) BxPC-3 (Trop-2 +) cells; B) MDA-MB-468 (Trop-2 +) cells; and C) L-540 (Trop-2 -) cells.

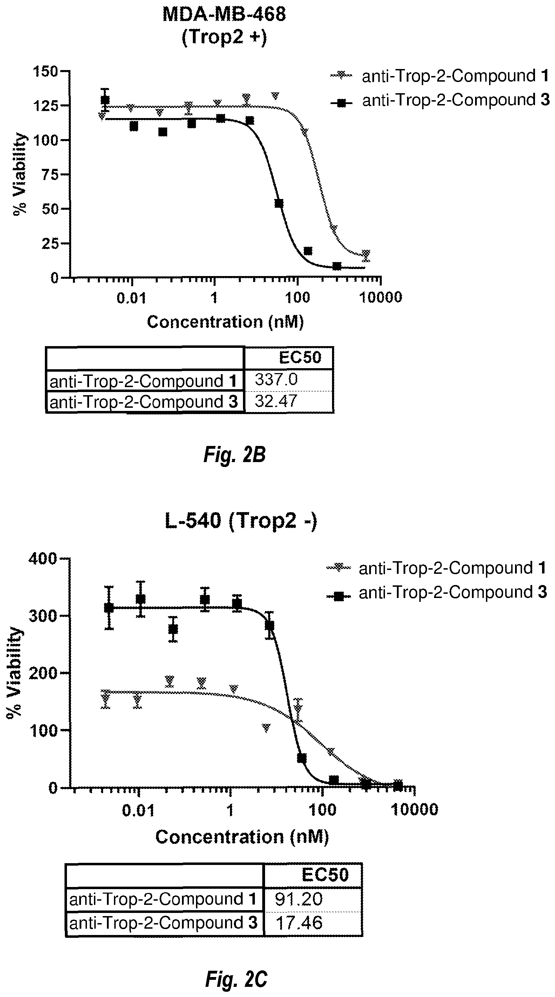

FIG. 2 shows results of an *in vitro* efficacy study of anti-Trop-2-Compound **1** (shown with triangles) and anti-Trop-2-Compound **3** (shown with squares) using: A) BxPC-3 (Trop-2 +) cells; B) MDA-MB-468 (Trop-2 +) cells; and C) L-540 (Trop-2 -) cells.

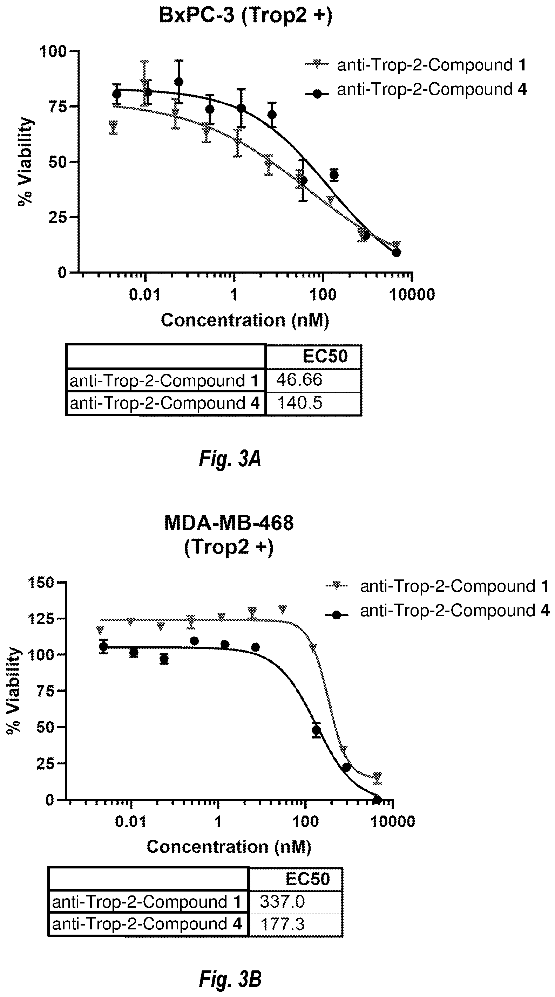

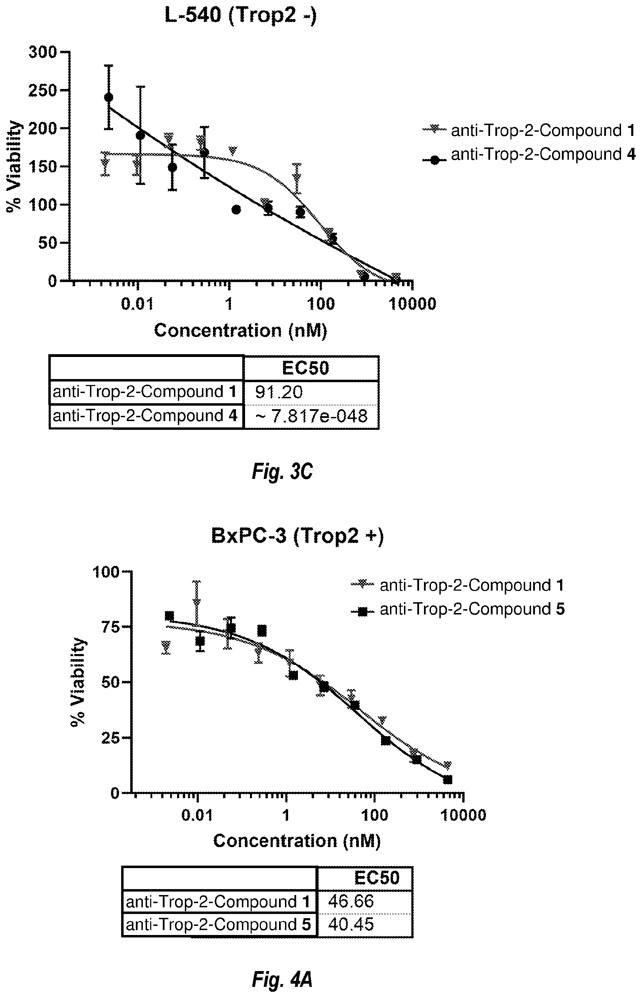

FIG. 3 shows results of an *in vitro* efficacy study of anti-Trop-2-Compound **1** (shown with triangles) and anti-Trop-2-Compound **4** (shown with circles) using: A) BxPC-3 (Trop-2 +) cells; B) MDA-MB-468 (Trop-2 +) cells; and C) L-540 (Trop-2 -) cells.

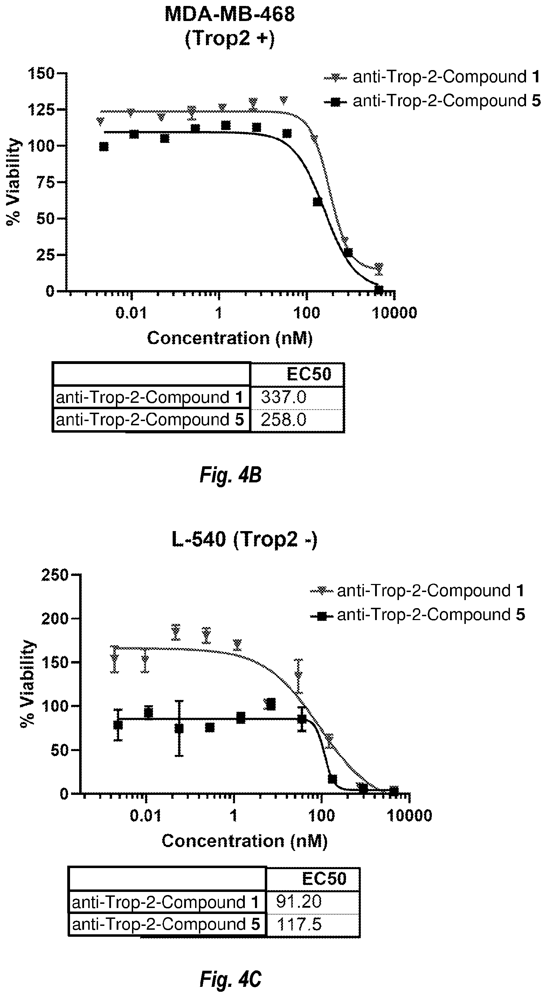

FIG. 4 shows results of an *in vitro* efficacy study of anti-Trop-2-Compound **1** (shown with triangles) and anti-Trop-2-Compound **5** (shown with squares) using: A) BxPC-3 (Trop-2 +) cells; B) MDA-MB-468 (Trop-2 +) cells; and C) L-540 (Trop-2 -) cells.

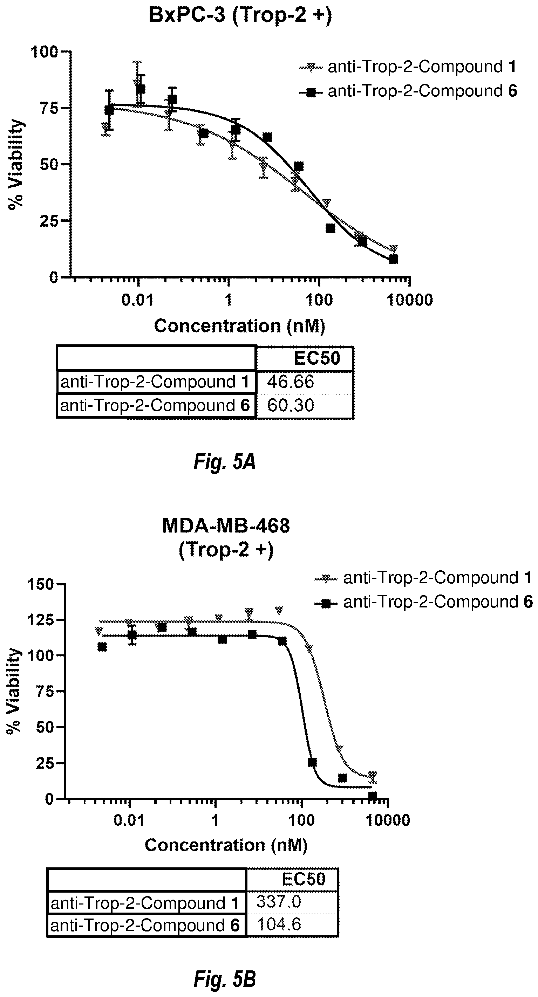

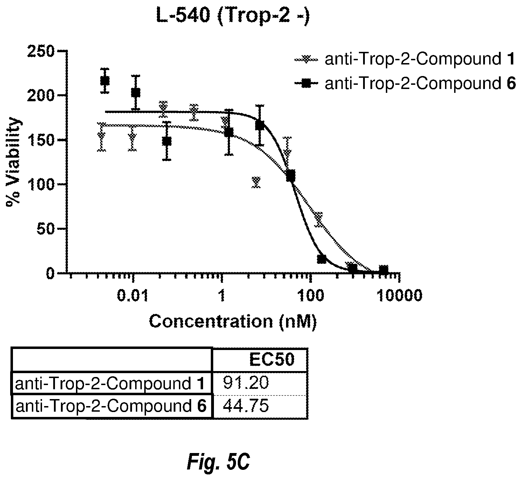

FIG. 5 shows results of an *in vitro* efficacy study of anti-Trop-2-Compound **1** (shown with triangles) and anti-Trop-2-Compound **6** (shown with squares) using: A) BxPC-3 (Trop-2 +) cells; B) MDA-MB-468 (Trop-2 +) cells; and C) L-540 (Trop-2 -) cells.

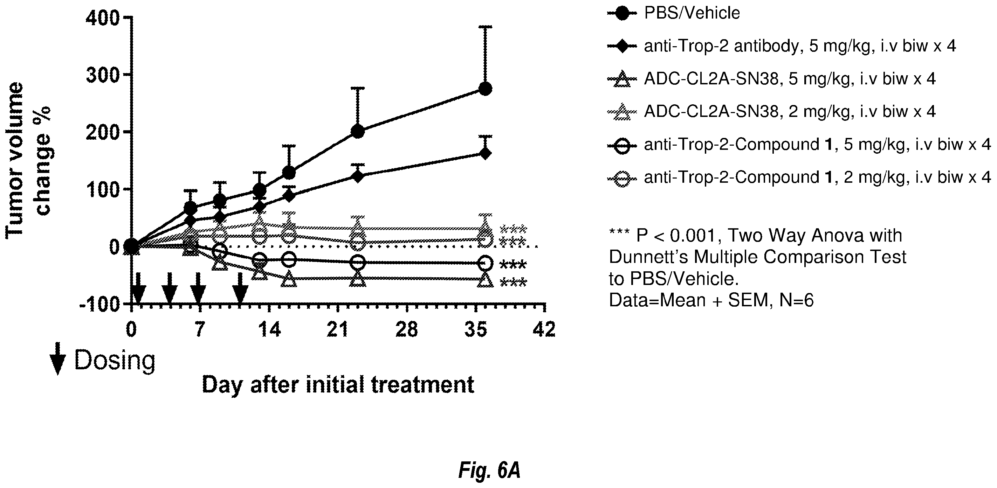

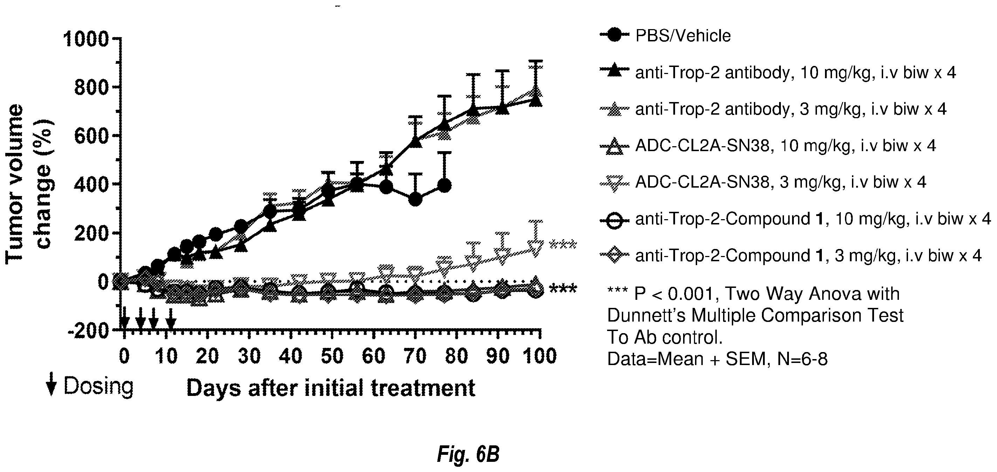

FIG. 6A shows results of an *in vivo* efficacy study in MDA-MB-468 xenograft in nude mice of anti-Trop-2-Compound **1** (2 mg/kg: shown with grey open circles; 5 mg/kg: shown with black open circles) and ADC-CL2A-SN38 (2 mg/kg: shown with grey open triangles; 5 mg/kg: shown with black open triangles). PBS/vehicle (shown with solid circles) and anti-Trop-2 antibody alone (5 mg/kg, shown with solid diamonds) were used as controls. *** P < 0.001, two way ANOVA with Dunnett's multiple comparison test to PBS/vehicle; data = mean + SEM, N = 6. FIG. 6B shows results of an *in vivo* efficacy study of anti-Trop-2-Compound **1** (3 mg/kg: shown with open diamonds; 10 mg/kg: shown with open circles) and ADC-CL2A-SN38 (3 mg/kg: shown with grey open triangles (upside down); 10 mg/kg: shown with black open triangles). PBS/vehicle (shown with solid circles) and anti-Trop-2 antibody alone (3 mg/kg, shown with grey solid triangles; 10 mg/kg: shown with black solid triangles) were used as controls. *** P < 0.001, two way ANOVA with Dunnett's multiple comparison test to antibody control; data = mean + SEM, N = 6-8. The data demonstrate that anti-Trop-2-Compound **1** significantly inhibited MDA-MB-468 xenograft tumor growth in nude mice.

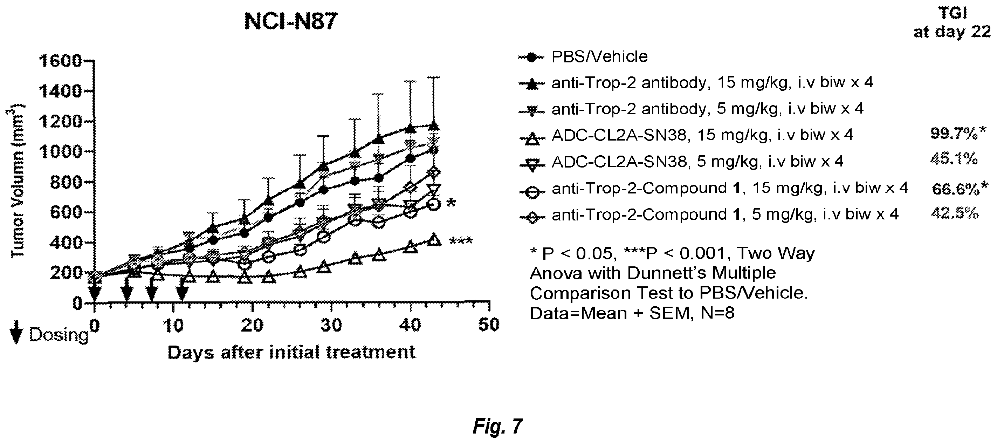

FIG. 7 shows results of an *in vivo* efficacy study in NCI-N87 xenograft in nude mice of anti-Trop-2-Compound **1** (5 mg/kg: shown with open diamonds; 15 mg/kg: shown with open circles) and ADC-CL2A-SN38 (5 mg/kg: shown with grey open triangles (upside down); 15 mg/kg: shown with black open triangles). PBS/vehicle (shown with solid circles) and anti-Trop-2 antibody alone (5 mg/kg, shown with grey solid triangles (upside down); 15 mg/kg, shown with black solid triangles) were used as controls. * P < 0.05, *** P < 0.001, two way ANOVA with Dunnett's multiple comparison test to PBS/vehicle; data = mean + SEM, N = 8. The data demonstrate that anti-Trop-2-Compound **1** significantly inhibited NCI-N87 xenograft tumor growth in nude mice.

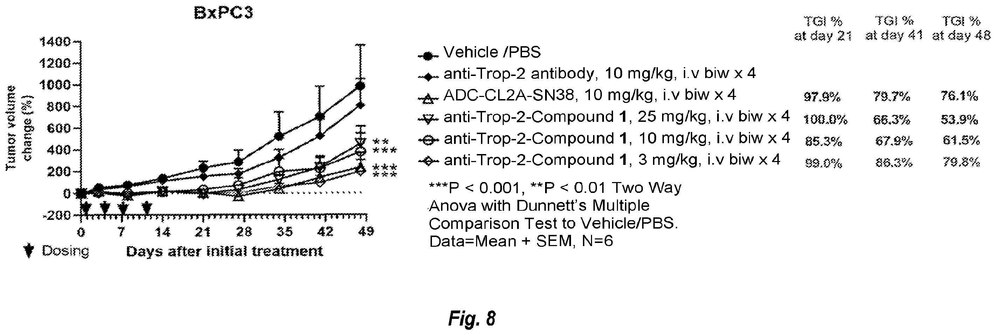

FIG. 8 shows results of an *in vivo* efficacy study in BxPC3 xenograft in nude mice of anti-Trop-2-Compound **1** (3 mg/kg: shown with open diamonds; 10 mg/kg: shown with open circles; 25 mg/kg, shown with open triangles (upside down)) and ADC-CL2A-SN38 (10 mg/kg: shown with open triangles). PBS/vehicle (shown with solid circles) and anti-Trop-2 antibody alone (10 mg/kg, shown with solid diamonds) were used as controls. *** P < 0.001, ** P < 0.01 two way ANOVA with Dunnett's multiple comparison test to PBS/vehicle; data = mean + SEM, N = 6. The data demonstrate that anti-Trop-2-Compound **1** significantly inhibited BxPC3 xenograft tumor growth in nude mice.

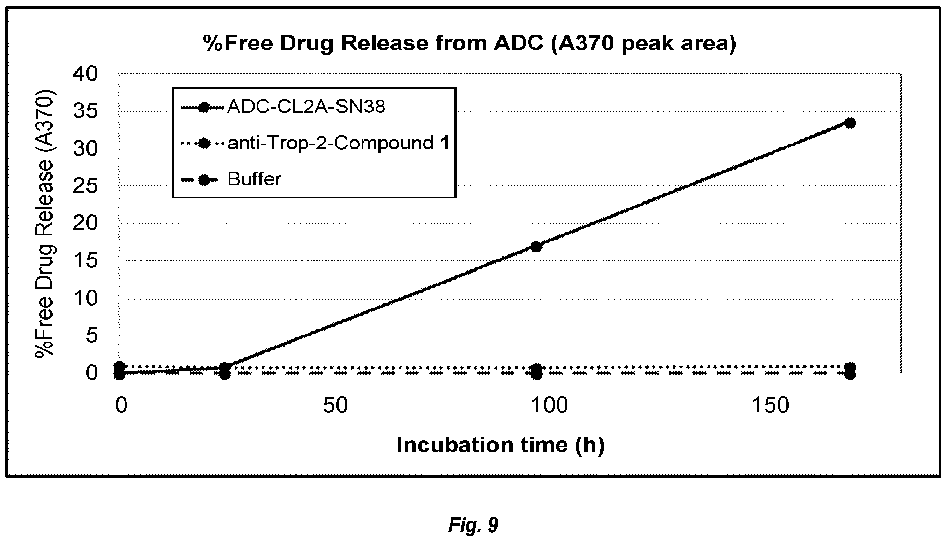

FIG. 9 illustrates the results of a stability study of ADC-CL2A-SN38 and anti-Trop-2-Compound **1** in PBS over a time course of 168 hours. Detection of free drug release was monitored at 370 nm. The data demonstrate that anti-Trop-2-Compound **1** does not release significant amounts of free Compound **1** over this time course and is considerably more stable than ADC-CL2A-SN38.

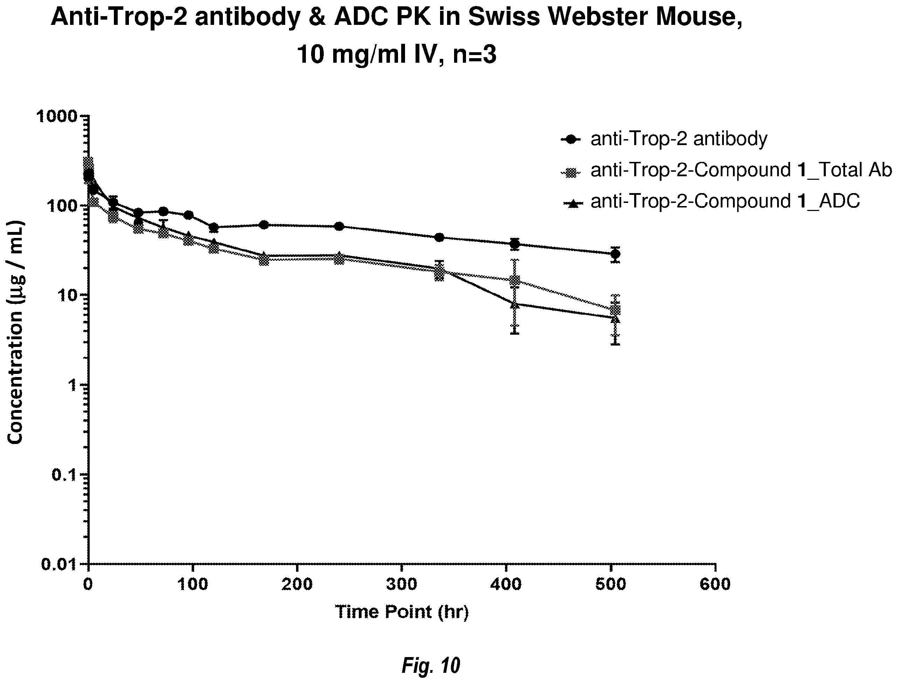

FIG. 10 illustrates a plasma stability study using Swiss Webster mice. The concentration (µg/mL) of unconjugated anti-Trop-2 (shown with circles), total antibody content of the ADC anti-Trop-2-Compound **1** (shown with squares), and ADC anti-Trop-2-Compound **1** (shown with triangles) over a time course of 500 hours indicates that anti-Trop-2-Compound **1** is stable and does not significantly release the drug from the ADC.

### DETAILED DESCRIPTION

This specification describes exemplary embodiments and applications of the disclosure. The disclosure, however, is not limited to these exemplary embodiments and applications or to the manner in which the exemplary embodiments and applications operate or are described herein. The term "or" is used in an inclusive sense, i.e., equivalent to "and/or," unless the context dictates otherwise. It is noted that, as used in this specification and the appended claims, the singular forms "a," "an," and "the," and any singular use of any word, include plural referents unless expressly and unequivocally limited to one referent. As used herein, the terms "comprise," "include," and grammatical variants thereof are intended to be nonlimiting, such that recitation of items in a list is not to the exclusion of other like items that can be substituted or added to the listed items. Section divisions in the specification are provided for the convenience of the reader only and do not limit any combination of elements discussed.

### Definitions

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-Trop-2 antibody" and "an antibody that binds to Trop-2" refer to an antibody that is capable of binding Trop-2 with sufficient affinity such that the antibody is useful as a therapeutic agent in targeting Trop-2. In one embodiment, the extent of binding of an anti-Trop-2 antibody to an unrelated, non-Trop-2 protein is less than about 10% of the binding of the antibody to Trop-2 as measured, e.g., by a radioimmunoassay (RIA). In certain embodiments, an antibody that binds to Trop-2 has a dissociation constant (Kd) of ≤ 1µM, ≤ 100 nM, ≤ 10 nM, , ≤ 5 Nm, , ≤ 4 nM, , ≤ 3 nM, , ≤ 2 nM, ≤ 1 nM, ≤ 0.1 nM, ≤ 0.01 nM, or ≤ 0.001 nM (e.g., 10⁻⁸ M or less, e.g. from 10⁻⁸ M to 10⁻¹³ M, e.g., from 10⁻⁹ M to 10⁻¹³ M). In certain embodiments, an anti-Trop-2 antibody binds to an epitope of Trop-2 that is conserved among Trop-2 from different species.

The term "antibody" is used herein in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody and that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

The terms "cancer" and "cancerous" refer to or describe the physiological condition in mammals that is typically characterized by unregulated cell growth/proliferation. Examples of cancer include, but are not limited to, melanoma, carcinoma, lymphoma (e.g., Hodgkin's and non-Hodgkin's lymphoma), blastoma, sarcoma, and leukemia. Particular nonlimiting examples include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, urethelial cancer, esophageal cancer, vulval cancer, thyroid cancer, hepatic carcinoma, leukemia and other lymphoproliferative disorders, and various types of head and neck cancer.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "cytotoxic agent" as used herein refers to a substance that inhibits or prevents a cellular function and/or causes cell death or destruction. Cytotoxic agents include, but are not limited to, radioactive isotopes (e.g., ²¹¹At, ¹³¹I, ¹²⁵I, ⁹⁰Y, ¹⁸⁶Re, ¹⁸⁸Re, ¹⁵³Sm, ²¹²Bi, ³²P, ²¹²Pb and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" is a chemical compound useful in the treatment of cancer. Examples of chemotherapeutic agents include alkylating agents such as thiotepa and cyclosphosphamide (CYTOXAN^{®}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL^{®}); beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); thiotepa; taxoids, e.g., paclitaxel (TAXOL^{®}; Bristol-Myers Squibb Oncology, Princeton, N.J.), ABRAXANETM Cremophor-free, albumin-engineered nanoparticle formulation of paclitaxel (American Pharmaceutical Partners, Schaumberg, Illinois), and docetaxel (TAXOTERE^{®}; Rhône-Poulenc Rorer, Antony, France); chloranbucil; gemcitabine (GEMZAR^{®}); 6-thioguanine; mercaptopurine; methotrexate; platinum analogs such as cisplatin and carboplatin; vinblastine (VELBAN^{®}); platinum; etoposide (VP-16); ifosfamide; mitoxantrone; vincristine (ONCOVIN^{®}); oxaliplatin; leucovovin; vinorelbine (NAVELBINE^{®}); novantrone; edatrexate; daunomycin; aminopterin; ibandronate; topoisomerase inhibitor RFS 2000; difluoromethylornithine (DMFO); retinoids such as retinoic acid; capecitabine (XELODA^{®}); pharmaceutically acceptable salts, acids or derivatives of any of the above; as well as combinations of two or more of the above such as CHOP, an abbreviation for a combined therapy of cyclophosphamide, doxorubicin, vincristine, and prednisolone; CVP, an abbreviation for a combined therapy of cyclophosphamide, vincristine, and prednisolone; and FOLFOX, an abbreviation for a treatment regimen with oxaliplatin (ELOXATINTM) combined with 5-FU and leucovorin.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "epitope" refers to the particular site on an antigen molecule to which an antibody binds.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., supra. In one embodiment, for the VH, the subgroup is subgroup III as in Kabat et al., supra.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., supra.

An "antibody-drug conjugate" or "ADC" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human. In certain embodiments, the subject is an adult, an adolescent, a child, or an infant. In some embodiments, the terms "individual" or "patient" are used and are intended to be interchangeable with "subject".

The term "Trop-2," as used herein, refers to any native Trop-2 from any vertebrate source, including mammals such as primates (e.g. humans, cynomolgus monkey (cyno)) and rodents (e.g., mice and rats), unless otherwise indicated. The term encompasses "full-length," unprocessed Trop-2 as well as any form of Trop-2 that results from processing in the cell. The term also encompasses naturally occurring variants of Trop-2, e.g., splice variants, allelic variants, and isoforms. The amino acid sequence of an exemplary human Trop-2 protein is shown in SEQ ID NO: 9.

The term "Trop-2-expressing cancer" refers to a cancer comprising cells that express Trop-2 on their surface.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity. Alignment for purposes of determining percent amino acid sequence identity can be achieved in various ways that are within the skill in the art, for instance, using publicly available computer software implementing a suitable algorithm such as the local homology algorithm of Smith and Waterman (Add. APL. Math. 2:482, 1981), by the global homology alignment algorithm of Needleman and Wunsch (J. Mol. Biol. 48:443, 1970). Those skilled in the art can determine appropriate parameters for aligning sequences, including any algorithms needed to achieve maximal alignment over the full length of the sequences being compared. "Percentage of sequence identity" or "percent (%) [sequence] identity," as used herein, is determined by comparing two optimally locally aligned sequences over a comparison window defined by the length of the local alignment between the two sequences. (This may also be considered percentage of homology or "percent (%) homology".) The amino acid sequence in the comparison window may comprise additions or deletions (e.g., gaps or overhangs) as compared to the reference sequence for optimal alignment of the two sequences. Local alignment between two sequences only includes segments of each sequence that are deemed to be sufficiently similar according to a criterion that depends on the algorithm used to perform the alignment. The percentage identity is calculated by determining the number of positions at which the identical nucleic acid base or amino acid residue occurs in both sequences to yield the number of matched positions, dividing the number of matched positions by the total number of positions in the window of comparison and multiplying the result by 100. GAP and BESTFIT, for example, can be employed to determine the optimal alignment of two sequences that have been identified for comparison. Typically, the default values of 5.00 for gap weight and 0.30 for gap weight length are used.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

A "pharmaceutically acceptable salt" refers to a salt that is pharmaceutically acceptable. A compound described herein may be administered as a pharmaceutically acceptable salt.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the ADCs as described herein are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "C₁₋₆ alkyl," as used herein refers to a straight chain or branched, saturated hydrocarbon having from 1 to 6 carbon atoms. Representative straight chain C₁₋₆ alkyl groups include methyl, ethyl, n-propyl, n-butyl, n-pentyl, and n-hexyl; representative branched C₁₋₆ alkyl groups include, but are not limited to, isopropyl, sec-butyl, isobutyl, tert-butyl, isopentyl, 2-methylbutyl. Unless specifically indicated, it is understood that C₁₋₆ alkyl refers to an unsubstituted group.

The term "C₁₋₄ alkyl," as used herein refers to a straight chain or branched, saturated hydrocarbon having from 1 to 4 carbon atoms. Representative "C₁₋₄ alkyl" groups include methyl, ethyl, n-propyl, n-butyl; representative branched C₁₋₄ alkyl groups include, but are not limited to, isopropyl, sec-butyl, isobutyl, tert-butyl. Unless specifically indicated, it is understood that C₁₋₄ alkyl refers to an unsubstituted group.

"Linker" refers to a chemical moiety comprising a covalent bond or a chain of atoms that covalently attaches an antibody to a drug moiety. In various embodiments, linkers include a divalent radical. In various embodiments, linkers can comprise one or more amino acid residues.

The term "protecting group" refers to a substituent that is commonly employed to block or protect a particular functionality while reacting other functional groups on the compound. For example, an "amino-protecting group" is a substituent attached to an amino group that blocks or protects the amino functionality in the compound. Suitable amino-protecting groups include, but are not limited to, acetyl, trifluoroacetyl, t-butoxycarbonyl (BOC), benzyloxycarbonyl (CBZ) and 9-fluorenylmethylenoxycarbonyl (Fmoc). For a general description of protecting groups and their use, see T. W. Greene, Protective Groups in Organic Synthesis, John Wiley & Sons, New York, 1991, or a later edition.

As used herein, "substantially" and other grammatical forms thereof mean sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

### Overview

Antibody-Drug Conjugates (ADCs) allow for the targeted delivery of a drug moiety to a tumor, and, in some embodiments intracellular accumulation therein, where systemic administration of unconjugated drugs may result in unacceptable levels of toxicity to normal cells (Polakis P. (2005) Current Opinion in Pharmacology 5:382-387). ADCs are targeted chemotherapeutic molecules which combine properties of both antibodies and cytotoxic drugs by targeting potent cytotoxic drugs to antigen-expressing tumor cells (Teicher, B.A. (2009) Current Cancer Drug Targets 9:982-1004), thereby enhancing the therapeutic index by maximizing efficacy and minimizing off-target toxicity (Carter, P.J. and Senter P.D. (2008) The Cancer Jour. 14(3):154-169; Chari, R.V. (2008) Acc. Chem. Res. 41:98-107 .

The present disclosure provides ADCs comprising an anti-Trop-2 antibody conjugated to the drug moiety SN-38 through a linker moiety. The anti-Trop-2 antibody can bind to Trop-2-expressing cancer cells and allow for selective uptake of the ADC into the cancer cells. In some embodiments, an ADC provided herein is used to selectively deliver an effective amount of SN-38 to tumor tissue while avoiding the toxicity associated with other ADCs in which different linkers are used to conjugate SN-38 to an anti-Trop-2 antibody. The ADC compounds described herein include those with anticancer activity.

In one aspect, provided herein are antibody-drug conjugates (ADCs) comprising an anti-Trop-2 antibody. In another aspect, provided herein are methods of preparing ADCs comprising an anti-Trop-2 antibody. Also provided herein are methods for treating cancers, such as Trop-2-expressing cancers, using the ADCs disclosed herein.

### I. Compositions

### Antibody-Drug Conjugates

In one aspect, provided herein is an antibody-drug conjugate (ADC) comprising an anti-Trop-2 antibody (Ab), the drug moiety SN-38, and a linker moiety that covalently attaches the anti-Trop-2 antibody to SN-38.

In some embodiments, the ADC is of formula (I): 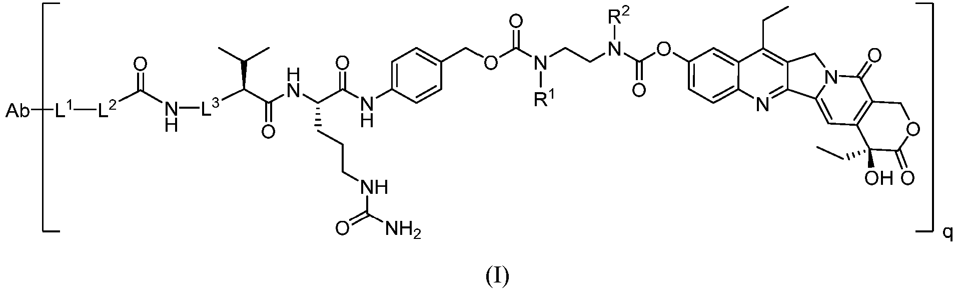 or is a pharmaceutically acceptable salt thereof, wherein:
Ab is an anti-Trop-2 antibody;
q is a value in the range of 1 to 20;
L¹ is a linker bound to the anti-Trop-2 antibody;
L² is -(CH₂)ₚ- where p is 4, 5, 6, 7, or 8;
L³ is a bond or a polyoxyethylene-based divalent linker; and
R¹ and R² are each independently C₁₋₆ alkyl.

In some embodiments, L¹ is a linker bound to a sulfur of the anti-Trop-2 antibody. In some embodiments, L¹ is 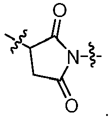 In some embodiments, -L¹-L²- is

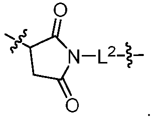

In some embodiments, q is 1, 2, 3, 4, 5, 6, 7, 8, 9, 10, 11, 12, 13, 14, 15, 16, 17, 18, 19, or 20. In some embodiments, q is a value in the range of 1 to 10. In some embodiments, q is a value in the range of 6 to 8. In some embodiments, q is a value in the range of 6 to 7. In some embodiments, q is a value in the range of 7 to 8. In some embodiments, q is 6, 7, or 8. In some embodiments, q is 6. In some embodiments, q is 7. In some embodiments, q is 8.

In some embodiments, p is 4, 5, or 6. In some embodiments, p is 4. In some embodiments, p is 5. In some embodiments, p is 6. In some embodiments, p is 7 or 8. In some embodiments, p is 7. In some embodiments, p is 8.

In some embodiments, L³ is a bond. In other embodiments, L³ is a polyoxyethylene-based divalent linker. In some embodiments, the polyoxyethylene-based divalent linker comprises a polyoxyethylene portion and an alkylene portion. In some embodiments, the polyoxyethylene-based divalent linker comprises a polyoxyethylene portion and an arylene portion. In some embodiments, the polyoxyethylene-based divalent linker comprises a polyoxyethylene portion, an alkylene portion, and an arylene portion. In some embodiments, the polyoxyethylene-based divalent linker comprises a polyoxyethylene portion and an amide portion. In some embodiments, the polyoxyethylene-based divalent linker comprises a polyoxyethylene portion, an alkyl portion, and an amide portion. In some embodiments, the polyoxyethylene-based divalent linker comprises a polyoxyethylene portion, an arylene portion, and an amide portion. In some embodiments, the polyoxyethylene-based divalent linker comprises a polyoxyethylene portion, an alkylene portion, an arylene portion, and an amide portion. In some embodiments, the polyoxyethylene-based divalent linker comprises up to 24 -(CH₂CH₂O)- units.

In some embodiments, R¹ is C₁₋₄ alkyl. In some embodiments, R¹ is C₁₋₃ alkyl. In some embodiments, R¹ is methyl. In some embodiments, R¹ is ethyl. In some embodiments, R¹ is propyl, such as *n*-propyl or *iso-*propyl. In some embodiments, R¹ is butyl, such as *n*-butyl or *tert*-butyl*.* In other embodiments, R¹ is pentyl or hexyl.

In some embodiments, R² is C₁₋₄ alkyl. In some embodiments, R² is C₁₋₃ alkyl. In some embodiments, R² is methyl. In some embodiments, R² is ethyl. In some embodiments, R² is propyl, such as *n*-propyl or *iso*-propyl. In some embodiments, R² is butyl, such as *n*-butyl or *tert*-butyl. In other embodiments, R² is pentyl or hexyl.

In some embodiments, R¹ and R² are identical. In some embodiments, R¹ and R² are each methyl. In some embodiments, R¹ and R² are each ethyl. In some embodiments, R¹ and R² are each propyl. In some embodiments, R¹ and R² are each butyl. In some embodiments, R¹ and R² are each pentyl. In some embodiments, R¹ and R² are each hexyl.

In some embodiments, R¹ and R² are different. In some embodiments, R¹ is methyl and R² is ethyl. In some embodiments, R¹ is ethyl and R² is methyl. In some embodiments, R¹ is methyl and R² is C₂₋₆ alkyl. In some embodiments, R¹ is C₂₋₆ alkyl and R² is methyl.

In some embodiments, the ADC is of formula (IIa): 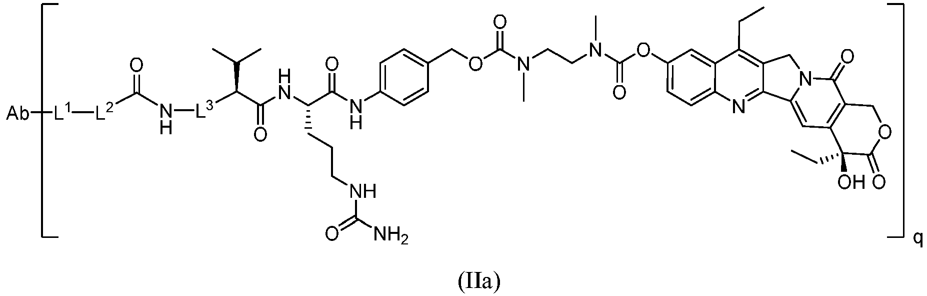 or a pharmaceutically acceptable salt thereof. In one variation, L³ is a bond and the ADC is of formula (IIa-1): 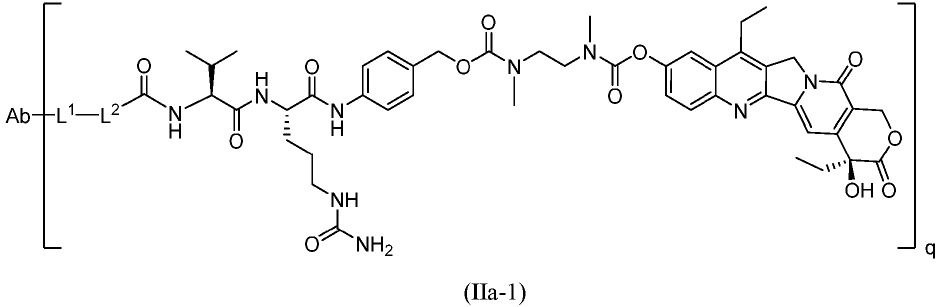 or a pharmaceutically acceptable salt thereof.

In some embodiments, the ADC is of formula (IIb): 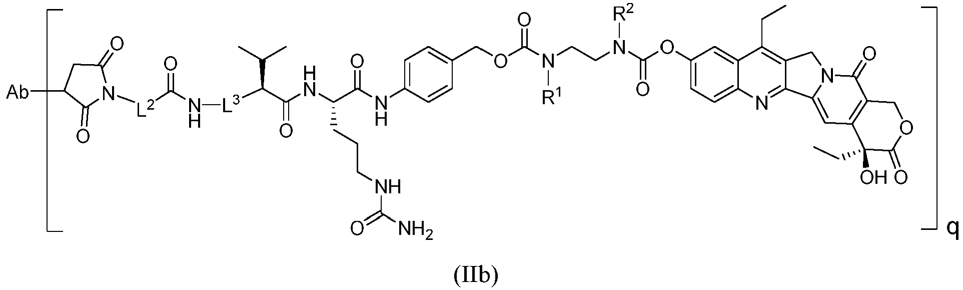 or a pharmaceutically acceptable salt thereof. In one variation, L³ is a bond and the ADC is of formula (IIb-1): 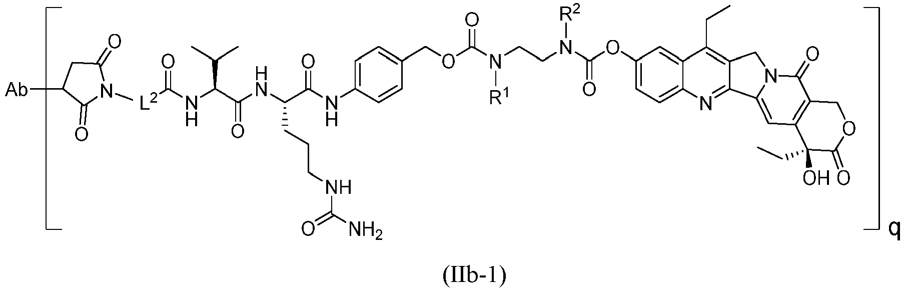 or a pharmaceutically acceptable salt thereof.

In some embodiments, the ADC is of formula (IIc): 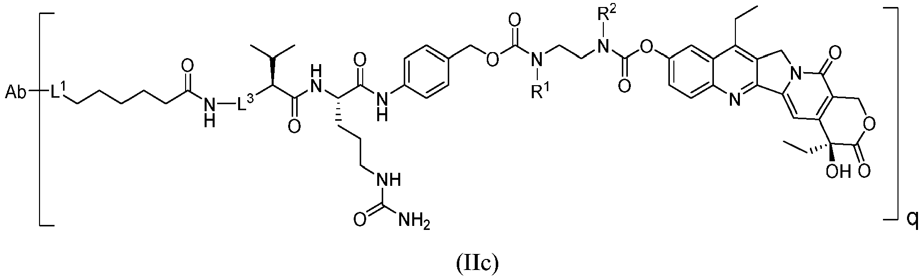 or a pharmaceutically acceptable salt thereof. In one variation, L³ is a bond and the ADC is of formula (IIc-1): 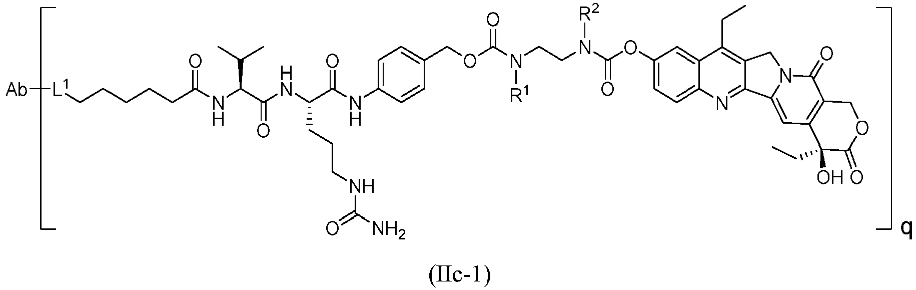 or a pharmaceutically acceptable salt thereof.

In some embodiments, the ADC is of formula (IIIa): 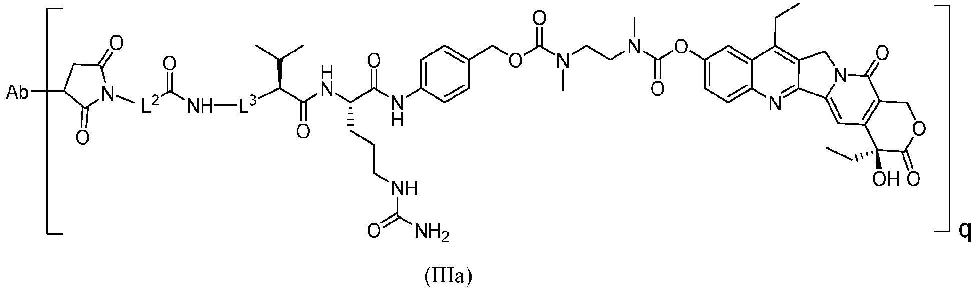 or a pharmaceutically acceptable salt thereof. In one variation, L³ is a bond and the ADC is of formula (IIIa-1): 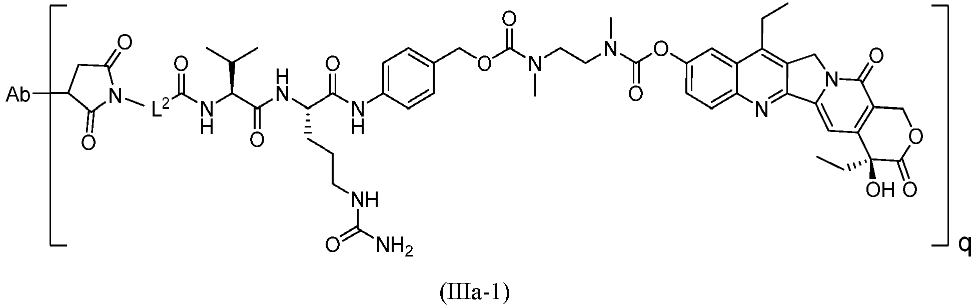 or a pharmaceutically acceptable salt thereof.

In some embodiments, the ADC is of formula (IIIb): 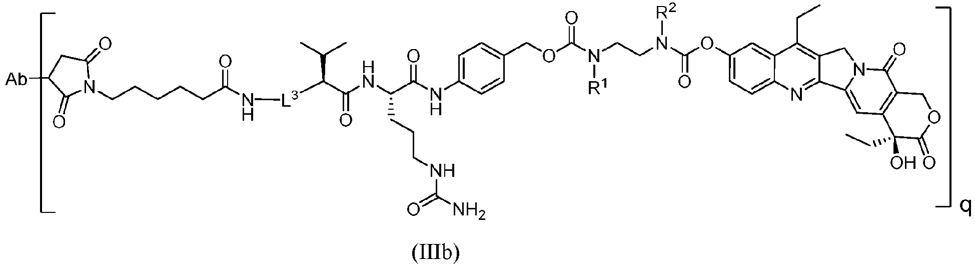 or a pharmaceutically acceptable salt thereof. In one variation, L³ is a bond and the ADC is of formula (IIIb-1): 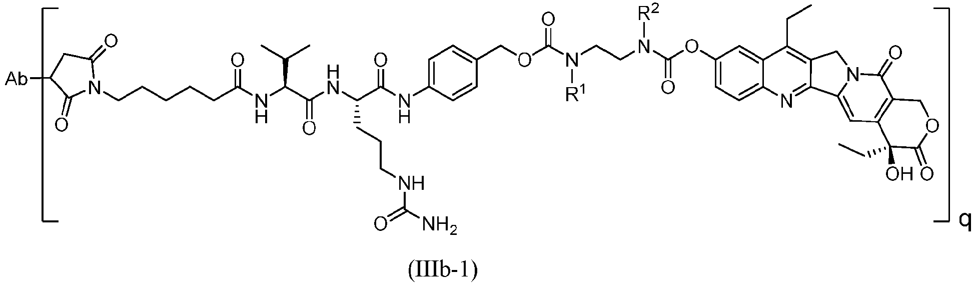 or a pharmaceutically acceptable salt thereof.

In some embodiments, the ADC is of formula (IIIc): 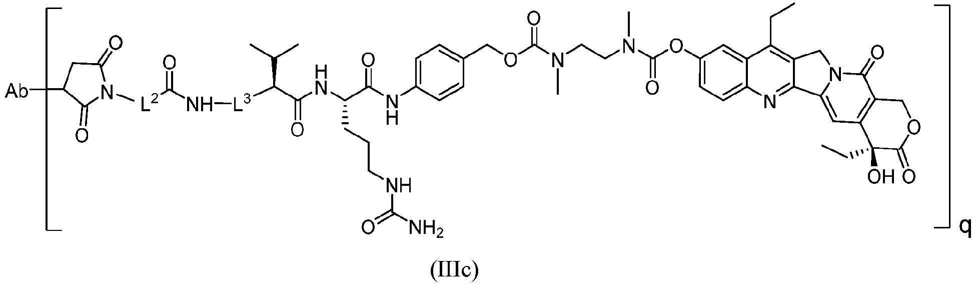 or a pharmaceutically acceptable salt thereof. In one variation, L³ is a bond and the ADC is of formula (IIIc-1): 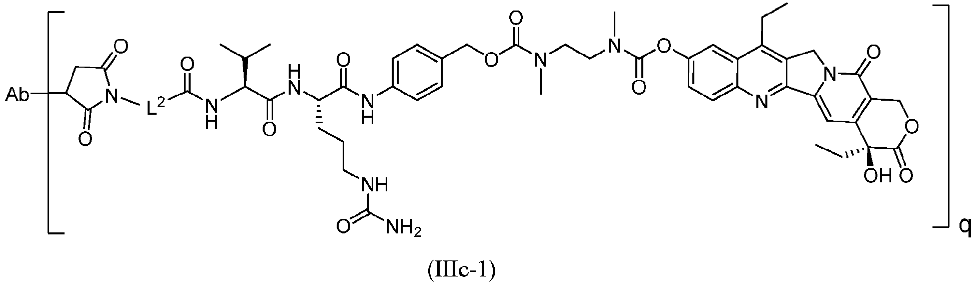 or a pharmaceutically acceptable salt thereof.

In the descriptions herein, it is understood that every description, variation, embodiment, or aspect of a moiety may be combined with every description, variation, embodiment, or aspect of other moieties the same as if each and every combination of descriptions is specifically and individually listed. For example, every description, variation, embodiment, or aspect provided herein with respect to L¹ of formula (I) may be combined with every description, variation, embodiment, or aspect of L², L³, p, R¹, R², Ab, and q the same as if each and every combination were specifically and individually listed. It is also understood that all descriptions, variations, embodiments, or aspects of formula (I), where applicable, apply equally to other formulae detailed herein, and are equally described, the same as if each and every description, variation, embodiment, or aspect were separately and individually listed for all formulae. For example, all descriptions, variations, embodiments, or aspects of formula (I), where applicable, apply equally to any of the formulae as detailed herein, such as formulae (IIa), (IIa-1), (IIb), (IIb-1), (IIc), (IIc-1), (IIIa), (IIIa-1), (IIIb), (IIIb-1), (IIIc), and (IIIc-1), and are equally described, the same as if each and every description, variation, embodiment, or aspect were separately and individually listed for all formulae.

In one embodiment, the ADC is of formula (IV): 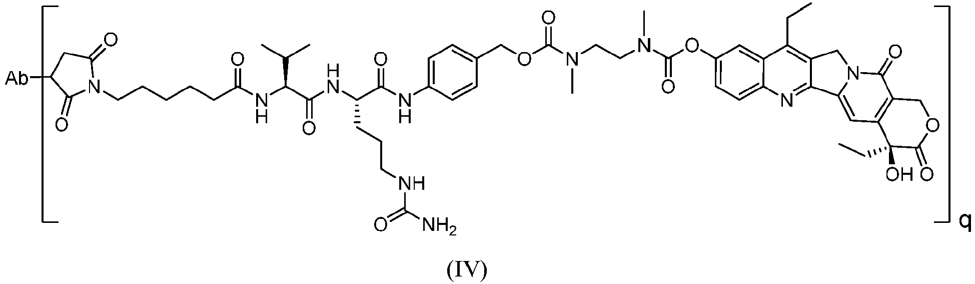 or a pharmaceutically acceptable salt thereof.

### Drug Loading

Drug loading is represented by q, the average number of drug moieties (i.e., SN-38) per anti-Trop-2 antibody in a molecule of formula (I) and variations thereof. Drug loading may range from 1 to 20 drug moieties per antibody. The ADCs of formula (I), and any embodiment, variation, or aspect thereof, include collections of antibodies conjugated with a range of drug moieties, from 1 to 20. The average number of drug moieties per antibody in preparations of ADCs from conjugation reactions may be characterized by conventional means such as mass spectroscopy, ELISA assay, and HPLC. The quantitative distribution of ADCs in terms of q may also be determined. In some instances, separation, purification, and characterization of homogeneous ADCs where q is a certain value from ADCs with other drug loadings may be achieved by means such as reverse phase HPLC or electrophoresis.

For some ADCs, q may be limited by the number of attachment sites on the antibody. For example, where the attachment is a cysteine thiol, as in certain exemplary embodiments described herein, an antibody may have only one or several cysteine thiol groups, or may have only one or several sufficiently reactive thiol groups through which a linker may be attached. In certain embodiments, the average drug loading for an ADC ranges from 1 to about 10, or from about 6 to about 8.

In certain embodiments, fewer than the theoretical maximum of drug moieties are conjugated to an antibody during a conjugation reaction. An antibody may contain, for example, lysine residues that do not react with the drug-linker intermediate or linker reagent. Generally, antibodies do not contain many free and reactive cysteine thiol groups which may be linked to a drug moiety; indeed, most cysteine thiol residues in antibodies exist as disulfide bridges. In certain embodiments, an antibody may be reduced with a reducing agent such as dithiothreitol (DTT) or tricarbonylethylphosphine (TCEP), under partial or total reducing conditions, to generate reactive cysteine thiol groups. In certain embodiments, an antibody is subjected to denaturing conditions to reveal reactive nucleophilic groups such as lysine or cysteine. The loading (drug/antibody ratio or "dar") of an ADC may be controlled in different ways, and for example, by: (i) limiting the molar excess of drug-linker intermediate or linker reagent relative to antibody, (ii) limiting the conjugation reaction time or temperature, and (iii) partial or limiting reductive conditions for cysteine thiol modification.

It is to be understood that where more than one nucleophilic group reacts with a drug-linker intermediate or linker reagent, then the resulting product is a mixture of ADC compounds with a distribution of one or more drug moieties attached to an antibody. The average number of drugs per antibody may be calculated from the mixture by a dual ELISA antibody assay, which is specific for antibody and specific for the drug. Individual ADC molecules may be identified in the mixture by mass spectroscopy and separated by HPLC, e.g. hydrophobic interaction chromatography (*see, e.g.,* McDonagh et al (2006) Prot. Engr. Design & Selection 19(7):299-307; Hamblett et al (2004) Clin. Cancer Res. 10:7063-7070; Hamblett, K.J., et al. "Effect of drug loading on the pharmacology, pharmacokinetics, and toxicity of an anti-CD30 antibody-drug conjugate," Abstract No. 624, American Association for Cancer Research, 2004 Annual Meeting, March 27-31, 2004, Proceedings of the AACR, Volume 45, March 2004; Alley, S.C., et al. "Controlling the location of drug attachment in antibody-drug conjugates," Abstract No. 627, American Association for Cancer Research, 2004 Annual Meeting, March 27-31, 2004, Proceedings of the AACR, Volume 45, March 2004). In certain embodiments, a homogeneous ADC with a single loading value may be isolated from the conjugation mixture by electrophoresis or chromatography.

### anti-Trop-2 Antibodies

### i. Exemplary Antibodies and Antibody Sequences

In some embodiments, the ADC comprises an antibody that binds to Trop-2. Trop-2 has been reported to be upregulated in many cancer types independent of baseline levels of Trop-2 expression. The ADC compounds described herein comprise an anti-Trop-2 antibody.

In some embodiments, the anti-Trop-2 antibody provided herein comprises a cysteine. In some embodiments, the anti-Trop-2 antibody is bound to a drug through the sulfur of a cysteine residue. Exemplary anti-Trop-2 antibodies include any of the hRS7 antibodies, or variations thereof, disclosed in U.S. Patent No. 7,238,785.

In some embodiments, the ADC provided herein comprises an anti-Trop-2 antibody comprising at least one, two, three, four, five, or six HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising at least one HVR selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising at least two HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising at least three HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising at least four HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising at least five HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising at least six HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6.

In some embodiments, the ADC comprises an anti-Trop-2 antibody comprising one HVR selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising two HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising three HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising four HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising five HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6. In some embodiments, the ADC comprises an anti-Trop-2 antibody comprising six HVRs selected from (a) VL HVR1 comprising the sequence of SEQ ID NO: 1; (b) VL HVR2 comprising the sequence of SEQ ID NO: 2; (c) VL HVR3 comprising the sequence of SEQ ID NO: 3; (d) VH HVR1 comprising the sequence of SEQ ID NO: 4; (e) VH HVR2 comprising the sequence of SEQ ID NO: 5; and (f) VH HVR3 comprising the sequence of SEQ ID NO: 6.

In some embodiments, the anti-Trop-2 antibody comprises a VL HVR1 comprising the sequence of SEQ ID NO: 1, a VL HVR2 comprising the sequence of SEQ ID NO: 2, a VL HVR3 comprising the sequence of SEQ ID NO: 3, a VH HVR1 comprising the sequence of SEQ ID NO: 4, a VH HVR2 comprising the sequence of SEQ ID NO: 5, and a VH HVR3 comprising the sequence of SEQ ID NO: 6. In some embodiments, the anti-Trop-2 antibody comprises a VL HVR1 comprising the sequence of SEQ ID NO: 1. In some embodiments, the anti-Trop-2 antibody comprises a VL HVR2 comprising the sequence of SEQ ID NO: 2. In some embodiments, the anti-Trop-2 antibody comprises a VL HVR3 comprising the sequence of SEQ ID NO: 3. In some embodiments, the anti-Trop-2 antibody comprises a VH HVR1 comprising the sequence of SEQ ID NO: 4. In some embodiments, the anti-Trop-2 antibody comprises a VH HVR2 comprising the sequence of SEQ ID NO: 5. In some embodiments, the anti-Trop-2 antibody comprises and a VH HVR3 comprising the sequence of SEQ ID NO: 6.

In some embodiments, the anti-Trop-2 antibody comprises a VL having a sequence with at least 95%, 96%, 97%, 98%, or 99% identity to SEQ ID NO: 7. In some embodiments, the anti-Trop-2 antibody comprises a VL having the sequence of SEQ ID NO: 7. In certain embodiments, a VL sequence having at least 95%, 96%, 97%, 98%, or 99% identity to SEQ ID NO: 7 contains substitutions (e.g., conservative substitutions), insertions, or deletions relative to the reference sequence, but an anti-Trop-2 antibody comprising that sequence retains the ability to bind to Trop-2. In certain embodiments, a total of 1 to 10 amino acids have been substituted, inserted and/or deleted in SEQ ID NO: 7. In certain embodiments, a total of 1 to 5 amino acids have been substituted, inserted and/or deleted in SEQ ID NO: 7. In certain embodiments, substitutions, insertions, or deletions occur in regions outside the HVRs (i.e., in the FRs). In some embodiments, the anti-Trop-2 antibody comprises the VL sequence of SEQ ID NO: 7, and includes post-translational modifications of that sequence.

In some embodiments, the anti-Trop-2 antibody comprises a VH having a sequence with at least 95%, 96%, 97%, 98%, or 99% identity to SEQ ID NO: 8. In some embodiments, the anti-Trop-2 antibody comprises a VH having the sequence of SEQ ID NO: 8. In certain embodiments, a VH sequence having at least 95%, 96%, 97%, 98%, or 99% identity to SEQ ID NO: 8 contains substitutions (e.g., conservative substitutions), insertions, or deletions relative to the reference sequence, but an anti-Trop-2 antibody comprising that sequence retains the ability to bind to Trop-2. In certain embodiments, a total of 1 to 10 amino acids have been substituted, inserted and/or deleted in SEQ ID NO: 8. In certain embodiments, a total of 1 to 5 amino acids have been substituted, inserted and/or deleted in SEQ ID NO: 8. In certain embodiments, substitutions, insertions, or deletions occur in regions outside the HVRs (i.e., in the FRs). In some embodiments, the anti-Trop-2 antibody comprises the VH sequence of SEQ ID NO: 8, and includes post-translational modifications of that sequence.

In some embodiments, the anti-Trop-2 antibody comprises a kappa light chain. In some embodiments, the anti-Trop-2 antibody is an IgG antibody. In some embodiments, the anti-Trop-2 antibody is an IgG1 antibody.

In some embodiments, an anti-Trop-2 antibody binds a human Trop-2. In some embodiments, the human Trop-2 has the amino acid sequence of SEQ ID NO: 9.

In any of the above embodiments, an anti-Trop-2 antibody is humanized. In one embodiment, an anti-Trop-2 antibody comprises HVRs as in any of the above embodiments, and further comprises a human acceptor framework, e.g. a human immunoglobulin framework or a human consensus framework. In certain embodiments, the human acceptor framework is the human VL kappa 1 (VL_{KI}) framework and/or the VH framework VH_{III}. In some embodiments, a humanized anti-Trop-2 antibody comprises (a) a VL HVR1 comprising the sequence of SEQ ID NO: 1; (b) a VL HVR2 comprising the sequence of SEQ ID NO: 2; (c) a VL HVR3 comprising the sequence of SEQ ID NO: 3; (d) a VH HVR1 comprising the sequence of SEQ ID NO: 4; (e) a VH HVR2 comprising the sequence of SEQ ID NO: 5; and (f) a VH HVR3 comprising the sequence of SEQ ID NO: 6.

In some embodiments, the anti-Trop-2 antibody according is a monoclonal antibody, including a chimeric, humanized, or human antibody. In one embodiment, an anti-Trop-2 antibody is an antibody fragment, e.g., a Fv, Fab, Fab', scFv, diabody, or F(ab')₂ fragment. In another embodiment, the antibody is a substantially full length antibody, e.g., an IgG1 antibody or other antibody class or isotype as defined herein.

### ii. Antibody Affinity

In some embodiments, an anti-Trop-2 antibody provided herein binds a human Trop-2 with an affinity of ≤ 10 nM, or ≤ 5 nM, or ≤ 4 nM, or ≤ 3 nM, or ≤ 2 nM. In some embodiments, an anti-Trop-2 antibody binds a human Trop-2 with an affinity of ≥ 0.0001 nM, or ≥ 0.001 nM, or ≥ 0.01 nM. Standard assays known to the skilled artisan can be used to determine binding affinity. For example, whether an anti-Trop-2 antibody "binds with an affinity of" ≤ 10 nM, or ≤ 5 nM, or ≤ 4 nM, or ≤ 3 nM, or ≤ 2 nM, can be determined using standard Scatchard analysis utilizing a non-linear curve fitting program (see, for example, Munson et al., Anal Biochem, 107: 220-239, 1980).

In some embodiments, the anti-Trop-2 antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM, and optionally is ≥ 10⁻¹³ M. (e.g. 10⁻⁸ M or less, e.g. from 10⁻⁸ M to 10⁻¹³ M, e.g., from 10⁻⁹ M to 10⁻¹³ M).

In some embodiments, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., up to about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20^{®}) in PBS. When the plates have dried, 150 µL/well of scintillant (MICROSCINT-20 TM; Packard) is added, and the plates are counted on a TOPCOUNT TM gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE^{®}-2000 or a BIACORE ^{®}-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µL/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 µL/min. Association rates (kon) and dissociation rates (koff) are calculated using a simple one-to-one Langmuir binding model (BIACORE ^{®} Evaluation Software version 3.2) by simultaneously fitting the association and dissociation sensorgrams. The equilibrium dissociation constant (Kd) is calculated as the ratio koff/kon. See, e.g., Chen et al., J. Mol. Biol. 293:865-881 (1999). If the on-rate exceeds 106 M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO TM spectrophotometer (ThermoSpectronic) with a stirred cuvette.

### iii. Antibody Fragments

In certain embodiments, the anti-Trop-2 antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E*. *coli* or phage), as described herein.

### iv. Chimeric and Humanized Antibodies

In certain embodiments, the anti-Trop-2 antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall' Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### v. Human Antibodies

In certain embodiments, the anti-Trop-2 antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE^{™} technology; U.S. Patent No. 5,770,429 describing HUMAB^{®} technology; U.S. Patent No. 7,041,870 describing K-M MOUSE^{®} technology, and U.S. Patent Application Publication No. US 2007/0061900, describing VELOCIMOUSE^{®} technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### vi. Library-Derived Antibodies

In certain embodiments, the anti-Trop-2 antibody provided herein is derived from an antibody library. Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### vii. Multispecific Antibodies

In certain embodiments, the anti-Trop-2 antibody provided herein is a multispecific antibody, e.g. a bispecific antibody. Multispecific antibodies are monoclonal antibodies that have binding specificities for at least two different sites. In certain embodiments, one of the binding specificities is for Trop-2 and the other is for any other antigen. In certain embodiments, bispecific antibodies may bind to two different epitopes of Trop-2. Bispecific antibodies may also be used to localize cytotoxic agents to cells which express Trop-2. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); crosslinking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to Trop-2 as well as another, different antigen (see, US 2008/0069820, for example).

### viii. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, the anti-Trop-2 antibody provided herein has one or more amino acid substitutions. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**Table 1. Exemplary Amino acid substitutions.**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.
Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex is used to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation Variants

In certain embodiments, an anti-Trop-2 antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibody variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.)*.* Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc Region Variants

In certain embodiments, one or more amino acid modifications may be introduced into the Fc region of an anti-Trop-2 antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### ix. Antibody Derivatives

**In** certain embodiments, an anti-Trop-2 antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. **In** general, the number and/or type of polymers used for derivatization can be determined based on considerations including, but not limited to, the particular properties or functions of the antibody to be improved, whether the antibody derivative will be used in a therapy under defined conditions, etc.

### x. Recombinant Methods and Compositions

Antibodies may be produced using recombinant methods and compositions, e.g., as described in U.S. Patent No. 4,816,567. One skilled in the art will be familiar with suitable host cells for antibody expression. Exemplary host cells include eukaryotic cells, e.g. a Chinese Hamster Ovary (CHO) cell or lymphoid cell (e.g., Y0, NS0, Sp20 cell).

For recombinant production of an anti-Trop-2 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E*. *coli.)* After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### xi. Assays

Anti-Trop-2 antibodies described herein may be identified, screened for, or characterized for their physical/chemical properties and/or biological activities by various assays known in the art.

In one aspect, an antibody is tested for its antigen binding activity, e.g., by known methods such as ELISA, BIACore^{®}, FACS, or Western blot.

In another aspect, competition assays may be used to identify an antibody that competes with any of the antibodies described herein for binding to Trop-2. In certain embodiments, such a competing antibody binds to the same epitope (e.g., a linear or a conformational epitope) that is bound by an antibody described herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized Trop-2 is incubated in a solution comprising a first labeled antibody that binds to Trop-2 and a second unlabeled antibody that is being tested for its ability to compete with the first antibody for binding to Trop-2. The second antibody may be present in a hybridoma supernatant. As a control, immobilized Trop-2 is incubated in a solution comprising the first labeled antibody but not the second unlabeled antibody. After incubation under conditions permissive for binding of the first antibody to Trop-2, excess unbound antibody is removed, and the amount of label associated with immobilized Trop-2 is measured. If the amount of label associated with immobilized Trop-2 is substantially reduced in the test sample relative to the control sample, then that indicates that the second antibody is competing with the first antibody for binding to Trop-2. In certain embodiments, immobilized Trop-2 is present on the surface of a cell or in a membrane preparation obtained from a cell expressing Trop-2 on its surface. *See* Harlow and Lane (1988) Antibodies: A Laboratory Manual ch.14 (Cold Spring Harbor Laboratory, Cold Spring Harbor, NY).

### II. Methods of Preparing Antibody-Drug Conjugates

An ADC of formula (I) may be prepared by several routes employing organic chemistry reactions, conditions, and reagents known to those skilled in the art, including: (1) reaction of a nucleophilic group of an antibody with a bivalent linker reagent (L) to form Ab-L via a covalent bond, followed by reaction with a drug moiety (i.e., SN-38 moiety); and (2) reaction of a nucleophilic group of a drug moiety D (i.e., SN-38 moiety) with a bivalent linker reagent (L) to form D-L via a covalent bond, followed by reaction with a nucleophilic group of an antibody. Exemplary methods for preparing an ADC via the latter route are described in U.S. Patent No. 7,498,298.

Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; and (iii) aldehydes, ketones, carboxyl, and maleimide groups. In addition to NHS esters, functional groups used for conjugation to cell surface lysines can include, as non-limiting examples, pentafluorophenyl, tetrafluorophenyl, tetrafluorobenzenesulfonate, nitrophenyl, isocyanate, isothiocyanate, and sulfonylchloride.

Certain antibodies have reducible interchain disulfides, i.e. cysteine bridges. Antibodies may be made reactive for conjugation with linker reagents by treatment with a reducing agent such as DTT (dithiothreitol) or tricarbonylethylphosphine (TCEP), such that the antibody is fully or partially reduced. Each cysteine bridge will thus form, theoretically, two reactive thiol nucleophiles. Additional nucleophilic groups can be introduced into antibodies through modification of lysine residues, e.g., by reacting lysine residues with 2-iminothiolane (Traut's reagent), resulting in conversion of an amine into a thiol. Reactive thiol groups may also be introduced into an antibody by introducing one, two, three, four, or more cysteine residues (e.g., by preparing variant antibodies comprising one or more non-native cysteine amino acid residues). Nonlimiting examples of functional groups that can react with reactive thiols include, without limitation, maleimide, pyridyldithio, bromoacetyl, iodoacetyl, bromobenzyl, iodobenzyl, and 4-(cyanoethynyl)benzoyl.

The ADCs described herein may also be produced by reaction between an electrophilic group on an antibody, such as an aldehyde or ketone carbonyl group, with a nucleophilic group on a linker reagent or drug. Useful nucleophilic groups on a linker reagent include, but are not limited to, hydrazide, oxime, amino, hydrazine, thiosemicarbazone, hydrazine carboxylate, and arylhydrazide. In one embodiment, an antibody is modified to introduce electrophilic moieties that are capable of reacting with nucleophilic substituents on the linker reagent or drug. In another embodiment, the sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either galactose oxidase or sodium meta-periodate may yield carbonyl (aldehyde and ketone) groups in the antibody that can react with appropriate groups on the drug (Hermanson, Bioconjugate Techniques). In another embodiment, antibodies containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such an aldehyde can be reacted with a drug moiety or linker nucleophile.

Exemplary nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

In yet another embodiment, an antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a drug or radionucleotide).

In one aspect, an ADC of formula (I) can be prepared by reacting an anti-Trop-2 antibody (Ab) with a molecule of formula (P-I): 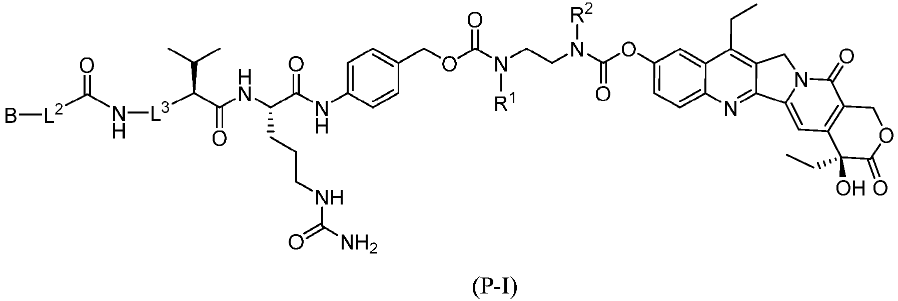 or a pharmaceutically acceptable salt thereof, wherein:
B is a reactive moiety capable of forming a bond with the anti-Trop-2 antibody;
L² is -(CH₂)ₚ- where p is 4, 5, 6, 7, or 8;
L³ is a bond or a polyoxyethylene-based divalent linker; and
R¹ and R² are each independently C₁₋₆ alkyl.

In some embodiments, B is a reactive moiety capable of forming a bond with a sulfhydryl of the anti-Trop-2 antibody. In some embodiments, B is N-maleimido. In some embodiments, B is 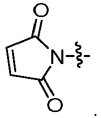 In some embodiments, B-L²- is

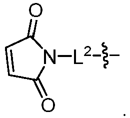

In some embodiments, p is 4, 5, or 6. In some embodiments, p is 4. In some embodiments, p is 5. In some embodiments, p is 6. In some embodiments, p is 7 or 8. In some embodiments, p is 7. In some embodiments, p is 8.

In some embodiments, L³ is a bond. In other embodiments, L³ is a polyoxyethylene-based divalent linker. In some embodiments, the polyoxyethylene-based divalent linker comprises a polyoxyethylene portion and an alkylene portion. In some embodiments, the polyoxyethylene-based divalent linker comprises a polyoxyethylene portion and an arylene portion. In some embodiments, the polyoxyethylene-based divalent linker comprises a polyoxyethylene portion, an alkylene portion, and an arylene portion. In some embodiments, the polyoxyethylene-based divalent linker comprises a polyoxyethylene portion and an amide portion. In some embodiments, the polyoxyethylene-based divalent linker comprises a polyoxyethylene portion, an alkyl portion, and an amide portion. In some embodiments, the polyoxyethylene-based divalent linker comprises a polyoxyethylene portion, an arylene portion, and an amide portion. In some embodiments, the polyoxyethylene-based divalent linker comprises a polyoxyethylene portion, an alkylene portion, an arylene portion, and an amide portion. In some embodiments, the polyoxyethylene-based divalent linker comprises up to 24 -(CH₂CH₂O)- units.

In some embodiments, R¹ is C₁₋₄ alkyl. In some embodiments, R¹ is C₁₋₃ alkyl. In some embodiments, R¹ is methyl. In some embodiments, R¹ is ethyl. In some embodiments, R¹ is propyl, such as n-propyl or *iso-p*ropyl. In some embodiments, R¹ is butyl, such as *n*-butyl or *tert*-butyl*.* In other embodiments, R¹ is pentyl or hexyl.

In some embodiments, R² is C₁₋₄ alkyl. In some embodiments, R² is C₁₋₃ alkyl. In some embodiments, R² is methyl. In some embodiments, R² is ethyl. In some embodiments, R² is propyl, such as n-propyl or iso-propyl. In some embodiments, R² is butyl, such as n-butyl or *tert*-butyl*.* In other embodiments, R² is pentyl or hexyl.

In some embodiments, R¹ and R² are identical. In some embodiments, R¹ and R² are each methyl. In some embodiments, R¹ and R² are each ethyl. In some embodiments, R¹ and R² are each propyl. In some embodiments, R¹ and R² are each butyl. In some embodiments, R¹ and R² are each pentyl. In some embodiments, R¹ and R² are each hexyl.

In some embodiments, R¹ and R² are different. In some embodiments, R¹ is methyl and R² is ethyl. In some embodiments, R¹ is ethyl and R² is methyl. In some embodiments, R¹ is methyl and R² is C₂₋₆ alkyl. In some embodiments, R¹ is C₂₋₆ alkyl and R² is methyl.

In some embodiments, the molecule of formula (P-I) is a molecule of formula formula (P-IIa): 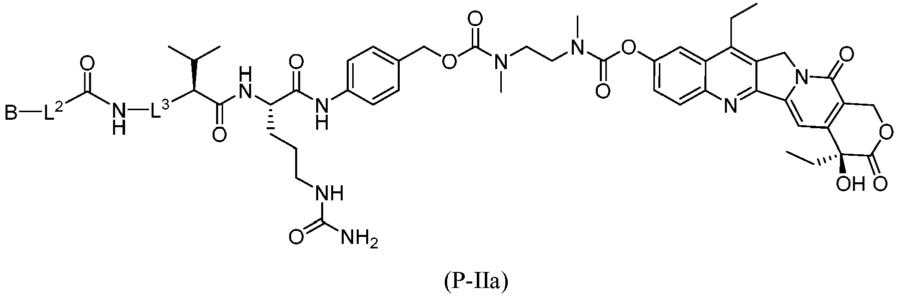 or a pharmaceutically acceptable salt thereof. In one variation, L³ is a bond and the molecule is of formula (P-Ha-1): 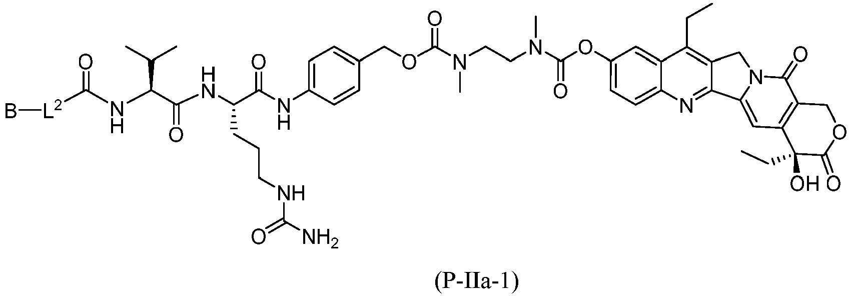 or a pharmaceutically acceptable salt thereof.

In some embodiments, the molecule is of formula (P-IIb): 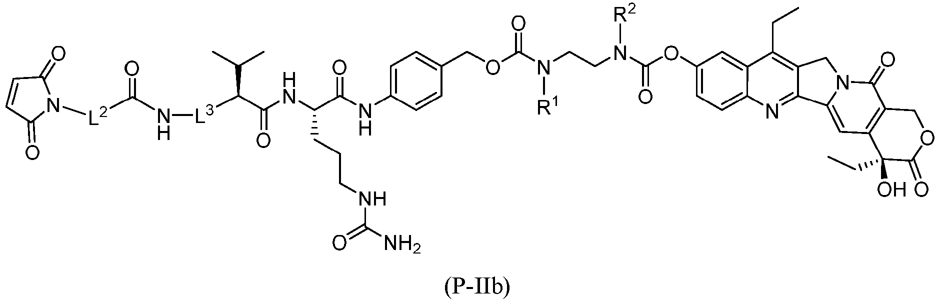 or a pharmaceutically acceptable salt thereof. In one variation, L³ is a bond and the molecule is of formula (P-IIb-1): 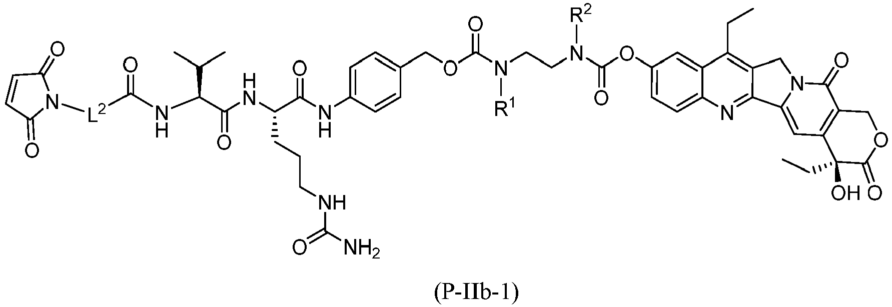 or a pharmaceutically acceptable salt thereof.

In some embodiments, the molecule is of formula (P-IIc): 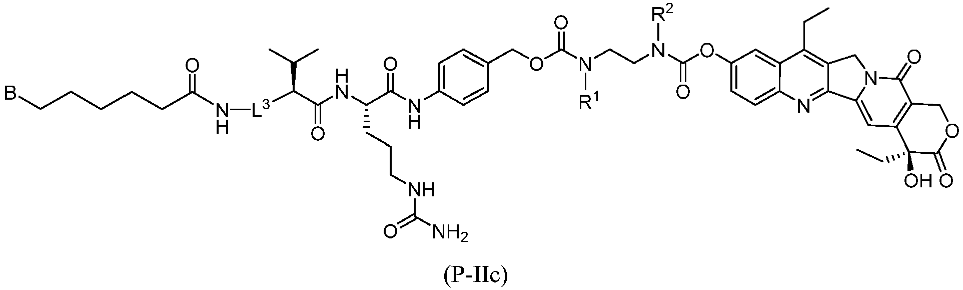 or a pharmaceutically acceptable salt thereof. In one variation, L³ is a bond and the molecule is of formula (P-IIc-1): 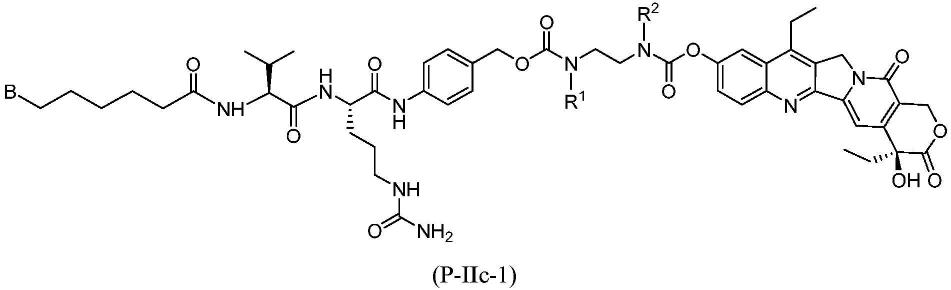 or a pharmaceutically acceptable salt thereof.

In some embodiments, the molecule is of formula (P-IIIa): 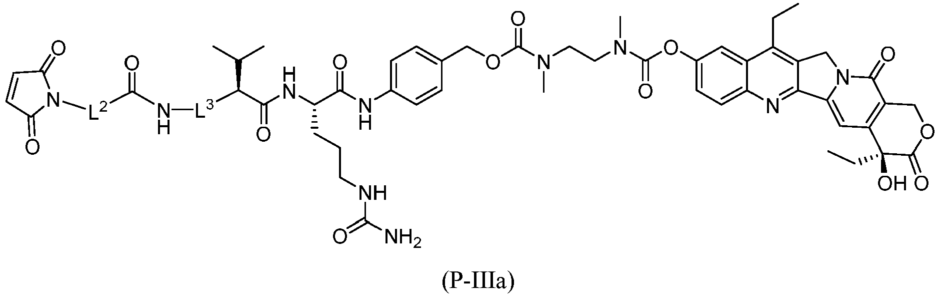 or a pharmaceutically acceptable salt thereof. In one variation, L³ is a bond and the molecule is of formula (P-IIIa-1): 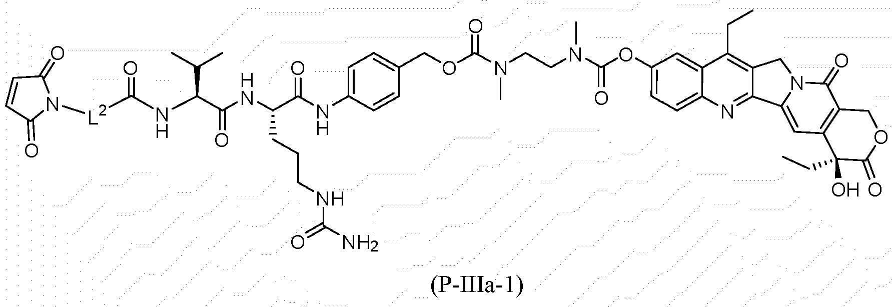 or a pharmaceutically acceptable salt thereof.

In some embodiments, the molecule is of formula (P-IIIb): 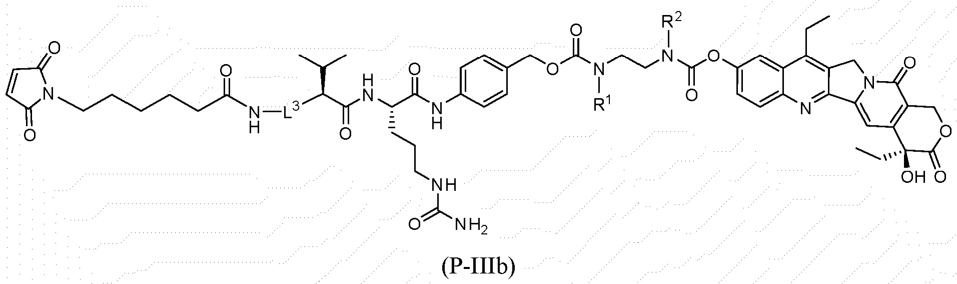 or a pharmaceutically acceptable salt thereof. In one variation, L³ is a bond and the molecule is of formula (P-IIIb-1): 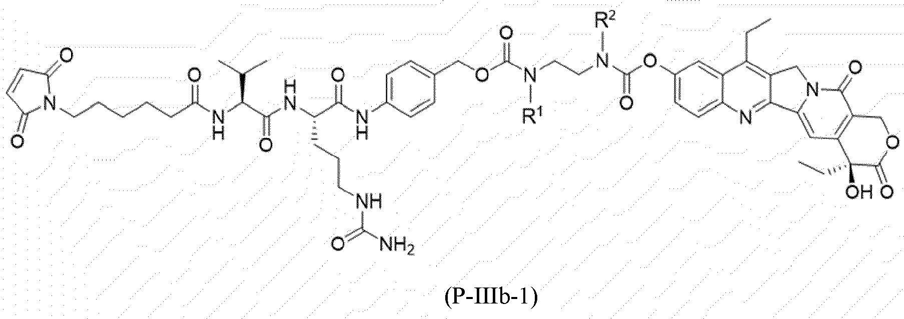 or a pharmaceutically acceptable salt thereof.

In some embodiments, the molecule is of formula (P-IIIc): 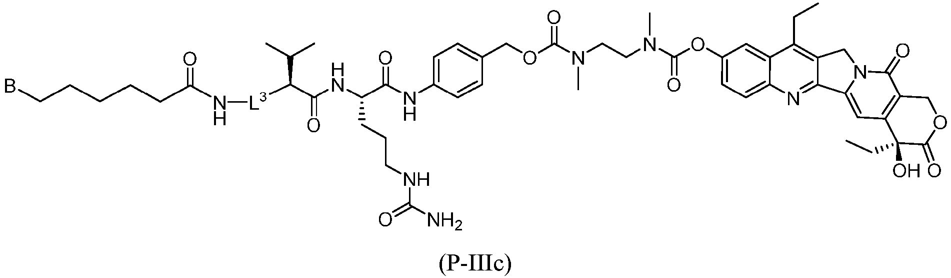 or a pharmaceutically acceptable salt thereof. In one variation, L³ is a bond and the molecule is of formula (P-IIIc-1): 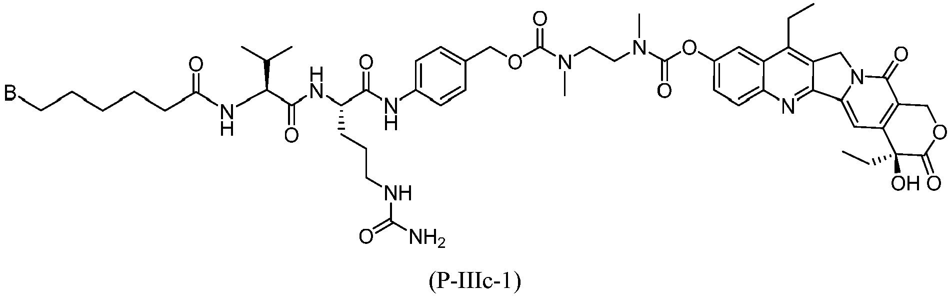 or a pharmaceutically acceptable salt thereof.

In some embodiments, the molecule is of formula (P-IV): 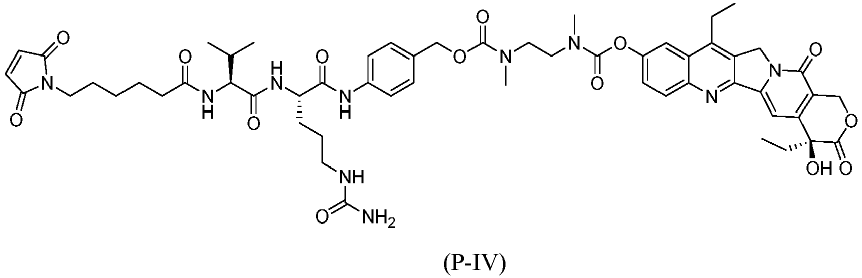 or a pharmaceutically acceptable salt thereof.

In the preparation methods described above, it is understood that every description, variation, embodiment, or aspect of a moiety may be combined with every description, variation, embodiment, or aspect of other moieties the same as if each and every combination of descriptions is specifically and individually listed. For example, every description, variation, embodiment, or aspect provided herein with respect to L² of formula (P-I) may be combined with every description, variation, embodiment, or aspect of L³, p, R¹, R², and B the same as if each and every combination were specifically and individually listed. It is also understood that all descriptions, variations, embodiments, or aspects of formula (P-I), where applicable, apply equally to other formulae detailed herein, and are equally described, the same as if each and every description, variation, embodiment, or aspect were separately and individually listed for all formulae. For example, all descriptions, variations, embodiments, or aspects of formula (P-I), where applicable, apply equally to any of formulae as detailed herein, such as formulae (P-IIa), (P-IIa-1), (P-IIb), (P-IIb-1), (P-IIc), (P-IIc-1), (P-IIIa), (P-IIIa-1), (P-IIIb), (P-IIIb-1), (P-IIIc), and (P-IIIc-1), and are equally described, the same as if each and every description, variation, embodiment, or aspect were separately and individually listed for all formulae.

### III. Pharmaceutical Formulations

Pharmaceutical formulations of the ADCs described herein are prepared by mixing such ADC having the desired degree of purity with one or more optional pharmaceutically acceptable carriers (Remington's Pharmaceutical Sciences 16th edition, Osol, A. Ed. (1980)) in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized ADC formulations are described in U.S. Patent No. 6,267,958. Aqueous ADC formulations include those described in U.S. Patent No. 6,171,586 and WO2006/044908, the latter formulations including a histidine-acetate buffer.

The formulation provided herein may also contain more than one active ingredient as necessary for the particular indication being treated, preferably those with complementary activities that do not adversely affect each other.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the ADC, which matrices are in the form of shaped articles, e.g. films, or microcapsules.

The formulations to be used for *in vivo* administration are generally sterile. Sterility may be readily accomplished, e.g., by filtration through sterile filtration membranes.

### IV. Therapeutic Methods and Compositions

Any of the ADCs provided herein may be used in methods, e.g., therapeutic methods.

In one aspect, an ADC provided herein is used in a method of inhibiting proliferation of a Trop-2-expressing cell, the method comprising exposing the cell to the ADC under conditions permissive for binding of the anti-Trop-2 antibody of the ADC on the surface of the cell, thereby inhibiting the proliferation of the cell. In certain embodiments, the method is an *in vitro* or an *in vivo* method. In some embodiments, the cell is a B cell. In some embodiments, the cell is a neoplastic B cell, such as a lymphoma cell or a leukemia cell.

Inhibition of cell proliferation in vitro may be assayed using the CellTiter-Glo^{™} Luminescent Cell Viability Assay, which is commercially available from Promega (Madison, WI). That assay determines the number of viable cells in culture based on quantitation of ATP present, which is an indication of metabolically active cells. *See* Crouch et al. (1993) J. Immunol. Meth. 160:81-88, US Pat. No. 6602677. The assay may be conducted in 96- or 384-well format, making it amenable to automated high-throughput screening (HTS). *See* Cree et al. (1995) AntiCancer Drugs 6:398-404. The assay procedure involves adding a single reagent (CellTiter-Glo^{®} Reagent) directly to cultured cells. This results in cell lysis and generation of a luminescent signal produced by a luciferase reaction. The luminescent signal is proportional to the amount of ATP present, which is directly proportional to the number of viable cells present in culture. Data can be recorded by luminometer or CCD camera imaging device. The luminescence output is expressed as relative light units (RLU).

In another aspect, an ADC for use as a medicament is provided. In further aspects, an ADC for use in a method of treatment is provided. In certain embodiments, an ADC for use in treating cancer is provided. In some embodiments, the cancer is associated with overexpression of Trop-2. In certain embodiments, provided herein is an ADC for use in a method of treating an individual having a Trop-2-expressing cancer, the method comprising administering to the individual an effective amount of the ADC. In one such embodiment, the method further comprises administering to the individual an effective amount of at least one additional therapeutic agent, e.g., as described below.

In a further aspect, the present disclosure provides for the use of an ADC in the manufacture or preparation of a medicament. In one embodiment, the medicament is for treatment of Trop-2-expressing cancer. In a further embodiment, the medicament is for use in a method of treating Trop-2-expressing cancer, the method comprising administering to an individual having Trop-2-expressing cancer an effective amount of the medicament. In one such embodiment, the method further comprises administering to the individual an effective amount of at least one additional therapeutic agent, e.g., as described below.

In a further aspect, the invention provides a method for treating Trop-2-expressing cancer. In some embodiments, the Trop-2-expressing cancer is an epithelial-cell-derived cancer. In some embodiments, the Trop-2-expressing cancer is a carcinoma. In some embodiments, the carcinoma is a basal cell carcinoma, a squamous cell carcinoma, a renal cell carcinoma, a ductal carcinoma in situ, an invasive ductal carcinoma, or an adenocarcinoma. In some embodiments, the Trop-2-expressing cancer comprises a solid tumor. In some embodiments, the Trop-2-expressing cancer is metastatic. In some embodiments, the Trop-2-expressing cancer a relapsed cancer.

In some embodiments, the Trop-2-expressing cancer is a pancreatic cancer, a gastric cancer, a breast cancer, a melanoma, a kidney cancer, a colorectal cancer, an endometrial cancer, a prostate cancer, a urothelial cancer, a glioblastoma, a lung cancer, a cervical cancer, an esophageal cancer, or an ovarian cancer. In some embodiments, the Trop-2-expressing cancer is a pancreatic cancer. In some embodiments, the Trop-2-expressing cancer is a gastric cancer. In some embodiments, the Trop-2-expressing cancer is a breast cancer. In some embodiments, the breast cancer is triple-negative breast cancer. In any of these embodiments, the cancer can be a metastatic cancer. In certain embodiments, the cancer is a relapsed cancer.

In some embodiments, a Trop-2 expressing cancer is a cancer that receives an anti-Trop-2 immunohistochemistry (IHC) or in situ hybridization (ISH) score greater than "0," which corresponds to very weak or no staining in >90% of tumor cells. In another embodiment, a Trop-2 expressing cancer expresses Trop-2 at a 1+, 2+ or 3+ level, wherein 1+ corresponds to weak staining in >50% of neoplastic cells, 2+ corresponds to moderate staining in >50% neoplastic cells, and 3+ corresponds to strong staining in >50% of neoplastic cells. In some embodiments, a Trop-2 expressing cancer is a cancer that expresses Trop-2 according to a reverse-transcriptase PCR (RT-PCR) assay that detects Trop-2 mRNA. In some embodiments, the RT-PCR is quantitative RT-PCR.

In some embodiments, methods of treating an individual having a Trop-2 expressing cancer are provided, wherein the Trop-2 expressing cancer is resistant to a first therapeutic. In some embodiments, the method comprises administering to the individual an effective amount of an ADC as described herein. In some embodiments, the Trop-2 expressing cancer is selected from a pancreatic cancer, a gastric cancer, a breast cancer including a triple-negative breast cancer, a cervical cancer, an esophageal cancer, or an ovarian cancer. In some embodiments, the first therapeutic comprises a first cytotoxic agent other than SN-38. In some embodiments, the first therapeutic comprises a first antibody that binds an antigen other than Trop-2. In some embodiments, the first therapeutic is a first ADC comprising a first antibody that binds an antigen other than Trop-2 and a first cytotoxic agent.

An "individual" according to any of the above embodiments may be a human.

In a further aspect, provided herein are pharmaceutical formulations comprising any of the ADCs provided herein, e.g., for use in any of the above therapeutic methods. In one embodiment, a pharmaceutical formulation comprises any of the ADCs provided herein and a pharmaceutically acceptable carrier. In another embodiment, a pharmaceutical formulation comprises any of ADCs provided herein and at least one additional therapeutic agent.

The ADCs described herein can be used either alone or in combination with other agents in a therapy. For instance, an ADC as described herein may be co-administered with at least one additional therapeutic agent. In some embodiments, other therapeutic regimens may be combined with the administration of the ADC including, without limitation, radiation therapy and/or bone marrow and peripheral blood transplants, and/or a cytotoxic agent. In some embodiments, a cytotoxic agent is an agent or a combination of agents such as, for example, cyclophosphamide, hydroxydaunorubicin, adriamycin, doxorubincin, vincristine (Oncovin^{™}), prednisolone, CHOP (combination of cyclophosphamide, doxorubicin, vincristine, and prednisolone), or CVP (combination of cyclophosphamide, vincristine, and prednisolone).

Such combination therapies noted above encompass combined administration (where two or more therapeutic agents are included in the same or separate formulations), and separate administration, in which case, administration of the ADC can occur prior to, simultaneously, and/or following, administration of the additional therapeutic agent and/or adjuvant. The ADCs described herein can also be used in combination with radiation therapy.

An ADC as described herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein. The ADCs of the present disclosclosure would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The ADC need not be, but is optionally formulated with one or more agents currently used to prevent or treat the disorder in question. The effective amount of such other agents depends on the amount of ADC present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an ADC as described herein (when used alone or in combination with one or more other additional therapeutic agents) will depend on the type of disease to be treated, the type of ADC, the severity and course of the disease, whether the ADC is administered for preventive or therapeutic purposes, previous therapy, the patient's clinical history and response to the ADC, and the discretion of the attending physician. The ADC is suitably administered to the patient at one time or over a series of treatments. Depending on the type and severity of the disease, about 1 µg/kg to 15 mg/kg (e.g. 0.1mg/kg-10mg/kg) of ADC can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the ADC would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the antibody). An initial higher loading dose, followed by one or more lower doses may be administered. However, other dosage regimens may be useful. The progress of this therapy is easily monitored by conventional techniques and assays.

### V. Articles of Manufacture

In a further aspect, provided herein is an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the disorder and may have a sterile access port (for example the container may be an intravenous solution bag or a vial having a stopper pierceable by a hypodermic injection needle). At least one active agent in the composition is an ADC as described herein. The label or package insert indicates that the composition is used for treating the condition of choice. Moreover, the article of manufacture may comprise (a) a first container with a composition contained therein, wherein the composition comprises an ADC as described herein; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. "About" indicates a degree of variation that does not substantially affect the properties of the described subject matter, e.g., within 10%, 5%, 2%, or 1%. Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

### EXAMPLES

The following examples are provided to illustrate certain disclosed embodiments and are not to be construed as limiting the scope of this disclosure in any way.

The chemical reactions described in the Examples can be readily adapted to prepare a number of other compounds of the present disclosure, and alternative methods for preparing the compounds of this disclosure are deemed to be within the scope of this disclosure. For example, the synthesis of non-exemplified compounds according to the present disclosure can be successfully performed by modifications apparent to those skilled in the art, e.g., by utilizing other suitable reagents known in the art other than those described, or by making routing modifications of reaction conditions, reagents, and starting materials. Alternatively, other reactions disclosed herein or known in the art will be recognized as having applicability for preparing other compounds of the present disclosure.

The following abbreviations may be relevant for the application.

### Abbreviations

BOC or Boc: tert-butoxycarbonyl
DCM: methylene chloride
DIEA: diisopropylethylamine
DMF: N,N-dimethylformamide
DMSO: dimethyl sulfoxide
FBS: fetal bovine serum
Fmoc: 9-fluorenylmethoxycarbonyl
Fmoc-AAN-PAB-PNP: 9-fluorenylmethyloxycarbonyl-alanyl-alanyl-asparaginyl-(4-aminobenzyl)-(4-nitrophenyl)carbonate
Fmoc-Ala-PAB-PNP: 9-fluorenylmethyloxycarbonyl-alanyl-(4-aminobenzyl)-(4-nitrophenyl) carbonate
h: hour(s)
HIC: hydrophobic interaction chromatography
HMW: high molecular weight
HPLC: high-performance liquid chromatography
LC/MS: liquid chromatography-mass spectrometry
LC-MS/MS: liquid chromatography with tandem mass spectrometry
LLOQ: lower limit of quantification
m or min: minute(s)
Mal-C6-OH: 6-maleimidocaproic acid
Mal-C6-VA-PAB-PNP: 6-maleimidocaproyl-valinyl-alanyl-(4-aminobenzyl)-(4-nitrophenyl) carbonate
PAB: p-aminobenzyl
PBS: phosphate-buffered saline
PG: propylene glycol
(PNP)₂CO: bis(4-nitrophenyl)carbonate
PyAOP: (7-azabenzotriazol-1-yloxy)tripyrrolidinophosphonium hexafluorophosphate
RP-HPLC: reverse phase HPLC
SEC: size exclusion chromatography
TCEP: tris(2-carboxyethyl)phosphine
TFA: trifluoracetic acid
THF: tetrahydrofuran

### Synthetic Examples

### Example S1: Synthesis of Compound 1.

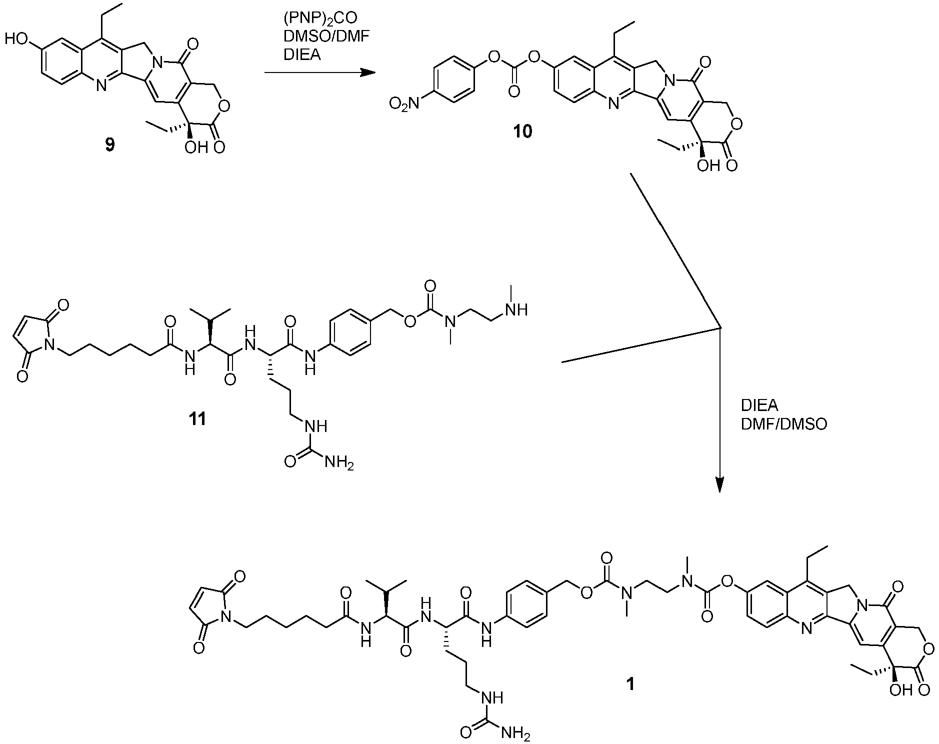

Compound 9 (Sigma-Aldrich Cat. #: H0165-50MG; 392 mg, 1 mmol) was dissolved in a mixture of DMSO (3 mL) and DMF (3 mL), followed by addition of a solution of (PNP)₂CO (912 mg, 3 mmol) in DMF (3 mL). The resulting mixture was cooled in an ice bath. Next, DIEA (174 µL, 1 mmol) was added and the reaction mixture was stirred for 15 min. The reaction mixture was added to 200 mL of diethyl ether. The resulting precipitate was collected and washed with ether (100 mL), and dried to give 10 (333 mg, 60%).

To a solution of 10 (55.7 mg, 0.1 mmol) in DMSO (1 mL) was added 11 (synthesized according to the procedure described in U.S. Patent No. 9,814,784) (TFA salt, 80 mg, 0.1 mmol) in DMF (2 mL). Next, DIEA (35 µL, 0.2 mmol) was added and the resulting reaction mixture was stirred for 30 min. Purification of the resulting material was performed by HPLC (0.1% TFA in water/acetonitrile), and the collected fractions were lyophilized to give 1 (84.6 mg, 77%).

### Example S2: Synthesis of Compound 13.

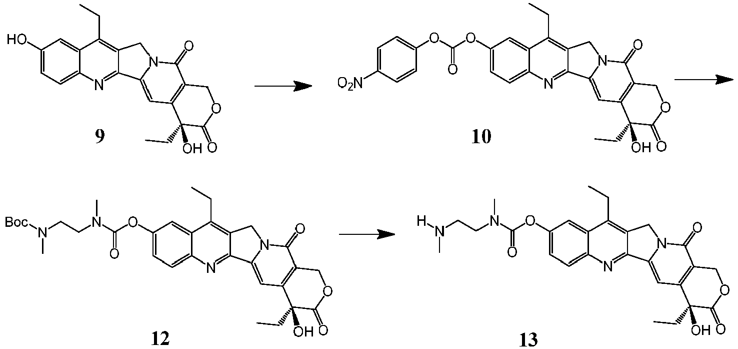

To a mixture of **9** (534 mg, 1.36 mmol) and bis(4-nitrophenyl) carbonate (900 mg, 2.96 mmol) in DMF (10 mL) was added DIEA (0.237 mL, 1.36 mmol). The resulting reaction mixture was stirred at room temperature until 9 was consumed. The reaction was monitored by LC/MS. Next, N-Boc-N,N'-dimethylethylenediamine (640 mg, 3.40 mmol) was added, followed by DIEA (0.525 mL, 3.00 mmol). The resulting mixture was stirred at room temperature for 2 hours. Compound 12 was obtained (400 mg) by preparative HPLC.

Compound 12 was treated with 25% TFA in DCM (5 mL) for 1 hour. The solvent was removed under vacuum, and crude 13 was used without further purification.

### Example S3: Synthesis of Compound 2.

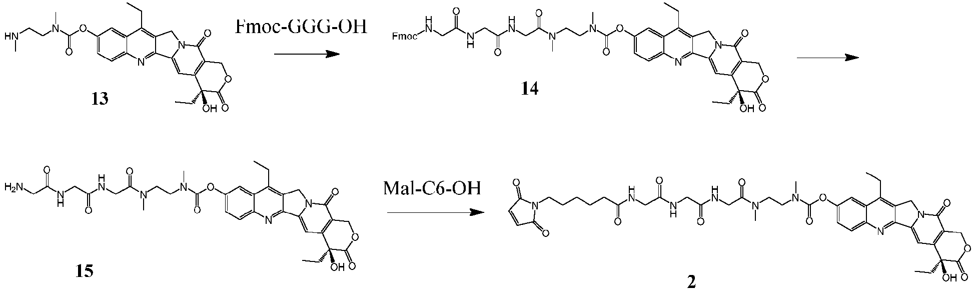

To a mixture of 13 (31 mg, 0.042 mmol), Fmoc-GGG-OH (17.4 mg, 0.042 mmol), and PyAOP (22 mg, 0.126 mmol) in DMF (2 mL) was added DIEA (25 uL, 1.36 mmol). The resulting reaction mixture was stirred at room temperature until 13 was consumed. The reaction was monitored by LC/MS. Next, piperidine (200 µL) was added and the resulting mixture was stirred at room temperature for 15 minutes. Compound 15 was obtained (25 mg) by preparative HPLC.

To a mixture of 15 (25 mg, 0.028 mmol), Mal-C6-OH (6.7 mg, 0.028 mmol), and PyAOP (15 mg, 0.028 mmol) in DMF (2 mL) was added DIEA (15 µL, 0.084 mmol). The resulting mixture was stirred at room temperature for 1 hour. Compound 2 was obtained by preparative HPLC.

### Example S4: Synthesis of Compound 3.

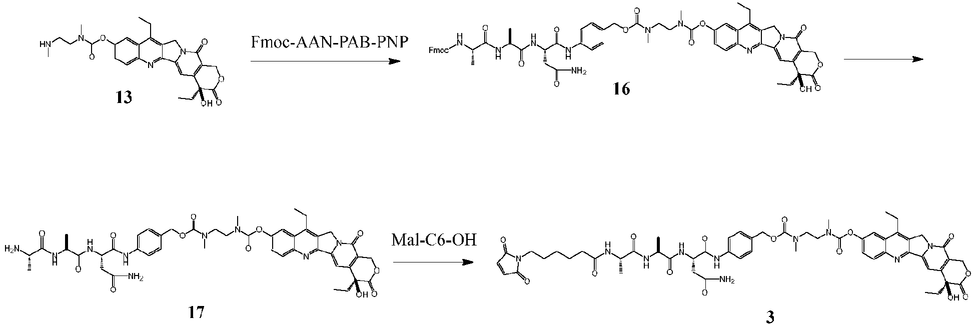

To a mixture of 13 (31 mg, 0.042 mmol) and Fmoc-AAN-PAB-PNP (31 mg, 0.042 mmol) in DMF (1 mL) was added DIEA (15 µL, 0.084 mmol). The resulting reaction mixture was stirred at room temperature overnight, followed by addition of piperidine (50 µL). The resulting mixture was stirred at room temperature for 15 minutes. Compound 17 was obtained (16 mg) by preparative HPLC.

To a mixture of 17 (16 mg, 0.014 mmol), Mal-C6-OH (4.6 mg, 0.022 mmol), and PyAOP (11.4 mg, 0.022 mmol) in DMF (2 mL) was added DIEA (16 µL, 0.088 mmol). The resulting mixture was stirred at room temperature for 1 hour. Compound 3 was obtained by preparative HPLC.

### Example S5: Synthesis of Compound 4.

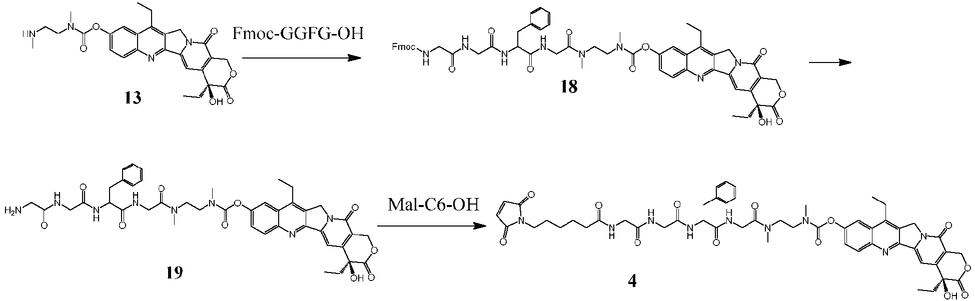

Compound 4 (10 mg, 20%) was synthesized according to the synthesis outlined for preparing 2 (Example S3) and as specifically shown in the scheme above.

### Example S6: Synthesis of Compound 5.

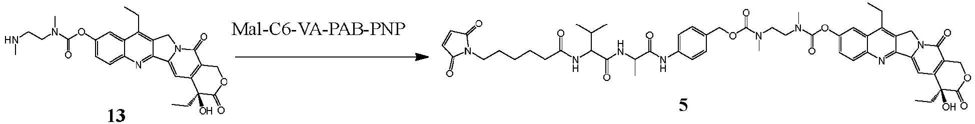

Compound 5 (8 mg, 25%) was synthesized according to the synthesis outlined for preparing 3 (Example S4) and as specifically shown in the scheme above using Mal-C6-VA-PAB-PNP.

### Example S7: Synthesis of Compound 6.

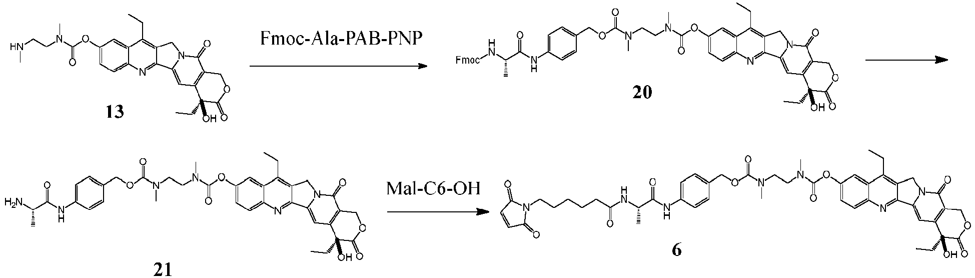

Compound 6 (12 mg, 23%) was synthesized according to the synthesis outlined for preparing 3 (Example S4) and as specifically shown in the scheme above using Fmoc-Ala-PAB-PNP.

### Example S8: Preparation of Antibody-Drug Conjugate (ADC) anti-Trop-2-Compound 1.

The anti-Trop-2 antibody used in this Example has the antibody sequence of the hRS7 antibody described in U.S. Patent No. 7,238,785. Affinity purified anti-Trop-2 antibody was buffer exchanged into sodium phosphate buffer (50 mM, pH 7.0-7.2) with EDTA (4 mM) at a concentration of 3-10 mg/mL. To a portion of this antibody stock was added a freshly prepared aqueous solution of TCEP (10 mM) in up to 20-fold molar excess. The resulting mixture was incubated at 4-8 °C overnight. The excess TCEP was removed by gel-filtration chromatography or several rounds of centrifugal filtration. UV-Vis quantification of recovered, reduced antibody was followed by confirmation of sufficient free thiol-to-antibody (SH/Ab) molar ratio. Briefly, a 1 mM aliquot of a freshly prepared solution of 5,5'-dithiobis-(2-nitrobenzoic acid) in sodium phosphate (50 mM, pH 7.0-7.2, 4 mM EDTA) was mixed with an equal volume of purified antibody solution. The resulting absorbance at 412 nm was measured and the reduced cysteine content was determined using the extinction coefficient of 14,150 M⁻¹cm⁻¹. The resulting SH/Ab measured ~8, indicating complete reduction of interchain cysteine thiol residues.

To initiate conjugation of Compound 1 to the anti-Trop-2 antibody, 1 was first dissolved in a 3:2 acetonitrile/water mixture at a concentration of 5 mM. Propylene glycol (PG) was then added to an aliquot of the reduced, purified anti-Trop-2 antibody to give a final concentration of 10-30% (v/v) PG before addition of the freshly prepared solution of 1 in 12-15-fold molar excess. After thorough mixing and incubation at ambient temperature for ≥1.5 h, the crude conjugation reaction was analyzed by HIC-HPLC to confirm reaction completion (disappearance of starting antibody peak) at 280 nm wavelength detection. Purification of the ADC anti-Trop-2-Compound **1** was then carried out by gel-filtration chromatography using an AKTA system equipped with a Superdex 200 pg column (GE Healthcare) equilibrated with PBS. The drug-to-antibody ratio (DAR) of 6-8 was calculated based on UV-VIS and HIC-HPLC. The HIC-HPLC of the resulting purified sample further indicates <1% (undetected) starting antibody material. Confirmation of low percent (<5%) HMW aggregates was also determined using analytical SEC-HPLC. After final characterization, an aliquot of sterile trehalose and Tween-80 solutions in water were added to the purified ADC anti-Trop-2-Compound **1** in PBS to give a final composition of 6% trehalose/0.02% Tween-80/94% PBS (v/v/v). These mixtures were then flash frozen in liquid nitrogen and stored at -80 °C until further use.

### Example S9: Preparation of Antibody-Drug Conjugates (ADCs) anti-Trop-2-Compound 2, anti-Trop-2-Compound 3, anti-Trop-2-Compound 4, anti-Trop-2-Compound 5, anti-Trop-2-Compound 6, and ADC-CL2A-SN38.

The additional ADCs anti-Trop-2-Compound **2,** anti-Trop-2-Compound **3,** anti-Trop-2-Compound 4, anti-Trop-2-Compound **5,** and anti-Trop-2-Compound **6** were prepared as outlined in Example S8 using **2, 3, 4, 5,** or **6,** respectively, in place of **1.** The comparative ADC molecule ADC-CL2A-SN38 was prepared as outlined in Example S8 using the SN38 moiety (prepared according to the procedures outlined in J. Med. Chem., 2008, 51, 6916-6926) in place of **1.**

### Biological Examples

### Example B1: In vitro Efficacy of Antibody-Drug Conjugates (ADCs) anti-Trop-2-Compound 2, anti-Trop-2-Compound 3, anti-Trop-2-Compound 4, anti-Trop-2-Compound 5, and anti-Trop-2-Compound 6.

The *in vitro* efficacies of ADCs anti-Trop-2-Compound 1, anti-Trop-2-Compound **2,** anti-Trop-2-Compound **3,** anti-Trop-2-Compound **4,** anti-Trop-2-Compound **5,** and anti-Trop-2-Compound 6 were evaluated using the following cell lines: BxPC-3 (pancreatic cancer), MDA-MB-468 (mammary gland/breast cancer), and L-540 (Hodgkin lymphoma). The *in vitro* assays were performed as follows. Cells were plated (375 cells/well for MDA-MB-468 and BxPC-3; 2,500 cells/well for L-540) in 12.5 µL per well of 384-well white clear bottom plates (2 plates per cell line) and maintained at 37°C for 2-4 hr. Next, 25 µL media was added only to the unused wells. Separately, working solutions were prepared at 2× final concentration. The cells were treated by adding 12.5 µL of respective working solution and cells were maintained for 120 hr at 37°C. Cell viability was then measured by CTG (CellTiter-Glo^{®} Luminescent Cell Viability Assay, Promega).

The cell viability for anti-Trop-2-Compound **1,** anti-Trop-2-Compound **2,** anti-Trop-2-Compound **3,** anti-Trop-2-Compound **4,** anti-Trop-2-Compound **5,** and anti-Trop-2-Compound **6** is shown in FIG. 1-FIG. 5. The data demonstrate that the tested ADCs have *in vitro* efficacy with EC₅₀ values ranging from approximately 46 to 340 nM.

### Example B2: In vivo Efficacy of Antibody-Drug Conjugates (ADCs) anti-Trop-2-Compound 1 and ADC-CL2A-SN38.

### Tumor cell inoculation and establishments of tumors

The human tumor cell lines MDA-MB-468 (triple negative breast cancer), NCI-N87 (gastric cancer), and BxPC-3 (pancreatic cancer) were cultured and expanded with 10% FBS RPMI 1640 medium. The cells were harvested with 0.05% Trypsin. Next, 5x10⁶ cells of each tumor cell line (in a total of 0.1 mL, 1:1 ratio of PBS and Matrigel) were injected subcutaneously into the upper right flank of each mouse (6 week old female of Nu/Nu mice from Charles River). Tumor growth was monitored by tumor volume measurement using a digital caliper starting 5-7 days after inoculation and followed 1-2 times per week until tumor volume reached ~100-250 mm³.

### Treatment

Once tumors were staged to the desired volume, animals were randomized and mice with very large or small tumors were culled. Mice were randomly assigned into control or treatment groups with 6-8 animals per group. Mice were then treated with either PBS/vehicle, anti-Trop-2 antibody, or ADC compounds anti-Trop-2-Compound **1** and ADC-CL2A-SN38. The treatments were given by tail vein injection with different combination of dosages at 2, 3, 5, 10, 15, and 25 mg/kg, twice weekly for a total of four treatments in a volume of 0.2 mL, respectively.

### Tumor growth measurement

Tumor growth responses were monitored once or twice weekly. Tumor volumes were measured by using a digital caliper once or twice weekly through the whole experiment period. The volume was calculated using the following formula: $volume \left({mm}^{3}\right) = \left[length \left(mm\right)\times width {\left(mm\right)}^{2}\right] / 2 .$ TGI % (percentage of tumor growth inhibition) was calculated using the following formula: $TGI % = \left\{1-\left[TVtd-TVt0\right]/CVtd-\left.CVt0\right]\right\} \times 100$ wherein:
TV = tumor volume of treated group,
CV = tumor volume of control group,
td = day after initial treatment, and
t0 = at day 0 treatment.
Mice were sacrificed by CO₂ asphyxiation when tumor load reached IACUC protocol limits (2000 mm³) or by the predetermined time.

### Results

### MDA-MB-468 xenograft

The efficacies of ADCs anti-Trop-2-Compound **1** and ADC-CL2A-SN38 were evaluated in MDA-MB-468 s.c xenograft in nude mice in two studies with different dose regimens. In one study, treatments of ADCs anti-Trop-2-Compound **1** and ADC-CL2A-SN38 were given at 2 and 5 mg/kg i.v biw x 4 compared with controls of PBS/vehicle and anti-CD38 antibody alone (5 mg/kg) (FIG. 6A). Both anti-Trop-2-Compound **1** and ADC-CL2A-SN38 showed very strong and dose dependent inhibition of MDA-MB-468 tumor growth. At 5 mg/kg, both anti-Trop-2-Compound **1** and ADC-CL2A-SN38 completely inhibited MDA-MB-468 tumor growth and reduced tumor sizes by 28.8% and 56.6%, respectively. In the lower dose treatment at 2 mg/kg, both ADCs anti-Trop-2-Compound **1** and ADC-CL2A-SN38 still demonstrated strong inhibition of tumor growth with sustained 95.4% and 88.6% of TGI up to 36 days after initial treatment, respectively.

In a second study, dose regimens with 3 and 10 mg/kg, i.v biw x 4 were tested. Strong inhibition was again evident in treatments of both **anti-Trop-2-Compound** 1 and ADC-CL2A-SN38 at 3 and 10 mg/kg by 90-100% of TGI and reduced tumor sizes, respectively (FIG. 6B). In this study, the inhibition effect was sustained up to ~100 days after initial treatment. The data demonstrate that ADC **anti-Trop-2-Compound** 1 significantly inhibited MDA-MB-468 xenograft tumor growth in nude mice.

### NCI-N87 xenograft

The efficacies of ADCs **anti-Trop-2-Compound** 1 and ADC-CL2A-SN38 were evaluated in NCI-N87 s.c xenograft in nude mice with dose regimens at 5 and 15 mg/kg i.v biw x 4 compared with controls of PBS/vehicle and anti-Trop-2 antibody alone (FIG. 7). The data demonstrate that both **anti-Trop-2-Compound** 1 and ADC-CL2A-SN38 inhibit tumor growth in a dose-dependent manner. At 15 mg/kg, **anti-Trop-2-Compound** 1 and ADC-CL2A-SN38 significantly inhibited tumor growth with 66.6% and 99.7% of TGI on day 22 after initial treatment, respectively. At 5 mg/kg, both **anti-Trop-2-Compound** 1 and ADC-CL2A-SN38 showed about 45.0% of nonsignificant tumor growth inhibition in the NCI-N87 xenograft model. The data demonstrate that **anti-Trop-2-Compound** 1 significantly inhibited NCI-N87 xenograft tumor growth in nude mice.

### BxPC3 xenograft

The efficacy of **anti-Trop-2-Compound** 1 was evaluated in BxPC3 s.c xenograft in nude mice with dose regimens at 3, 10, and 25 mg/kg i.v biw x 4 compared with ADC-CL2A-SN38 (10 mg/kg), PBS/vehicle, and anti-Trop-2 antibody alone (10 mg/kg) (FIG. 8). All three dosages of **anti-Trop-2-Compound** 1 significantly inhibited tumor growth by 85-100% of TGI at day 21 after initial treatment. Dose response of **anti-Trop-2-Compound** 1 treatment was not observed in the BxPC3 xenograft model. The data demonstrate that **anti-Trop-2-Compound** 1 significantly inhibited BxPC3 xenograft tumor growth in nude mice.

Tumor growth inhibition (TGI) of the above xenograft studies are presented in Table 2.

**Table 2. Tumor growth inhibition (TGI) of ADCs in Xenograft Tumor Models.**

| **ADC** | **Dose tested (i.v. biw x 4)** | **TGI % in Xenograft Tumor Models** | | |
|---|---|---|---|---|
| | | **MDA-MB-468** | **NCI-N87** | **BxPC3** |
| **ADC-CL2A-SN38** | 15 mg/kg | n/a | *99.7% (day 22) | n/a |
| | 10 mg/kg | *100% (day 77) | n/a | *85.4% (day 14) |
| | 5 mg/kg | *100% (day 36) | 45.1% (day 22) | n/a |
| | 3 mg/kg | *87.3% (day 77) | n/a | n/a |
| | 2 mg/kg | *88.6% (day 36) | n/a | n/a |
| **anti-Trop-2-Compound 1** | 25 mg/kg | n/a | n/a | *86.4% (day 14) |
| | 15 mg/kg | n/a | *66.6% (day 22) | n/a |
| | 10 mg/kg | *100% (day 77) | n/a | *89.4% (day 14) |
| | 5 mg/kg | *100% (day 36) | 42.5% (day 22) | n/a |
| | 3 mg/kg | *100% (day 77) | n/a | *82.6% (day 14) |
| | 2 mg/kg | *95.4% (day 36) | n/a | n/a |

| | | | | |
|---|---|---|---|---|
| $TGI % = \left\{1-\left[TVtd-TVt0\right]/CVtd-\left.CVt0\right]\right\} \times 100$ **TV** = tumor volume of treated group, **CV** = tumor volume of control group, **td** = day after initial treatment, **t0** = at day 0 treatment * P < 0.05, One way or Two way Anova with Dunnette's multiple comparison to vehicle/PBS | | | | |

### Example B3: In vitro stability of ADC anti-Trop-2-Compound 1.

The presence of both high molecular weight (HMW) aggregates and cleaved/released drug-linker fragments for ADC-CL2A-SN38 was monitored over time using analytical SEC (Tosoh TSKgel G3000SW-Xl column) under isocratic elution conditions containing neutral phosphate buffer and 15% isopropanol. Samples were monitored at both 280 nm absorbance (for detection of protein and drug-linker) and 370 nm (detection of drug-containing species only). The initial time point is defined as <1h following main peak elution during purification and includes the time needed for routine final processing. The final processing steps, all carried out at room temperature, include partial concentration to >2 mg/mL (via centrifugal ultrafiltration), sterile filtration, and final ADC dilution to 6% trehalose/PBS. Following the initial time point, the ADC mixture was stored for 24h at 4 °C before subsequent incubation at room temperature (protected from light) for an additional 144 hr (6 days). SEC analysis and monitoring of anti-Trop-2-Compound **1** was conducted both parallel to and in an identical manner to ADC-CL2A-SN38. The data demonstrate that anti-Trop-2-Compound **1** is significantly more stable than ADC-CL2A-SN38 with respect to both protein aggregation and spontaneous drug release (FIG. 9). The results of the stability study are summarized in Table 3.

**Table 3. Stability data of ADCs.**

| **Incubation time (hr)** | **ADC-CL2A-SN38** | | **anti-Trop-2-Compound 1** | |
|---|---|---|---|---|
| | **% HMW aggregate (280 nm)** | **% free drug release (370 nm)** | **%HMW aggregate (280 nm)** | **% free drug release (370 nm)** |
| <1 | 1.9 | 0.0 | 0.0 | 1.0 |
| 24 | 2.3 | 0.9 | 0.7 | 0.8 |
| 96 | 4.7 | 17.0 | 1.1 | 0.8 |
| 168 | 5.3 | 33.5 | 1.1 | 0.9 |

### Example B4: In vivo stability of ADC anti-Trop-2-Compound 1.

The *in vivo* stability of ADC anti-Trop-2-Compound **1** in serum was evaluated using Swiss Webster mice in 21 days with 14 time points. Briefly, anti-Trop-2-Compound **1** was administered by i.v. at 10 mg/mL. Whole blood samples (~150 µL) were collected through retro-orbital venous plexus at 5 min, 30 min, 1 hr, 5 hr, 24 hr, 48 hr, 72 hr, 96 hr, 120 hr, 168 hr, 240 hr, 336 hr, 408 hr, and 504 hr, respectively. Experiments were run in triplicate with 3 mice per each time points (n = 3). Serum was then collected and stored at -80 °C by centrifugation at 8000 rpm for 10 minutes after sitting the blood samples at 4 °C for 40 min.

Plasma stability of anti-Trop-2-Compound **1** was compared to unconjugated anti-Trop-2. The amount of conjugated anti-Trop-2-Compound **1** was found to closely match the amount of total antibody of the ADC anti-Trop-2-Compound **1,** which demonstrates that SN-38 was not significantly released from the ADC into plasma (FIG. 10). Thus, anti-Trop-2-Compound **1** is stable in plasma.

### Example B5: Pharmacokinetics/Pharmacodynamics of Antibody-Drug Conjugates (ADCs) anti-Trop-2-Compound 1 and ADC-CL2A-SN38.

The purpose of this study was to evaluate the pharmacokinetic parameters of the ADCs anti-Trop-2-Compound **1** and ADC-CL2A-SN38 following repeated intravenous infusions to cynomolgus macaques.

*Experimental Design.* A total of 12 cynomolgus macaques (*Macaca fascicularis*) were used in this study. The cynomolgus macaques (6 males, 6 females) were divided into 3 groups. Each group had 2 females and 2 males. Group 1 was treated with ADC-CL2A-SN38, and Groups 2 and 3 were treated with anti-Trop-2 Compound 1. Repeated intravenous infusions of ADCs were administered on Day 1 and Day 4. The ADCs were administered at a dose 60 mg/kg (drug concentration: 6 mg/mL). Blood samples were taken from the animals as follow: Groups 1 and 2 - Day 1 (before ADC administration), Day 4 (before ADC administration), 5 min, 30 min, 2 h, 4 h, 8 h, 24 h, 48 h, 72 h, 120 h, and 168 h after ADC administration on Day 4; Group 3 - Day 1 (before ADC administration), Day 4 (before ADC administration), 5 min, 30 min, 2 h, 4 h, 8 h, 24 h, 48 h, 72 h, 120 h, 168 h, 240 h, and 336 h after ADC administration on Day 4.

Samples of about 0.8 mL of blood were taken from a vein of the hind limbs or forelimbs at each time point. Each blood sample was transferred to a sample tube containing a separation gel and coagulant at room temperature, and centrifuged within 2 hours (1500 g, room temperature, 10 min). The centrifuged serum was transferred to a fresh centrifuge tube and stored below -70 °C.

The total antibody concentration was determined using Human TROP2/TACSTD2 Protein (His Tag) antigen together with a Goat anti-Human IgG Fc cross-adsorbed secondary antibody-HRP as the detection antibody (LLOQ: 19.5 ng/mL). The concentration of conjugated antibody of ADC-CL2A-SN38 was measured using a combination of an anti-SN38 antibody and Goat-anti-human IgG Monkey antibody (LLOQ: 19.5 ng/mL). The concentration of conjugated antibody of ADC anti-Trop-2-Compound **1** was determined using a combination of an anti-SN38 antibody and Goat anti-human IgG Monkey antibody (LLOQ: 19.5 ng/mL). Free or unconjugated SN-38 was quantitatively measured using LC-MS/MS (LLOQ: 0.200 ng/mL).

Data was processed using Watson LIMS v.7.5 SP1 (Thermo Science Inc.) software. Sample concentrations were calculated using Watson's calculation module based on equations obtained from fitting the analytical batch standard curve. WinNonLin v 5.2.1 (Pharsight Inc.) software was used to analyze drug metabolism parameters (Noncompartmental Analysis).

*Results.* The amounts of total antibody, conjugated antibody, and free SN-38 are summarized below in Table 4. The data demonstrate that administration of ADC-CL2A-SN38 to cynomolgus macaques results in the release of a significant amount of free SN-38 (*i.e.,* unconjugated drug), whereas only small amounts of free SN-38 are released from ADC anti-Trop-2-Compound **1.**

**Table 4. Total antibody, conjugated antibody, and free SN-38 in blood samples.**

| Day | Time | ADC-CL2A-SN38 (n=4)* | | | anti-Trop-2-Compound **1** (n=8)* | | |
|---|---|---|---|---|---|---|---|
| | | Total Antibody (µg/mL) | Conjugated Antibody (µg/mL) | SN-38 (µg/mL) | Total Antibody (µg/mL) | Conjugated Antibody (µg/mL) | SN-38 (µg/mL) |
| 1 | 0 | BQL | BQL | BQL | BQL | BQL | BQL |
| 4 | 0 | 486 ± 39.6 | 115 ± 76.7 | 216 ± 70.1 | 74.9 ± 28.3 | 49.9 ± 19.1 | BQL |
| 4 | 5 m | 1930 ± 370 | 1610 ± 156 | 2145 ± 186.3 | 1810 ± 145 | 1480 ± 313 | 3.4 ± 0.9 |
| 4 | 30 m | 1720 ± 193 | 1520 ± 243 | 1930 ± 331.3 | 1610 ± 112 | 1320 ± 266 | 3.8 ± 1.7 |
| 4 | 2 h | 1820 ± 392 | 1590 ± 257 | 1710 ± 340.9 | 1330 ± 106 | 1110 ± 198 | 9.3 ± 5.4 |
| 4 | 4 h | 1470 ± 190 | 1560 ± 296 | 1668 ± 443.7 | 1040 ± 77.8 | 893 ± 119 | 10.0 ± 3.8 |
| 4 | 8 h | 1340 ± 161 | 1540 ± 340 | 1388 ± 252.6 | 770 ± 63.6 | 690 ± 119 | 15.2 ± 9.4 |
| 4 | 24 h | 1100 ± 62.9 | 1360 ± 334 | 771 ± 146.2 | 330 ± 73.6 | 274 ± 61.2 | 1.3 ± 0.5 |
| 4 | 48 h | 972 ± 105 | 566 ± 171 | 566 ± 79.9 | 182 ± 66.6 | 148 ± 38.7 | 0.3 ± 0.1 |
| 4 | 72 h | 810 ± 91.0 | 148 ± 52.7 | 142 ± 92.1 | 130 ± 44.5 | 96.1 ± 41.4 | 0.2 ± 0.0 |
| 4 | 120 h | 487 ± 22.3 | 8.96 ± 3.99 | 12 ± 2.9 | 64.9 ± 30.6 | 53.1 ± 23.4 | BQL |
| 4 | 168 h | 209 ± 178 | 1.40 ± 0.447 | 2 ± 0.3 | 41.4 ± 20.6 | 26.3 ± 10.4 | BQL |
| 4 | 240h | N.A. | N.A. | N.A. | 20.8 ± 13.1 | 10.1 ± 8.81 | BQL |
| 4 | 336 h | N.A. | N.A. | N.A. | 6.72 ± 6.23 | 2.22(0.218~9.48) | BQL |
| 4 | 408 h | N.A. | N.A. | N.A. | 0.401(0.0212~7.10)^{&} | 0.219(BQL~2.52)^{&} | BQL |
| 4 | 504 h | N.A. | N.A. | N.A. | BQL(BQL~2.49)^{&} | BQL(BQL~1.30)^{&} | BQL |

| | | | | | | | |
|---|---|---|---|---|---|---|---|
| * Data is shown as mean ± standard deviation; BQL= Below the Limit of Quantification; N.A. = not applicable/not measured. ^{&} C.V.% (coefficient of variation) greater than 100%, indicated as median. | | | | | | | |

A summary of the pharmacokinetic parameters for total antibody, conjugated antibody, and free SN-38 following administration of ADC-CL2A-SN38 ADC and anti-Trop-2-Compound 1 to cynomolgus macaques is provided in Table 5.

**Table 5. Pharmacokinetic parameters of ADCs.**

| PK Parameter | Unit | ADC-CL2A-SN38 | | | anti-Trop-2-Compound **1** | | |
|---|---|---|---|---|---|---|---|
| | | Total Antibody | Conjugated Antibody | SN-38 | Total Antibody | Conjugated Antibody | SN-38 |
| Kₑₗ | 1/hr | 0.0137 ± 0.00851 | 0.0508 ± 0.00332 | 0.0438 ± 0.00769 | 0.0154 ± 0.00383 | 0.0178 ± 0.00650 | 0.0607 ± 0.0348 |
| t_{1/2} | hr | 62.1 ± 25.8 | 13.7 ± 0.854 | 16.2 ± 2.53 | 47.1 ± 10.2 | 43.2 ± 13.9 | 16.1 ± 10.8 |
| Tₘₐₓ^{#} | hr | 0.0833~2 | 0.0833~8 | 2.63 ± 1.70 | 0.0833 | 0.0833 | 6.75 ± 2.38 |
| Cₘₐₓ | µg/mL | 2010 ± 332 | 1690 ± 277 | 2018 ± 295 ^{a} | 1810 ± 145 | 1480 ± 313 | 15.7 ± 7.82 ^{a} |
| AUC₍₀₋₁₆₈₎ | hr* µg/mL | 122000 ± 6850 | 71100 ± 17200 | 57935 ± 12326 ^{a} | 34900 ± 7110 | 28800 ± 5700 | 235 ± 122 ^{a} |
| AUC₍₀₋₅₀₄₎ | hr* µg/mL | N.A. | N.A. | N.A. | 39700 ± 9710 | 34600 ± 2080 | 243 ± 89.7 ^{a} |
| AUC_{(0-inf)} | hr* µg/mL | 151000 ± 18500 | 71200 ± 17300 | 58039 ± 12432 ^{a} | 38400 ± 9270 | 30800 ± 6580 | 246 ± 98.3 ^{a} |
| AUC_{(t-inf)} | % | 18.3 ± 13.3 | 0.103 ± 0.115 | 0.159 ± 0.133 | 3.64 ± 4.11 | 3.06 ± 3.74 | 3.31 ± 3.24 |
| V_{d} | mL/kg | 34.5 ± 11.7 | 17.4 ± 4.33 | N.A. | 108 ± 19.7 | 124 ± 43.3 | N.A. |
| CL | mL/hr/kg | 0.401 ± 0.0553 | 0.876 ± 0.184 | N.A. | 1.64 ± 0.361 | 2.04 ± 0.527 | N.A. |
| MRT_{inf} | hr | 93.5 ± 27.8 | 26.4 ± 1.43 | N.A. | 53.6 ± 13.3 | 47.4 ± 12.2 | N.A. |

| | | | | | | | |
|---|---|---|---|---|---|---|---|
| N.A. = not applicable; ^{#} indicated by a range of values; ^{a} unit for SN-38 is hr* ng/mL. | | | | | | | |

After intravenous administration of 60 mg/kg ADC-CL2A-SN38 to cynomolgus macaques, the peak concentration (Cₘₐₓ) of total antibody and conjugated antibody was slightly higher than the peak concentration of anti-Trop-2-Compound **1** administered at the same dose. In addition, the exposure levels (AUC) of total antibody and conjugated antibody were significantly greater for ADC-CL2A-SN38 than for anti-Trop-2-Compound **1.**

Release of free SN-38 was significantly higher from ADC-CL2A-SN38 than from anti-Trop-2-Compound **1.** Specifically, the release of free SN-38 from ADC-CL2A-SN38 provided a peak concentration (Cₘₐₓ) that is approximately 129 times greater than the corresponding peak concentration from anti-Trop-2-Compound **1,** and an AUC value that is of approximately 247 times greater than the corresponding AUC parameter from anti-Trop-2-Compound 1. Furthermore, the half-life (t_{1/2}) of ADC-CL2A-SN38 total antibody is slightly longer than that of anti-Trop-2-Compound **1,** but due to rapid release of free SN-38, the half-life of ADC-CL2A-SN38 conjugated antibody is significantly shorter than the half-life of anti-Trop-2-Compound **1** conjugated antibody. The half-life of anti-Trop-2-Compound 1 total antibody and conjugated antibody are similar, about 40 hours. Furthermore, the AUC_{(0-inf)} ratio of total antibody to conjugated antibody for ADC-CL2A-SN38 is 2.1, whereas the corresponding value for anti-Trop-2-Compound 1 is 1.2.

Together, the data show that anti-Trop-2-Compound 1 has greater *in vivo* stability than ADC-CL2A-SN38 and releases less free SN-38. As the dissociation of SN-38 is related to a high adverse event frequency in subjects treated with SN-38-based ADCs, anti-Trop-2-Compound 1 offers improved safety in comparison to ADC-CL2A-SN38.

### Example B6: Toxicity Studies of Antibody-Drug Conjugates (ADCs) anti-Trop-2-Compound 1 and ADC-CL2A-SN38.

The purpose of this study was to evaluate the toxicity profiles of the ADCs anti-Trop-2-Compound **1** and ADC-CL2A-SN38 following repeated intravenous infusions to cynomolgus macaques.

### Experimental Design.

A total of 12 cynomolgus macaques *(Macaca fascicularis*) were used in this study. The cynomolgus macaques were randomly divided into 3 groups (4 animals/group, male and female) according to the weight of the animals.

Repeated intravenous infusions of ADCs were administered on Day 1 and Day 4. The ADCs were administered at a dose 60 mg/kg.

Group 1 animals were treated with ADC-CL2A-SN38 on Day 1 and Day 4. Group 2 and 3 animals were treated with anti-Trop-2-Compound **1** on Day 1 and Day 4. The ADCs were intravenously administered to the animals using a dosing capacity of 10 mL/kg and a dosing speed of approximately 0.33 mL/min/kg. Group 1 and Group 2 animals were euthanized one week after the final ADC treatment (Day 12). Group 3 animals were euthanized four weeks after the final ADC treatment (Day 30). During the study, clinical observations, weight, body temperature, electrocardiogram, blood cell counts, coagulation function, blood biochemistry, general anatomy, histopathology, and toxicokinetic were examined.

### Results.

*Death*/*Near Death.* During the study, 1 male animal treated with ADC-CL2A-SN38 was found dead on Day 11. The general anatomy of the dead animal showed small thymus; histopathological examination showed reduced number of diffuse cortical and medullary cells in the thymus (consistent with the results of general anatomy) and slightly reduced number of splenic white pulp multifocal cells. The dead animal clinically observed to have a small amount of yellow loose stool on Days 8 and 9, and showed lack of energy, lying prone, reduced spontaneous activity, and pale cheeks on Day 9. None of the animals treated with anti-Trop-2-Compound **1** died and none appeared near death.

*Clinical Observations.* During the study, the group of animals treated with ADC-CL2A-SN38 began to show abnormal clinical manifestations such as yellow loose stool, pale cheeks and gums, bleeding gums from Day 7. The group of animals treated with anti-Trop-2-Compound **1** began to show abnormal clinical signs of pale gums and cheeks, bleeding gum, and genital swelling from Day 7.

*Weight.* Relative to pre-treatment (Day -3), one male animal treated with ADC-CL2A-SN38 in Group 1 lost about 9.2% of its body weight (Day 7), and one female animal lost about 9.9% of its body weight (Day 7). The animals treated with anti-Trop-2-Compound 1 did not show significant abnormal changes in weight.

*Body Temperature and Electrocardiogram.* During the study, none of the animals treated with either ADC-CL2A-SN38 or anti-Trop-2-Compound **1** showed significant abnormal changes in body temperature or electrocardiogram parameters and waveforms.

*Blood Cell Count.* Relative to pre-treatment (Day -2), animals treated with ADC-CL2A-SN38 displayed a reduction in white blood cells, neutrophils, lymphocytes, and monocytes. These cells were not significantly reduced in the male and female animals treated with anti-Trop-2-Compound **1.** Relative to pre-treatment (Day -2), animals treated with ADC-CL2A-SN38 showed a reduction in red blood cells, hemoglobin, and red blood cell specific volume (Day 5 and/or Day 12). These cells were not significantly reduced in the male and female animals treated with anti-Trop-2-Compound **1.** Changes in these cell counts may be related to bone marrow inhibition._Relative to pre-treatment (Day -2), the platelet counts of two female animals treated with ADC-CL2A-SN38 increased (105.7% and 44.6%, Day 12).

*Coagulation Function.* Relative to pre-treatment (Day -2), the amount of fibrinogen increased in the animals treated with ADC-CL2A-SN38 on Day 12. The amount of fibrinogen also increased in the animals treated with anti-Trop-2-Compound **1,** and the activated partial thromboplastin time (aPTT) also increased.

*Blood Biochemistry.* Relative to pre-treatment (Day -2), animals treated with ADC-CL2A-SN38 or anti-Trop-2-Compound **1** showed an increase in total bilirubin (TBil) on Day 2 and Day 5, and a decrease in albumin on Day 12.

*General and Histological Pathology Examination.* End-of-treatment euthanasia (Day 12) for 3 animals treated with ADC-CL2A-SN38 showed that the animals had a small thymus, corresponding to the microscope observation results for slight to moderate reduction in cortical cell count and reduction in myelin cell count in thymus. The general lesion of the thymus are likely related to ADC-CL2A-SN38, due to their high occurrence and degree of lesions. End-of-treatment euthanasia (Day 12) for 2 female animals treated with anti-Trop-2-Compound **1** and end-of-treatment euthanasia (Day 30) for 1 male animal treated with anti-Trop-2-Compound **1** showed a slight reduction in the cortical diffuse cells in thymus. As such lesions are commonly observed as background lesions in cynomolgus monkeys and the degree of the lesions was relatively mild, it may or may not relate to anti-Trop-2-Compound **1.**

### Discussion.

Repeated intravenous infusions of ADC-CL2A-SN38 at a dose of 60 mg/kg to cynomolgus macaques can lead to animal death. The main toxic effects are: (i) weight loss; (ii) reduction of white blood cells, neutrophils, lymphocytes, monocytes, red blood cells, hemoglobin, HCT, Retic and albumin; and (iii) increased platelets, fibrinogen, and total bilirubin. The main target organs for toxicity are the thymus and spleen. In contrast, dosing of anti-Trop-2-Compound 1 led to significantly fewer toxic effects. Therefore, anti-Trop-2-Compound 1 provides an improvement with respect to toxicity (*i.e*., is less toxic) than ADC-CL2A-SN38.

Although the foregoing invention has been described in some detail by way of illustration and example for purposes of clarity of understanding, the descriptions and examples should not be construed as limiting the scope of the invention.

**Table of Sequences**

| SEQ ID NO | Description | Sequence |
|---|---|---|
| 1 | anti-Trop-2 antibody VL HVR1 | KASQDVSIAVA |
| 2 | anti-Trop-2 antibody VL HVR2 | SASYRYT |
| 3 | anti-Trop-2 antibody VL HVR3 | QQHYITPLT |
| 4 | anti-Trop-2 antibody VH HVR1 | NYGMN |
| 5 | anti-Trop-2 antibody VH HVR2 | WINTYTGEPTYTDDFKG |
| 6 | anti-Trop-2 antibody VH HVR3 | GGFGSSYWYFDV |
| 7 | anti-Trop-2 antibody VL | DIQLTQSPSSLSASVGDRVSITCKASQDVSIAVAWYQQKPGKAPKLLIYSASYRYTGV PDRFSGSGSGTDFTLTISSLQPEDFAVYYCQQHYITPLTFGAGTKVEIKR |
| 8 | anti-Trop-2 antibody VH | QVQLQQSGSELKKPGASVKVSCKASGYTFTNYGMNWVKQAPGQGLKWMGWINTYTGEP TYTDDFKGRFAFSLDTSVSTAYLQISSLKADDTAVYFCARGGFGSSYWYFV |
| 9 | Exemplary Human Trop-2 sequence (UniProt Accession No. P09758) | MARGPGLAPPPLRLPLLLLVLAAVTGHTAAQDNCTCPTNKMTVCSPDGPGGRCQCRAL GSGMAVDCSTLTSKCLLLKARMSAPKNARTLVRPSEHALVDNDGLYDPDCDPEGRFKA RQCNQTSVCWCVNSVGVRRTDKGDLSLRCDELVRTHHILIDLRHRPTAGAFNHSDLDA ELRRLFRERYRLHPKFVAAVHYEQPTIQIELRQNTSQKAAGDVDIGDAAYYFERDIKG ESLFQGRGGLDLRVRGEPLQVERTLIYYLDEIPPKFSMKRLTAGLIAVIVVVVVALVA GMAVLVITNRRKSGKYKKVEIKELGELRKEPSL |

## Claims

1. An antibody-drug conjugate (ADC) which is of formula (I): 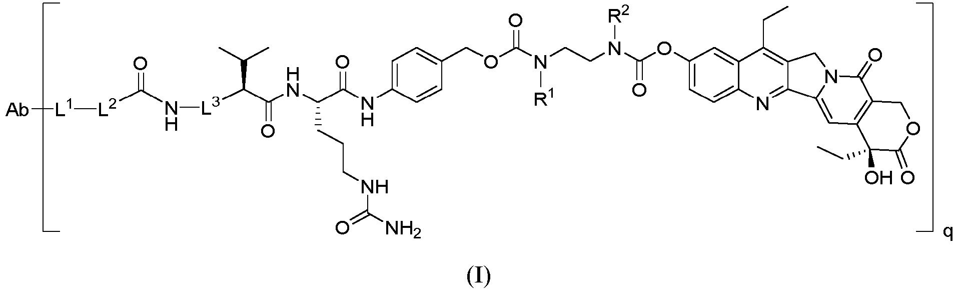 or is a pharmaceutically acceptable salt thereof, wherein:
Ab is an anti-Trop-2 antibody;
q is a value in the range of 1 to 20;
-L¹-L²- is 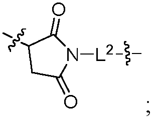
L² is -(CH₂)ₚ- where p is 4, 5, 6, 7, or 8;
L³ is a bond; and
R¹ and R² are each independently C₁₋₆ alkyl.

2. The ADC of claim 1,
wherein q is a value in the range of 1 to 10, preferably a value in the range of 6 to 8; and/or
wherein p is 4, 5, or 6, preferably 5.

3. The ADC of claim 1 or claim 2, wherein R¹ is C₁₋₄ alkyl or C₁₋₃ alkyl; preferably, wherein R¹ is methyl or ethyl.

4. The ADC of any one of claims 1-3, wherein R² is C₁₋₄ alkyl or C₁₋₃ alkyl; preferably, wherein R² is methyl or ethyl.

5. The ADC of any one of claims 1-4, wherein R¹ and R² are identical; and/or
wherein the ADC is of formula (IIIa-1) or (IIIb-1): 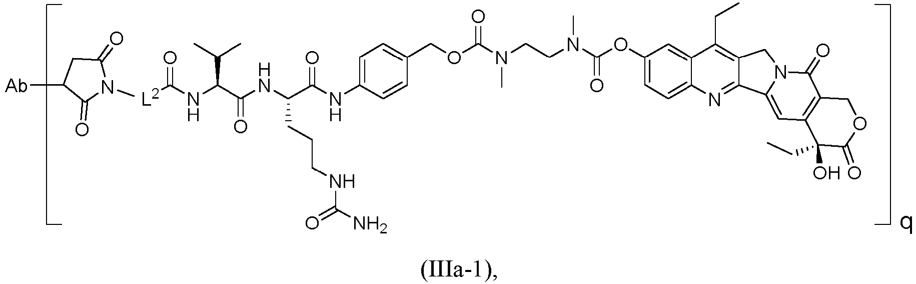 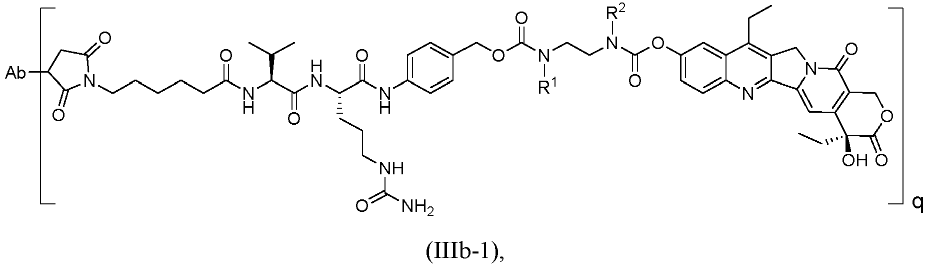 or a pharmaceutically acceptable salt thereof.

6. The ADC of claim 1, wherein the ADC is of formula (IV): 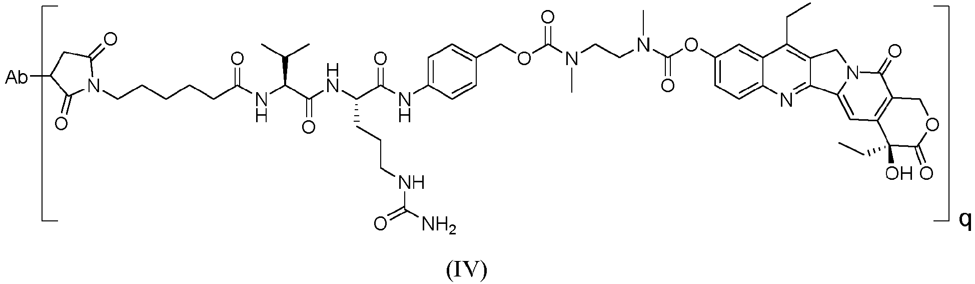 or a pharmaceutically acceptable salt thereof.

7. The ADC of any one of claims 1-6, wherein the anti-Trop-2 antibody comprises a VL HVR1 comprising the sequence of SEQ ID NO: 1, a VL HVR2 comprising the sequence of SEQ ID NO: 2, a VL HVR3 comprising the sequence of SEQ ID NO: 3, a VH HVR1 comprising the sequence of SEQ ID NO: 4, a VH HVR2 comprising the sequence of SEQ ID NO: 5, and a VH HVR3 comprising the sequence of SEQ ID NO: 6; and/or
wherein the anti-Trop-2 antibody comprises: a VL having a sequence with at least 95%, 96%, 97%, 98%, or 99% identity to SEQ ID NO: 7; a VH having a sequence with at least 95%, 96%, 97%, 98%, or 99% identity to SEQ ID NO: 8; a VL having the sequence of SEQ ID NO: 7; and/or a VH having the sequence of SEQ ID NO: 8; and/or
wherein the anti-Trop-2 antibody comprises: a kappa light chain, and/or an IgG antibody, optionally wherein the anti-Trop-2 antibody is an IgG1 antibody.

8. The ADC of any one of claims 1-7, for use in therapy, optionally for use in treating a Trop-2-expressing cancer.

9. The ADC for use of claim 8, wherein the Trop-2-expressing cancer is an epithelial-cell-derived cancer, optionally wherein the Trop-2-expressing cancer is a carcinoma; preferably,
wherein the carcinoma is a basal cell carcinoma, a squamous cell carcinoma, a renal cell carcinoma, a ductal carcinoma in situ, an invasive ductal carcinoma, or an adenocarcinoma.

10. The ADC for use of claim 9, wherein the Trop-2-expressing cancer comprises a solid tumor; and/or
wherein the Trop-2-expressing cancer is metastatic and/or a relapsed cancer.

11. The ADC for use of any one of claims 8-10, wherein the Trop-2-expressing cancer is a pancreatic cancer, a gastric cancer, a breast cancer, a melanoma, a kidney cancer, a colorectal cancer, an endometrial cancer, a prostate cancer, a urothelial cancer, a glioblastoma, a lung cancer, a cervical cancer, an esophageal cancer, or an ovarian cancer; preferably,
wherein the Trop-2-expressing cancer is a pancreatic cancer, a gastric cancer, or a breast cancer, optionally wherein the cancer is metastatic; preferably,
wherein the Trop-2-expressing cancer is triple-negative breast cancer, optionally wherein the cancer is metastatic.

12. A method of preparing the ADC of claim 1, comprising reacting an anti-Trop-2 antibody with a molecule of formula (P-I): 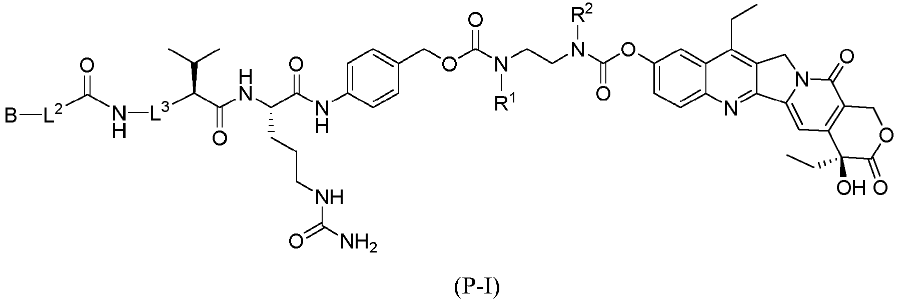
or a pharmaceutically acceptable salt thereof, wherein:
B-L²- is 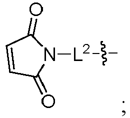
L² is -(CH₂)ₚ- where p is 4, 5, 6, 7, or 8;
L³ is a bond; and
R¹ and R² are each independently C₁₋₆ alkyl; preferably,
wherein the ADC is the ADC of any one of claims 1-7; and/or
wherein p is 4, 5, or 6, preferably 5; and/or
wherein R¹ is C₁₋₄ alkyl or C₁₋₃ alkyl; preferably,
wherein R¹ is methyl or ethyl.

13. The method of claim 12, wherein R² is C₁₋₄ alkyl or C₁₋₃ alkyl; preferably, wherein R² is methyl or ethyl.

14. The method of claim 12 or 13, wherein R¹ and R² are identical.

15. The method of any one of claims 12-14,
wherein the molecule is of formula (P-IIIa-1), or (P-IIIb-1): 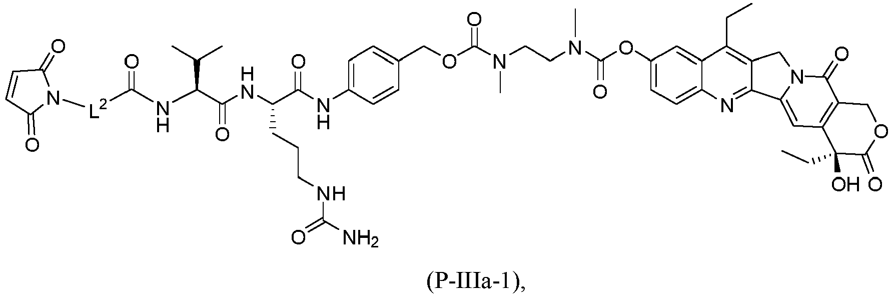 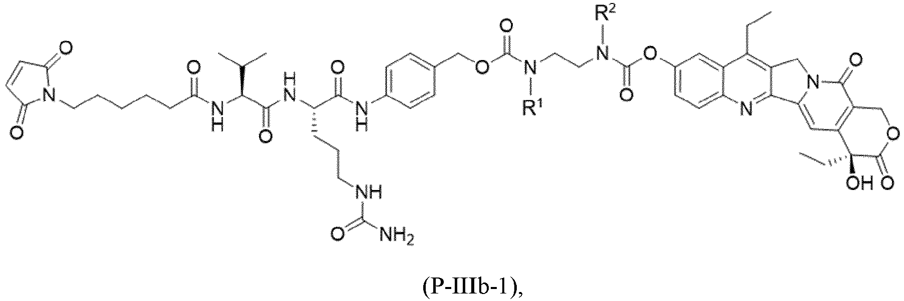 or a pharmaceutically acceptable salt thereof; or
wherein the molecule is of formula (P-IV): 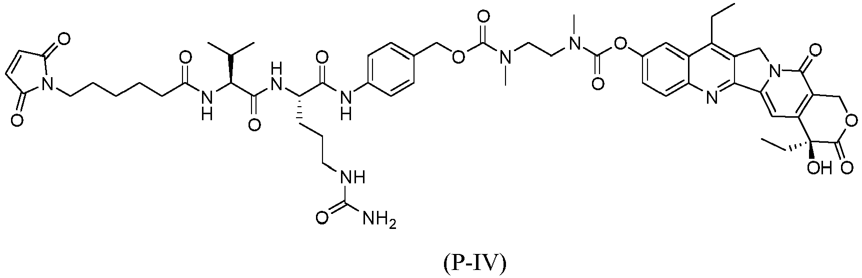 or a pharmaceutically acceptable salt thereof.

## Patentansprüche

1. Antikörper-Wirkstoff-Konjugat (ADC), das die Formel (I) aufweist: 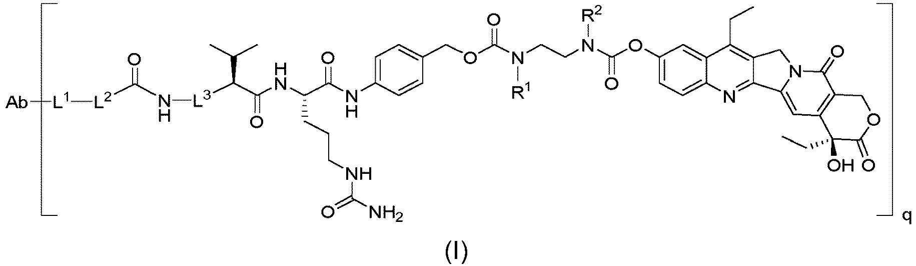 oder ein pharmazeutisch annehmbares Salz davon, wobei:
Ab ein Anti-Trop-2-Antikörper ist;
q ein Wert im Bereich von 1 bis 20 ist;
-L¹-L²- ist 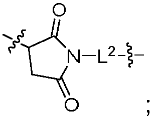
L² -(CH₂)ₚ- ist, wobei p 4, 5, 6, 7 oder 8 ist;
L³ eine Bindung ist; und
R¹ und R² jeweils unabhängig voneinander C₁₋₆-Alkyl sind.

2. ADC nach Anspruch 1,
wobei q ein Wert im Bereich von 1 bis 10 ist, vorzugsweise ein Wert im Bereich von 6 bis 8; und/oder
wobei p 4, 5 oder 6 ist, vorzugsweise 5.

3. ADC nach Anspruch 1 oder Anspruch 2, wobei R¹ C₁₋₄-Alkyl oder C₁₋₃-Alkyl ist; vorzugsweise,
wobei R¹ Methyl oder Ethyl ist.

4. ADC nach einem der Ansprüche 1 bis 3, wobei R² C₁₋₄-Alkyl oder C₁₋₃-Alkyl ist; vorzugsweise,
wobei R² Methyl oder Ethyl ist.

5. ADC nach einem der Ansprüche 1 bis 4, wobei R¹ und R² identisch sind; und/oder
wobei das ADC die Formel (IIIa-1) oder (IIIb-1) aufweist: 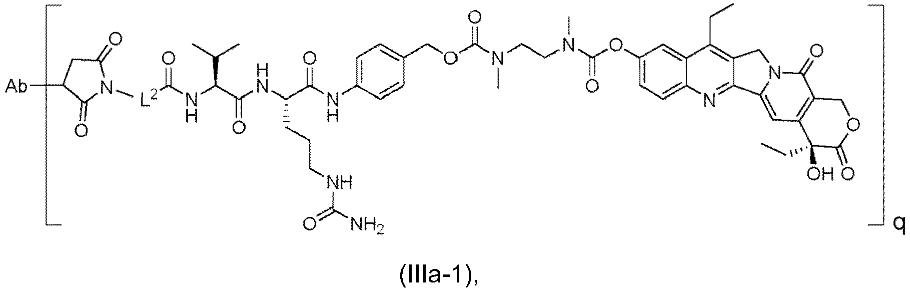 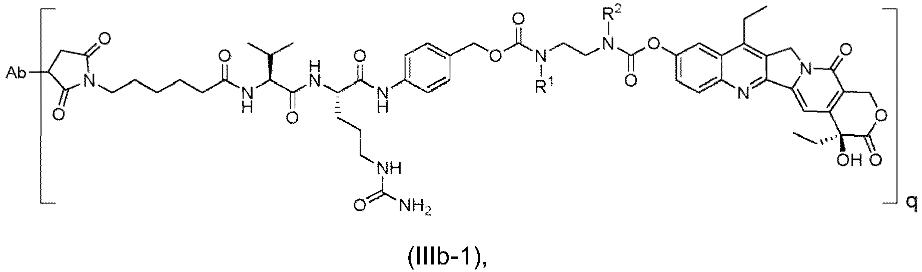 oder ein pharmazeutisch annehmbares Salz davon.

6. ADC nach Anspruch 1, wobei das ADC die Formel (IV) aufweist: 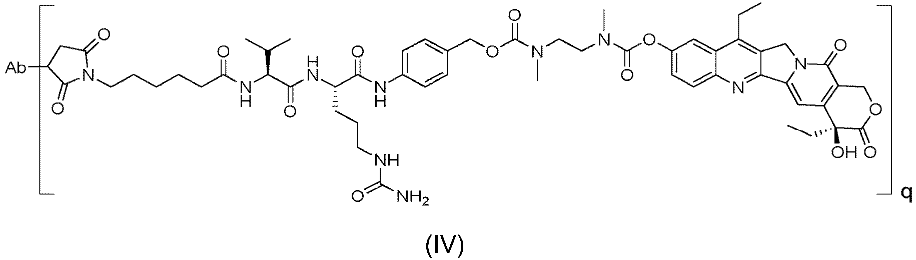 oder ein pharmazeutisch annehmbares Salz davon.

7. ADC nach einem der Ansprüche 1 bis 6, wobei der Anti-Trop-2- Antikörper ein VL HVR1 umfasst, umfassend die Sequenz von SEQ ID NO: 1, ein VL HVR2, umfassend die Sequenz von SEQ ID NO: 2, ein VL HVR3, umfassend die Sequenz von SEQ ID NO: 3, ein VH HVR1, umfassend die Sequenz von SEQ ID NO: 4, ein VH HVR2, umfassend die Sequenz von SEQ ID NO: 5, und ein VH HVR3, umfassend die Sequenz von SEQ ID NO: 6; und/oder
wobei der Anti-Trop-2-Antikörper umfasst: ein VL mit einer Sequenz von mindestens 95 %, 96 %, 97 %, 98 % oder 99 % Identität zu SEQ ID NO: 7; ein VH mit einer Sequenz von mindestens 95 %, 96 %, 97 %, 98 % oder 99 % Identität zu SEQ ID NO: 8; ein VL mit der Sequenz von SEQ ID NO: 7; und/oder ein VH mit der Sequenz von SEQ ID NO: 8; und/oder
wobei der Anti-Trop-2-Antikörper umfasst: eine Kappa-Leichtkette und/oder einen IgG-Antikörper, wobei der Anti-Trop-2-Antikörper optional ein IgG1-Antikörper ist.

8. ADC nach einem der Ansprüche 1 bis 7 zur Verwendung in der Therapie, optional zur Verwendung bei der Behandlung eines Trop-2-exprimierenden Krebses.

9. ADC zur Verwendung nach Anspruch 8, wobei der Trop-2-exprimierende Krebs ein aus Epithelzellen stammender Krebs ist, wobei der Trop-2-exprimierende Krebs optional ein Karzinom ist; vorzugsweise,
wobei das Karzinom ein Basalzellkarzinom, ein Plattenepithelkarzinom, ein Nierenzellkarzinom, ein duktales Karzinom in situ, ein invasives duktales Karzinom oder ein Adenokarzinom ist.

10. ADC zur Verwendung nach Anspruch 9, wobei der Trop-2-exprimierende Krebs einen soliden Tumor umfasst; und/oder
wobei der Trop-2-exprimierende Krebs metastasierend und/oder ein rezidivierender Krebs ist.

11. ADC zur Verwendung nach einem der Ansprüche 8 bis 10, wobei der Trop-2-exprimierende Krebs ein Pankreaskrebs, ein Magenkrebs, ein Brustkrebs, ein Melanom, ein Nierenkrebs, ein Darmkrebs, ein Gebärmutterkrebs, ein Prostatakrebs, ein Urothelkarzinom, ein Glioblastom, ein Lungenkrebs, ein Gebärmutterhalskrebs, ein Speiseröhrenkrebs oder ein Eierstockkrebs ist; vorzugsweise,
wobei der Trop-2-exprimierende Krebs ein Pankreaskrebs, ein Magenkrebs oder ein Brustkrebs ist, wobei optional der Krebs metastasierend ist; vorzugsweise,
wobei der Trop-2-exprimierende Krebs ein dreifach negativer Brustkrebs ist, wobei optional der Krebs metastasierend ist.

12. Verfahren zur Herstellung des ADC nach Anspruch 1, umfassend das Reagieren eines Anti-Trop-2-Antikörpers mit einem Molekül der Formel (P-I): 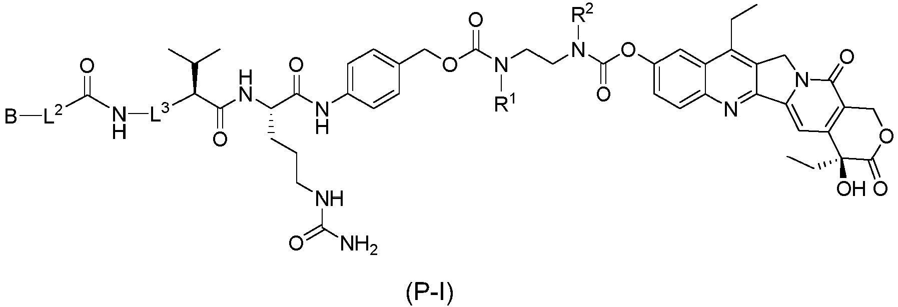
oder einem pharmazeutisch annehmbaren Salz davon, wobei:
B-L²- 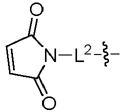 ist;
L² -(CH₂)ₚ- ist, wobei p 4, 5, 6, 7 oder 8 ist;
L³ eine Bindung ist; und
R¹ und R² jeweils unabhängig voneinander C₁₋₆-Alkyl sind; vorzugsweise,
wobei das ADC das ADC nach einem der Ansprüche 1 bis 7 ist; und/oder
wobei p 4, 5 oder 6 ist, vorzugsweise 5; und/oder
wobei R¹ C₁₋₄-Alkyl oder C₁₋₃-Alkyl ist; vorzugsweise,
wobei R¹ Methyl oder Ethyl ist.

13. Verfahren nach Anspruch 12, wobei R² C₁₋₄-Alkyl oder C₁₋₃-Alkyl ist; vorzugsweise, wobei R² Methyl oder Ethyl ist.

14. Verfahren nach einem der Ansprüche 12 oder 13, wobei R¹ und R² identisch sind.

15. Verfahren nach einem der Ansprüche 12 bis 14,
wobei das Molekül die Formel (P-IIIa-1) oder (P-IIIb-1) aufweist: 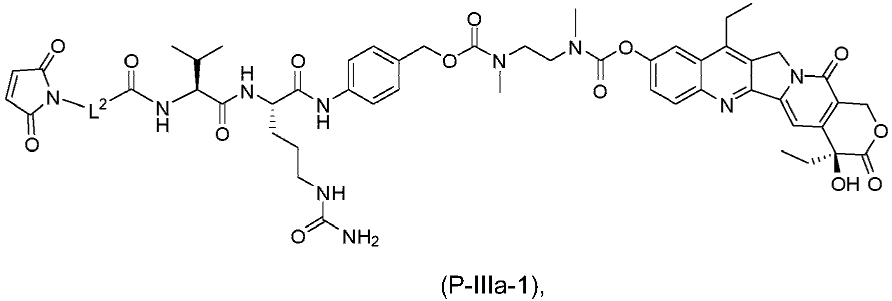 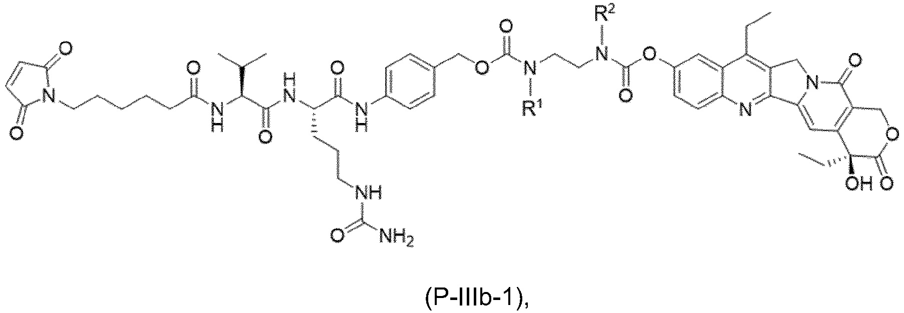 oder ein pharmazeutisch annehmbares Salz davon; oder
wobei das Molekül die Formel (P-IV) aufweist: 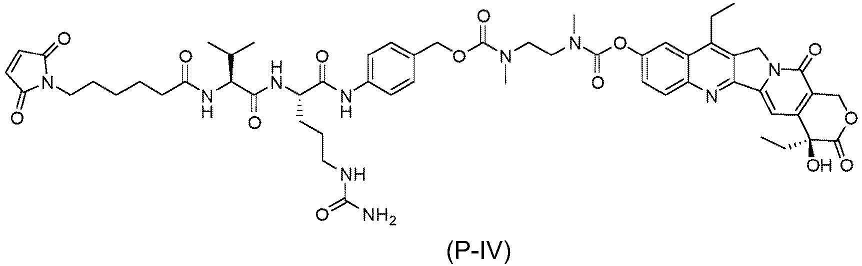 oder ein pharmazeutisch annehmbares Salz davon.

## Revendications

1. Conjugué anticorps-médicament (ADC) qui est de formule (I) : 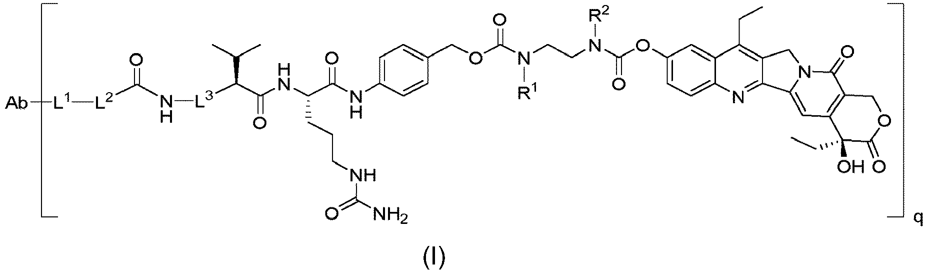 ou est un sel pharmaceutiquement acceptable de celui-ci, dans lequel :
Ab est un anticorps anti-Trop-2 ;
q est une valeur dans la plage de 1 à 20 ;
-L¹-L²- est 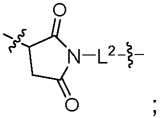
L² est -(CH₂)ₚ- où p est 4, 5, 6, 7 ou 8 ;
L³ est une liaison ; et
R¹ et R² sont chacun indépendamment alkyle en C₁₋₆.

2. ADC selon la revendication 1,
dans lequel q est une valeur dans la plage de 1 à 10, de préférence une valeur dans la plage de 6 à 8 ; et/ou
dans lequel p est 4, 5 ou 6, de préférence 5.

3. ADC selon la revendication 1 ou la revendication 2, dans lequel R¹ est alkyle en C₁₋₄ ou alkyle en C₁₋₃ ; de préférence,
dans lequel R¹ est méthyle ou éthyle.

4. ADC selon l'une quelconque des revendications 1 à 3, dans lequel R² est alkyle en C₁₋₄ ou alkyle en C₁₋₃ ; de préférence,
dans lequel R² est méthyle ou éthyle.

5. ADC selon l'une quelconque des revendications 1 à 4, dans lequel R¹ et R² sont identiques ; et/ou
dans lequel l'ADC est de formule (IIIa-1) ou (IIIb-1) : 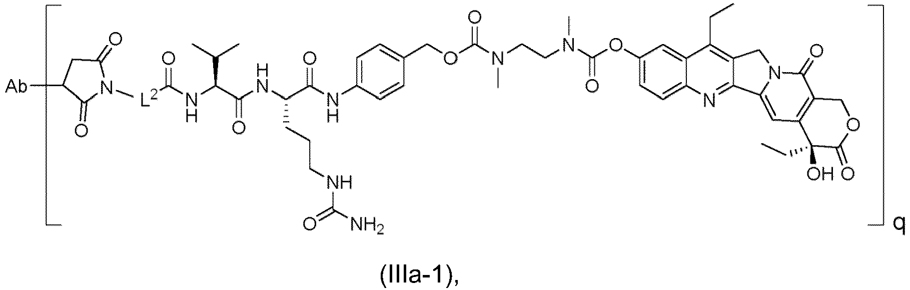 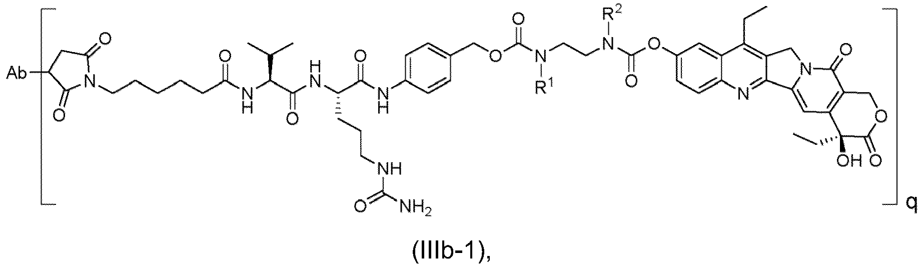 ou un sel pharmaceutiquement acceptable de celui-ci.

6. ADC selon la revendication 1, dans lequel l'ADC est de formule (IV) : 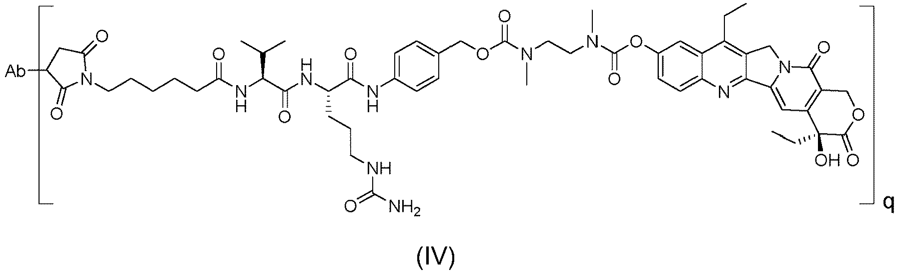 ou un sel pharmaceutiquement acceptable de celui-ci.

7. ADC selon l'une quelconque des revendications 1 à 6, dans lequel l'anticorps anti-Trop-2 comprend une HVR1 de VL comprenant la séquence de SEQ ID NO : 1, une HVR2 de VL comprenant la séquence de SEQ ID NO : 2, une HVR3 de VL comprenant la séquence de SEQ ID NO : 3, une HVR1 de VH comprenant la séquence de SEQ ID NO : 4, une HVR2 de VH comprenant la séquence de SEQ ID NO : 5 et une HVR3 de VH comprenant la séquence de SEQ ID NO : 6 ; et/ou
dans lequel l'anticorps anti-Trop-2 comprend : une VL ayant une séquence avec au moins 95 %, 96 %, 97 %, 98 % ou 99 % d'identité avec la SEQ ID NO : 7 ; une VH ayant une séquence avec au moins 95 %, 96 %, 97 %, 98 % ou 99 % d'identité avec la SEQ ID NO : 8 ; une VL ayant la séquence de SEQ ID NO : 7 et/ou une VH ayant la séquence de SEQ ID NO : 8 ; et/ou
dans lequel l'anticorps anti-Trop-2 comprend : une chaîne légère kappa et/ou un anticorps IgG, éventuellement dans lequel l'anticorps anti-Trop-2 est un anticorps IgG1.

8. ADC selon l'une quelconque des revendications 1 à 7, pour une utilisation en thérapie, éventuellement pour une utilisation dans le traitement d'un cancer exprimant Trop-2.

9. ADC pour une utilisation selon la revendication 8, dans lequel le cancer exprimant Trop-2 est un cancer dérivé de cellules épithéliales, éventuellement dans lequel le cancer exprimant Trop-2 est un carcinome ; de préférence,
dans lequel le carcinome est un carcinome basocellulaire, un carcinome à cellules squameuses, un carcinome à cellules rénales, un carcinome canalaire in situ, un carcinome canalaire invasif ou un adénocarcinome.

10. ADC pour une utilisation selon la revendication 9, dans lequel le cancer exprimant Trop-2 comprend une tumeur solide ; et/ou
dans lequel le cancer exprimant Trop-2 est métastatique et/ou un cancer récidivant.

11. ADC pour une utilisation selon l'une quelconque des revendications 8 à 10, dans lequel le cancer exprimant Trop-2 est un cancer du pancréas, un cancer de l'estomac, un cancer du sein, un mélanome, un cancer du rein, un cancer colorectal, un cancer de l'endomètre, un cancer de la prostate, un cancer urothélial, un glioblastome, un cancer du poumon, un cancer du col de l'utérus, un cancer de l'œsophage ou un cancer de l'ovaire ; de préférence,
dans lequel le cancer exprimant Trop-2 est un cancer du pancréas, un cancer de l'estomac ou un cancer du sein, éventuellement dans lequel le cancer est métastatique ; de préférence,
dans lequel le cancer exprimant Trop-2 est un cancer du sein triple négatif, éventuellement dans lequel le cancer est métastatique.

12. Procédé de préparation de l'ADC selon la revendication 1, comprenant la réaction d'un anticorps anti-Trop-2 avec une molécule de formule (P-I) : 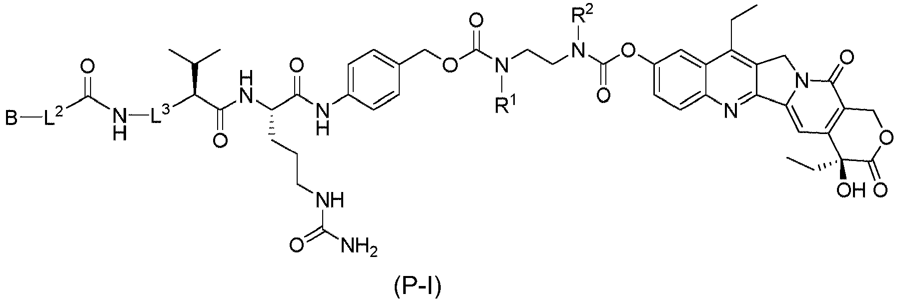
ou un sel pharmaceutiquement acceptable de celle-ci, dans lequel :
B-L²- est 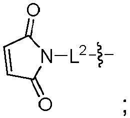
L² est -(CH₂)ₚ- où p est 4, 5, 6, 7 ou 8 ;
L³ est une liaison ; et
R¹ et R² sont chacun indépendamment alkyle en C₁₋₆ ; de préférence,
dans lequel l'ADC est l'ADC selon l'une quelconque des revendications 1 à 7 ; et/ou
dans lequel p est 4, 5 ou 6, de préférence 5 ; et/ou
dans lequel R¹ est alkyle en C₁₋₄ ou alkyle en C₁₋₃ ; de préférence,
dans lequel R¹ est méthyle ou éthyle.

13. Procédé selon la revendication 12, dans lequel R² est alkyle en C₁₋₄ ou alkyle en C₁₋₃ ; de préférence,
dans lequel R² est méthyle ou éthyle.

14. Procédé selon la revendication 12 ou 13, dans lequel R¹ et R² sont identiques.

15. Procédé selon l'une quelconque des revendications 12 à 14,
dans lequel la molécule est de formule (P-IIIa-1) ou (P-IIIb-1) : 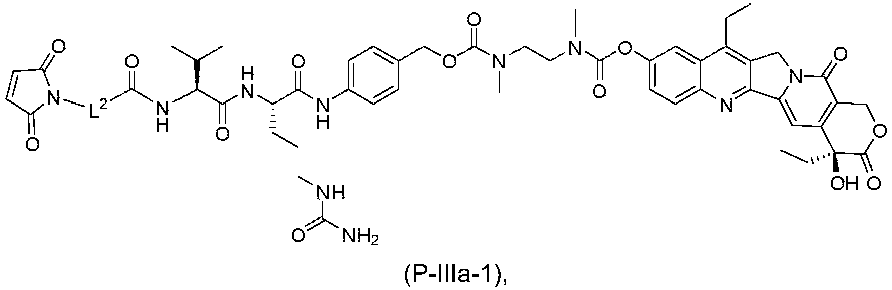 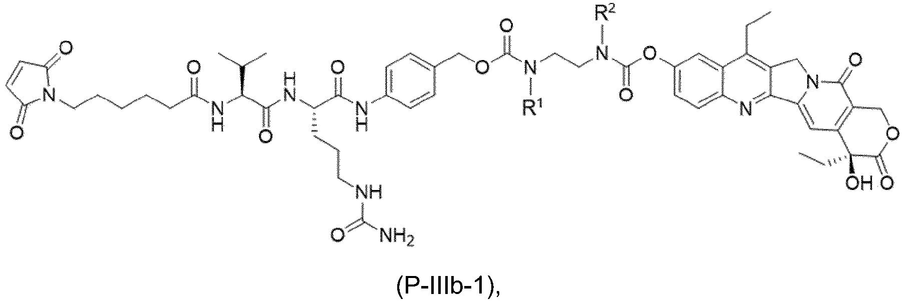 ou un sel pharmaceutiquement acceptable de celle-ci ; ou
dans lequel la molécule est de formule ou (P-IV) : 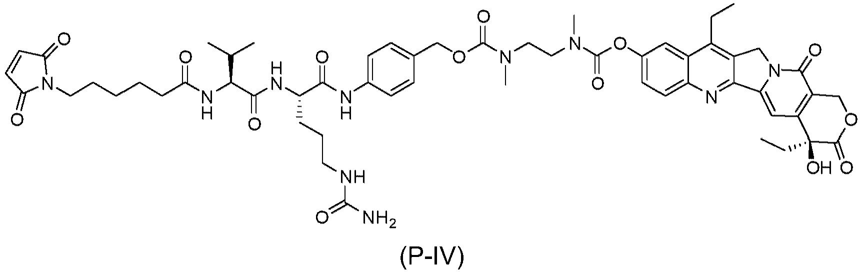 ou un sel pharmaceutiquement acceptable de celle-ci.
